(19) Europäisches Patentamt
European Patent Office
Office européen des brevets

(11) EP 1 664 351 B1

(12) EUROPÄISCHE PATENTSCHRIFT

(45) Veröffentlichungstag und Bekanntmachung des Hinweises auf die Patenterteilung:
**21.05.2008 Patentblatt 2008/21**

(21) Anmeldenummer: **04786995.3**

(22) Anmeldetag: **23.09.2004**

(51) Int Cl.:
*C12Q 1/70* (2006.01) *C12Q 1/68* (2006.01)

(86) Internationale Anmeldenummer:
**PCT/EP2004/010695**

(87) Internationale Veröffentlichungsnummer:
**WO 2005/031004 (07.04.2005 Gazette 2005/14)**

# (54) VERFAHREN ZUM SPEZIFISCHEN SCHNELLNACHWEIS GETR NKESCH DLICHER MIKROORGANISMEN

METHOD FOR THE SPECIFIC RAPID DETECTION OF BEVERAGE-SPOILING MICRO-ORGANISMS

PROCEDE DE DETECTION SPECIFIQUE RAPIDE DE MICRO-ORGANISMES NOCIFS POUR LES BOISSONS

(84) Benannte Vertragsstaaten:
**AT BE BG CH CY CZ DE DK EE ES FI FR GB GR HU IE IT LI LU MC NL PL PT RO SE SI SK TR**

(30) Priorität: **23.09.2003 DE 10344057**

(43) Veröffentlichungstag der Anmeldung:
**07.06.2006 Patentblatt 2006/23**

(73) Patentinhaber: **Vermicon AG**
**80992 München (DE)**

(72) Erfinder:
- **SNAIDR, Jiri**
  **80992 München (DE)**
- **BEIMFOHR, Claudia**
  **80797 München (DE)**
- **THELEN, Karin**
  **82166 Gräfelfing (DE)**
- **LEHNER, Angelika**
  **80011 München (DE)**

(74) Vertreter: **Bohmann, Armin K.**
**Bohmann & Loosen**
**Anwaltssozietät**
**Nymphenburger Strasse 1**
**80335 München (DE)**

(56) Entgegenhaltungen:
**WO-A-00/77259** **WO-A-01/53316**
**WO-A-98/55649** **WO-A-02/103043**
**WO-A-03/097868** **WO-A-20/04063699**
**DE-A1- 19 934 510**

- **JAMES S A ET AL: "Use of an rRNA internal transcribed spacer region to distinguish phylogenetically closely related species of the genera Zygosaccharomyces and Torulaspora" INTERNATIONAL JOURNAL OF SYSTEMATIC BACTERIOLOGY, SOCIETY FOR GENERAL MICROBIOLOGY, READING, GB, Bd. 46, Nr. 1, Januar 1996 (1996-01), Seiten 189-194, XP002102425 ISSN: 0020-7713**
- **MORRISON L E ET AL: "Solution-phase detection of polynucleotides using interacting fluorescent labels and competitive hybridization" ANALYTICAL BIOCHEMISTRY, ACADEMIC PRESS, NEW YORK, NY, US, Bd. 183, Nr. 2, Dezember 1989 (1989-12), Seiten 231-244, XP002113097 ISSN: 0003-2697**
- **BRETAGNE S ET AL: "DETECTION OF ASPERGILLUS SPECIES DNA IN BRONCHOALVEOLAR LAVAGE SAMPLES BY COMPETITIVE PCR" JOURNAL OF CLINICAL MICROBIOLOGY, WASHINGTON, DC, US, Bd. 33, Nr. 5, Mai 1995 (1995-05), Seiten 1164-1168, XP000874668 ISSN: 0095-1137**
- **NOVAK R ET AL: "COMPETITIVE DNA HYBRIDIZATION IN MICROTIRE PLATES FOR CHICKEN ANAEMIA VIRUS" MOLECULAR AND CELLULAR PROBES, ACADEMIC PRESS LTD, GB, Bd. 15, Nr. 1, Februar 2001 (2001-02), Seiten 1-11, XP001147365 ISSN: 0890-8508**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **TORTORELO M L ET AL: "DIRECT ENUMERATION OF ESCHERICHIA COLI AND ENTERIC BACTERIA IN WATER, BEVERAGES ANS SPROUTS BY 16S RRNA IN SITU HYBRIDIZATION" FOOD MICROBIOLOGY, ACADEMIC PRESS LTD, LONDON, GB, Bd. 17, Nr. 3, Juni 2000 (2000-06), Seiten 305-313, XP001077708 ISSN: 0740-0020**
- **DATABASE EMBL [Online] 2. November 2002 (2002-11-02), "KD2317.p1 Kluyveromyces delphensis Random Genomic Library Kluyveromyces delphensis genomic clone KD2317, DNA sequence." XP002319768 gefunden im EBI accession no. EM_PRO: BZ303298 Database accession no. BZ303298**
- **DATABASE EMBL [Online] 2. November 2002 (2002-11-02), "CG0215.f1 Candida glabrata Random Genomic Library Candida glabrata genomic clone CG0215, DNA sequence." XP002319769 gefunden im EBI accession no. EM_PRO:BZ293221 Database accession no. BZ293221**

SEQUENCE LISTING

<110> Vermicon AG

<120> Method for the specific fast detection of microorganisms which are harmful to beverages

<130> v 7588

<140> PCT/
<141> 2004-09-23

<150> DE 103 44 057.7
<151> 2003-09-23

<160> 1144

<170> PatentIn version 3.3

<210> 1
<211> 21
<212> DNA
<213> Artificial

<220>
<223> oligonucleotide

<400> 1
gtttgaccag attctccgct c 21

<210> 2
<211> 22
<212> DNA
<213> Artificial

<220>
<223> oligonucleotide

<400> 2
gtttgaccag attttccgct ct 22

<210> 3
<211> 22
<212> DNA
<213> Artificial

<220>
<223> oligonucleotide

<400> 3
gtttgaccaa attttccgct ct 22

<210> 4
<211> 22
<212> DNA
<213> Artificial

<220>
<223> oligonucleotide

<400> 4
gtttgtccaa attctccgct ct 22

<210> 5
<211> 18
<212> DNA
<213> Artificial

<220>
<223> oligonucleotide

<400> 5
cccggtcgaa ttaaaacc 18

<210> 6
<211> 18
<212> DNA
<213> Artificial

<220>
<223> oligonucleotide

<400> 6
gcccggtcga attaaaac 18

<210> 7
<211> 18
<212> DNA
<213> Artificial

<220>
<223> oligonucleotide

<400> 7
ggcccggtcg aattaaaa 18

<210> 8
<211> 18
<212> DNA
<213> Artificial

<220>
<223> oligonucleotide

<400> 8
aggcccggtc gaattaaa 18

<210> 9
<211> 18
<212> DNA
<213> Artificial

<220>
<223> oligonucleotide

<400> 9
aaggcccggt cgaattaa 18

<210> 10
<211> 18
<212> DNA
<213> Artificial

<220>
<223> oligonucleotide

<400> 10
atattcgagc gaaacgcc 18

<210> 11
<211> 18
<212> DNA
<213> Artificial

<220>
<223> oligonucleotide

<400> 11
aaagatccgg accggccg 18

<210> 12
<211> 18
<212> DNA

<213> Artificial

<220>
<223> oligonucleotide

<400> 12
ggaaagatcc ggaccggc 18

<210> 13
<211> 18
<212> DNA
<213> Artificial

<220>
<223> oligonucleotide

<400> 13
gaaagatccg gaccggcc 18

<210> 14
<211> 18
<212> DNA
<213> Artificial

<220>
<223> oligonucleotide

<400> 14
gatccggacc ggccgacc 18

<210> 15
<211> 18
<212> DNA
<213> Artificial

<220>
<223> oligonucleotide

<400> 15
agatccggac cggccgac 18

<210> 16
<211> 18
<212> DNA
<213> Artificial

<220>
<223> oligonucleotide

<400> 16
aagatccgga ccggccga 18

<210> 17
<211> 18
<212> DNA
<213> Artificial

<220>

<223> oligonucleotide

<400> 17
gaaaggcccg gtcgaatt 18

<210> 18
<211> 18
<212> DNA
<213> Artificial

<220>
<223> oligonucleotide

<400> 18
aaaggcccgg tcgaatta 18

<210> 19
<211> 18
<212> DNA
<213> Artificial

<220>
<223> oligonucleotide

<400> 19
ggaaaggccc ggtcgaat 18

<210> 20
<211> 18
<212> DNA
<213> Artificial

<220>
<223> oligonucleotide

<400> 20
aggaaaggcc cggtcgaa 18

<210> 21
<211> 18
<212> DNA
<213> Artificial

<220>
<223> oligonucleotide

<400> 21
aaggaaaggc ccggtcga 18

<210> 22
<211> 20
<212> DNA
<213> Artificial

<220>
<223> oligonucleotide

<400> 22

atagcactgg gatcctcgcc 20

<210> 23
<211> 20
<212> DNA
<213> Artificial

<220>
<223> oligonucleotide

<400> 23
ccagccccaa agttaccttc 20

<210> 24
<211> 20
<212> DNA
<213> Artificial

<220>
<223> oligonucleotide

<400> 24
tccttgacgt aaagtcgcag 20

<210> 25
<211> 18
<212> DNA
<213> Artificial

<220>
<223> oligonucleotide

<400> 25
ggaagaaaac cagtacgc 18

<210> 26
<211> 18
<212> DNA
<213> Artificial

<220>
<223> oligonucleotide

<400> 26
ccggtcggaa gaaaacca 18

<210> 27
<211> 18
<212> DNA
<213> Artificial

<220>
<223> oligonucleotide

<400> 27
gaagaaaacc agtacgcg 18

<210> 28

<211> 18
<212> DNA
<213> Artificial

<220>
<223> oligonucleotide

<400> 28
cccggtcgga agaaaacc 18

<210> 29
<211> 18
<212> DNA
<213> Artificial

<220>
<223> oligonucleotide

<400> 29
cggtcggaag aaaaccag 18

<210> 30
<211> 18
<212> DNA
<213> Artificial

<220>
<223> oligonucleotide

<400> 30
ggtcggaaga aaaccagt 18

<210> 31
<211> 18
<212> DNA
<213> Artificial

<220>
<223> oligonucleotide

<400> 31
aagaaaacca gtacgcgg 18

<210> 32
<211> 18
<212> DNA
<213> Artificial

<220>
<223> oligonucleotide

<400> 32
gtacgcggaa aaatccgg 18

<210> 33
<211> 18
<212> DNA
<213> Artificial

<220>
<223> oligonucleotide

<400> 33
agtacgcgga aaaatccg 18

<210> 34
<211> 18
<212> DNA
<213> Artificial

<220>
<223> oligonucleotide

<400> 34
gcggaaaaat ccggaccg 18

<210> 35
<211> 18
<212> DNA
<213> Artificial

<220>
<223> oligonucleotide

<400> 35
cggaagaaaa ccagtacg 18

<210> 36
<211> 18
<212> DNA
<213> Artificial

<220>
<223> oligonucleotide

<400> 36
gcccggtcgg aagaaaac 18

<210> 37
<211> 18
<212> DNA
<213> Artificial

<220>
<223> oligonucleotide

<400> 37
cgcggaaaaa tccggacc 18

<210> 38
<211> 18
<212> DNA
<213> Artificial

<220>
<223> oligonucleotide

<400> 38
cagtacgcgg aaaaatcc 18

<210> 39
<211> 18
<212> DNA
<213> Artificial

<220>
<223> oligonucleotide

<400> 39
agaaaaccag tacgcgga 18

<210> 40
<211> 18
<212> DNA
<213> Artificial

<220>
<223> oligonucleotide

<400> 40
ggcccggtcg gaagaaaa 18

<210> 41
<211> 18
<212> DNA
<213> Artificial

<220>
<223> oligonucleotide

<400> 41
ataaacacca cccgatcc 18

<210> 42
<211> 18
<212> DNA
<213> Artificial

<220>
<223> oligonucleotide

<400> 42
acgcggaaaa atccggac 18

<210> 43
<211> 18
<212> DNA
<213> Artificial

<220>
<223> oligonucleotide

<400> 43
gagaggcccg gtcggaag 18

<210> 44
<211> 18
<212> DNA
<213> Artificial

<220>
<223> oligonucleotide

<400> 44
agaggcccgg tcggaaga 18

<210> 45
<211> 18
<212> DNA
<213> Artificial

<220>
<223> oligonucleotide

<400> 45
gaggcccggt cggaagaa 18

<210> 46
<211> 18
<212> DNA
<213> Artificial

<220>
<223> oligonucleotide

<400> 46
aggcccggtc ggaagaaa 18

<210> 47
<211> 18
<212> DNA
<213> Artificial

<220>
<223> oligonucleotide

<400> 47
ccgagtgggt cagtaaat 18

<210> 48
<211> 18
<212> DNA
<213> Artificial

<220>
<223> oligonucleotide

<400> 48
ccagtacgcg gaaaaatc 18

<210> 49
<211> 18
<212> DNA

<213> Artificial

<220>
<223> oligonucleotide

<400> 49
taaacaccac ccgatccc 18

<210> 50
<211> 18
<212> DNA
<213> Artificial

<220>
<223> oligonucleotide

<400> 50
ggagaggccc ggtcggaa 18

<210> 51
<211> 18
<212> DNA
<213> Artificial

<220>
<223> oligonucleotide

<400> 51
gaaaaccagt acgcggaa 18

<210> 52
<211> 18
<212> DNA
<213> Artificial

<220>
<223> oligonucleotide

<400> 52
tacgcggaaa aatccgga 18

<210> 53
<211> 18
<212> DNA
<213> Artificial

<220>
<223> oligonucleotide

<400> 53
ggccacaggg acccaggg 18

<210> 54
<211> 18
<212> DNA
<213> Artificial

<220>

<223> oligonucleotide

<400> 54
tcaccaaggg ccacaggg 18

<210> 55
<211> 18
<212> DNA
<213> Artificial

<220>
<223> oligonucleotide

<400> 55
gggccacagg gacccagg 18

<210> 56
<211> 18
<212> DNA
<213> Artificial

<220>
<223> oligonucleotide

<400> 56
ttcaccaagg gccacagg 18

<210> 57
<211> 18
<212> DNA
<213> Artificial

<220>
<223> oligonucleotide

<400> 57
acagggaccc agggctag 18

<210> 58
<211> 18
<212> DNA
<213> Artificial

<220>
<223> oligonucleotide

<400> 58
agggccacag ggacccag 18

<210> 59
<211> 18
<212> DNA
<213> Artificial

<220>
<223> oligonucleotide

<400> 59

gttcaccaag ggccacag 18

<210> 60
<211> 18
<212> DNA
<213> Artificial

<220>
<223> oligonucleotide

<400> 60
gccacaggga cccagggc 18

<210> 61
<211> 18
<212> DNA
<213> Artificial

<220>
<223> oligonucleotide

<400> 61
cagggaccca gggctagc 18

<210> 62
<211> 18
<212> DNA
<213> Artificial

<220>
<223> oligonucleotide

<400> 62
agggacccag ggctagcc 18

<210> 63
<211> 18
<212> DNA
<213> Artificial

<220>
<223> oligonucleotide

<400> 63
accaagggcc acagggac 18

<210> 64
<211> 18
<212> DNA
<213> Artificial

<220>
<223> oligonucleotide

<400> 64
ccacagggac ccagggct 18

<210> 65

<211> 18
<212> DNA
<213> Artificial

<220>
<223> oligonucleotide

<400> 65
cacagggacc cagggcta 18

<210> 66
<211> 18
<212> DNA
<213> Artificial

<220>
<223> oligonucleotide

<400> 66
caccaagggc cacaggga 18

<210> 67
<211> 18
<212> DNA
<213> Artificial

<220>
<223> oligonucleotide

<400> 67
gggacccagg gctagcca 18

<210> 68
<211> 18
<212> DNA
<213> Artificial

<220>
<223> oligonucleotide

<400> 68
aggagaggcc cggtcgga 18

<210> 69
<211> 18
<212> DNA
<213> Artificial

<220>
<223> oligonucleotide

<400> 69
aaggagaggc ccggtcgg 18

<210> 70
<211> 18
<212> DNA
<213> Artificial

<220>
<223> oligonucleotide

<400> 70 18
gaaggagagg cccggtcg 18

<210> 71
<211> 18
<212> DNA
<213> Artificial

<220>
<223> oligonucleotide

<400> 71
agggctagcc agaaggag 18

<210> 72
<211> 18
<212> DNA
<213> Artificial

<220>
<223> oligonucleotide

<400> 72
gggctagcca gaaggaga 18

<210> 73
<211> 18
<212> DNA
<213> Artificial

<220>
<223> oligonucleotide

<400> 73
agaaggagag gcccggtc 18

<210> 74
<211> 18
<212> DNA
<213> Artificial

<220>
<223> oligonucleotide

<400> 74
caagggccac agggaccc 18

<210> 75
<211> 18
<212> DNA
<213> Artificial

<220>
<223> oligonucleotide

<400> 75
ccaagggcca cagggacc 18

<210> 76
<211> 18
<212> DNA
<213> Artificial

<220>
<223> oligonucleotide

<400> 76
gtcggaaaaa ccagtacg 18

<210> 77
<211> 18
<212> DNA
<213> Artificial

<220>
<223> oligonucleotide

<400> 77
gcccggtcgg aaaaacca 18

<210> 78
<211> 18
<212> DNA
<213> Artificial

<220>
<223> oligonucleotide

<400> 78
ccggtcggaa aaaccagt 18

<210> 79
<211> 18
<212> DNA
<213> Artificial

<220>
<223> oligonucleotide

<400> 79
cccggtcgga aaaaccag 18

<210> 80
<211> 18
<212> DNA
<213> Artificial

<220>
<223> oligonucleotide

<400> 80
tcggaaaaac cagtacgc 18

<210> 81
<211> 18
<212> DNA
<213> Artificial

<220>
<223> oligonucleotide

<400> 81
cggaaaaacc agtacgcg 18

<210> 82
<211> 18
<212> DNA
<213> Artificial

<220>
<223> oligonucleotide

<400> 82
ggaaaaacca gtacgcgg 18

<210> 83
<211> 18
<212> DNA
<213> Artificial

<220>
<223> oligonucleotide

<400> 83
gtacgcggaa aaatccgg 18

<210> 84
<211> 18
<212> DNA
<213> Artificial

<220>
<223> oligonucleotide

<400> 84
agtacgcgga aaaatccg 18

<210> 85
<211> 18
<212> DNA
<213> Artificial

<220>
<223> oligonucleotide

<400> 85
gcggaaaaat ccggaccg 18

<210> 86
<211> 18
<212> DNA

<213> Artificial

<220>
<223> oligonucleotide

<400> 86
ggtcggaaaa accagtac 18

<210> 87
<211> 18
<212> DNA
<213> Artificial

<220>
<223> oligonucleotide

<400> 87
actcctagtg gtgccctt 18

<210> 88
<211> 18
<212> DNA
<213> Artificial

<220>
<223> oligonucleotide

<400> 88
gctccactcc tagtggtg 18

<210> 89
<211> 18
<212> DNA
<213> Artificial

<220>
<223> oligonucleotide

<400> 89
cactcctagt ggtgccct 18

<210> 90
<211> 18
<212> DNA
<213> Artificial

<220>
<223> oligonucleotide

<400> 90
ctccactcct agtggtgc 18

<210> 91
<211> 18
<212> DNA
<213> Artificial

<220>

<223> oligonucleotide

<400> 91
tccactccta gtggtgcc 18

<210> 92
<211> 18
<212> DNA
<213> Artificial

<220>
<223> oligonucleotide

<400> 92
ccactcctag tggtgccc 18

<210> 93
<211> 18
<212> DNA
<213> Artificial

<220>
<223> oligonucleotide

<400> 93
ggctccactc ctagtggt 18

<210> 94
<211> 18
<212> DNA
<213> Artificial

<220>
<223> oligonucleotide

<400> 94
aggctccact cctagtgg 18

<210> 95
<211> 18
<212> DNA
<213> Artificial

<220>
<223> oligonucleotide

<400> 95
ggcccggtcg gaaaaacc 18

<210> 96
<211> 18
<212> DNA
<213> Artificial

<220>
<223> oligonucleotide

<400> 96

gaaaaaccag tacgcgga 18

<210> 97
<211> 18
<212> DNA
<213> Artificial

<220>
<223> oligonucleotide

<400> 97
cgcggaaaaa tccggacc 18

<210> 98
<211> 18
<212> DNA
<213> Artificial

<220>
<223> oligonucleotide

<400> 98
cagtacgcgg aaaaatcc 18

<210> 99
<211> 18
<212> DNA
<213> Artificial

<220>
<223> oligonucleotide

<400> 99
cggtcggaaa aaccagta 18

<210> 100
<211> 18
<212> DNA
<213> Artificial

<220>
<223> oligonucleotide

<400> 100
aaggcccggt cggaaaaa 18

<210> 101
<211> 18
<212> DNA
<213> Artificial

<220>
<223> oligonucleotide

<400> 101
caggctccac tcctagtg 18

<210> 102

<211> 18
<212> DNA
<213> Artificial

<220>
<223> oligonucleotide

<400> 102
ctcctagtgg tgcccttc 18

<210> 103
<211> 18
<212> DNA
<213> Artificial

<220>
<223> oligonucleotide

<400> 103
tcctagtggt gcccttcc 18

<210> 104
<211> 18
<212> DNA
<213> Artificial

<220>
<223> oligonucleotide

<400> 104
gcaggctcca ctcctagt 18

<210> 105
<211> 18
<212> DNA
<213> Artificial

<220>
<223> oligonucleotide

<400> 105
aggcccggtc ggaaaaac 18

<210> 106
<211> 18
<212> DNA
<213> Artificial

<220>
<223> oligonucleotide

<400> 106
acgcggaaaa atccggac 18

<210> 107
<211> 18
<212> DNA
<213> Artificial

<220>
<223> oligonucleotide

<400> 107
ccagtacgcg gaaaaatc 18

<210> 108
<211> 18
<212> DNA
<213> Artificial

<220>
<223> oligonucleotide

<400> 108
ctagtggtgc ccttccgt 18

<210> 109
<211> 18
<212> DNA
<213> Artificial

<220>
<223> oligonucleotide

<400> 109
gaaaggcccg gtcggaaa 18

<210> 110
<211> 18
<212> DNA
<213> Artificial

<220>
<223> oligonucleotide

<400> 110
aaaggcccgg tcggaaaa 18

<210> 111
<211> 18
<212> DNA
<213> Artificial

<220>
<223> oligonucleotide

<400> 111
tacgcggaaa aatccgga 18

<210> 112
<211> 18
<212> DNA
<213> Artificial

<220>
<223> oligonucleotide

<400> 112
ggaaaggccc ggtcggaa 18

<210> 113
<211> 18
<212> DNA
<213> Artificial

<220>
<223> oligonucleotide

<400> 113
atctcttccg aaaggtcg 18

<210> 114
<211> 18
<212> DNA
<213> Artificial

<220>
<223> oligonucleotide

<400> 114
catctcttcc gaaaggtc 18

<210> 115
<211> 18
<212> DNA
<213> Artificial

<220>
<223> oligonucleotide

<400> 115
ctcttccgaa aggtcgag 18

<210> 116
<211> 18
<212> DNA
<213> Artificial

<220>
<223> oligonucleotide

<400> 116
cttccgaaag gtcgagat 18

<210> 117
<211> 18
<212> DNA
<213> Artificial

<220>
<223> oligonucleotide

<400> 117
tctcttccga aaggtcga 18

<210> 118
<211> 18
<212> DNA
<213> Artificial

<220>
<223> oligonucleotide

<400> 118
tcttccgaaa ggtcgaga 18

<210> 119
<211> 18
<212> DNA
<213> Artificial

<220>
<223> oligonucleotide

<400> 119
cctagtggtg cccttccg 18

<210> 120
<211> 18
<212> DNA
<213> Artificial

<220>
<223> oligonucleotide

<400> 120
tagtggtgcc cttccgtc 18

<210> 121
<211> 18
<212> DNA
<213> Artificial

<220>
<223> oligonucleotide

<400> 121
agtggtgccc ttccgtca 18

<210> 122
<211> 18
<212> DNA
<213> Artificial

<220>
<223> oligonucleotide

<400> 122
gccaaggtta gactcgtt 18

<210> 123
<211> 18
<212> DNA

<213> Artificial

<220>
<223> oligonucleotide

<400> 123
ggccaaggtt agactcgt 18

<210> 124
<211> 18
<212> DNA
<213> Artificial

<220>
<223> oligonucleotide

<400> 124
ccaaggttag actcgttg 18

<210> 125
<211> 18
<212> DNA
<213> Artificial

<220>
<223> oligonucleotide

<400> 125
caaggttaga ctcgttgg 18

<210> 126
<211> 18
<212> DNA
<213> Artificial

<220>
<223> oligonucleotide

<400> 126
aaggttagac tcgttggc 18

<210> 127
<211> 20
<212> DNA
<213> Artificial

<220>
<223> oligonucleotide

<400> 127
ctcgcctcac ggggttctca 20

<210> 128
<211> 18
<212> DNA
<213> Artificial

<220>

<223> oligonucleotide

<400> 128
ggcccggtcg aaattaaa 18

<210> 129
<211> 18
<212> DNA
<213> Artificial

<220>
<223> oligonucleotide

<400> 129
aggcccggtc gaaattaa 18

<210> 130
<211> 18
<212> DNA
<213> Artificial

<220>
<223> oligonucleotide

<400> 130
aaggcccggt cgaaatta 18

<210> 131
<211> 18
<212> DNA
<213> Artificial

<220>
<223> oligonucleotide

<400> 131
aaaggcccgg tcgaaatt 18

<210> 132
<211> 18
<212> DNA
<213> Artificial

<220>
<223> oligonucleotide

<400> 132
gaaaggcccg gtcgaaat 18

<210> 133
<211> 18
<212> DNA
<213> Artificial

<220>
<223> oligonucleotide

<400> 133

atattcgagc gaaacgcc 18

<210> 134
<211> 18
<212> DNA
<213> Artificial

<220>
<223> oligonucleotide

<400> 134
ggaaaggccc ggtcgaaa 18

<210> 135
<211> 18
<212> DNA
<213> Artificial

<220>
<223> oligonucleotide

<400> 135
aaagatccgg accggccg 18

<210> 136
<211> 18
<212> DNA
<213> Artificial

<220>
<223> oligonucleotide

<400> 136
ggaaagatcc ggaccggc 18

<210> 137
<211> 18
<212> DNA
<213> Artificial

<220>
<223> oligonucleotide

<400> 137
gaaagatccg gaccggcc 18

<210> 138
<211> 18
<212> DNA
<213> Artificial

<220>
<223> oligonucleotide

<400> 138
gatccggacc ggccgacc 18

<210> 139

<211> 18
<212> DNA
<213> Artificial

<220>
<223> oligonucleotide

<400> 139
agatccggac cggccgac 18

<210> 140
<211> 18
<212> DNA
<213> Artificial

<220>
<223> oligonucleotide

<400> 140
aagatccgga ccggccga 18

<210> 141
<211> 18
<212> DNA
<213> Artificial

<220>
<223> oligonucleotide

<400> 141
aggaaaggcc cggtcgaa 18

<210> 142
<211> 18
<212> DNA
<213> Artificial

<220>
<223> oligonucleotide

<400> 142
aaggaaaggc ccggtcga 18

<210> 143
<211> 20
<212> DNA
<213> Artificial

<220>
<223> oligonucleotide

<400> 143
cgagcaaaac gcctgctttg 20

<210> 144
<211> 20
<212> DNA
<213> Artificial

<220>
<223> oligonucleotide

<400> 144
cgctctgaaa gagagttgcc 20

<210> 145
<211> 20
<212> DNA
<213> Artificial

<220>
<223> oligonucleotide

<400> 145
agttgccccc tacactagac 20

<210> 146
<211> 19
<212> DNA
<213> Artificial

<220>
<223> oligonucleotide

<400> 146
gcttctccgt cccgcgccg 19

<210> 147
<211> 21
<212> DNA
<213> Artificial

<220>
<223> oligonucleotide

<400> 147
agattytccg ctctgagatg g 21

<210> 148
<211> 19
<212> DNA
<213> Artificial

<220>
<223> oligonucleotide

<400> 148
cctggttcgc caaaaaggc 19

<210> 149
<211> 18
<212> DNA
<213> Artificial

<220>
<223> oligonucleotide

<400> 149
gattctcggc cccatggg 18

<210> 150
<211> 20
<212> DNA
<213> Artificial

<220>
<223> oligonucleotide

<400> 150
accctctacg gcagcctgtt 20

<210> 151
<211> 20
<212> DNA
<213> Artificial

<220>
<223> oligonucleotide

<400> 151
gatcggtctc cagcgattca 20

<210> 152
<211> 20
<212> DNA
<213> Artificial

<220>
<223> oligonucleotide

<400> 152
accctccacg gcggcctgtt 20

<210> 153
<211> 18
<212> DNA
<213> Artificial

<220>
<223> oligonucleotide

<400> 153
gattctccgc gccatggg 18

<210> 154
<211> 20
<212> DNA
<213> Artificial

<220>
<223> oligonucleotide

<400> 154
tcatcagacg ggattctcac 20

<210> 155
<211> 22
<212> DNA
<213> Artificial

<220>
<223> oligonucleotide

<400> 155
ctcatcgcac gggattctca cc 22

<210> 156
<211> 22
<212> DNA
<213> Artificial

<220>
<223> oligonucleotide

<400> 156
ctcgccacac gggattctca cc 22

<210> 157
<211> 20
<212> DNA
<213> Artificial

<220>
<223> oligonucleotide

<400> 157
agttgccccc tcctctaagc 20

<210> 158
<211> 20
<212> DNA
<213> Artificial

<220>
<223> oligonucleotide

<400> 158
ctgccacaag gacaaatggt 20

<210> 159
<211> 21
<212> DNA
<213> Artificial

<220>
<223> oligonucleotide

<400> 159
tgccccctct tctaagcaaa t 21

<210> 160
<211> 18
<212> DNA

<213> Artificial

<220>
<223> oligonucleotide

<400> 160
ccccaaagtt gccctctc 18

<210> 161
<211> 23
<212> DNA
<213> Artificial

<220>
<223> oligonucleotide

<400> 161
gccgccccaa agtcgccctc tac 23

<210> 162
<211> 20
<212> DNA
<213> Artificial

<220>
<223> oligonucleotide

<400> 162
gccccagagt cgccttctac 20

<210> 163
<211> 18
<212> DNA
<213> Artificial

<220>
<223> oligonucleotide

<400> 163
aagaccaggc cacctcat 18

<210> 164
<211> 18
<212> DNA
<213> Artificial

<220>
<223> oligonucleotide

<400> 164
catcatagaa caccgtcc 18

<210> 165
<211> 20
<212> DNA
<213> Artificial

<220>

<223> oligonucleotide

<400> 165
ccttccgaag tcgaggtttt 20

<210> 166
<211> 17
<212> DNA
<213> Artificial

<220>
<223> oligonucleotide

<400> 166
gggagtgttg ccaactc 17

<210> 167
<211> 19
<212> DNA
<213> Artificial

<220>
<223> oligonucleotide

<400> 167
agcggtcgtt cgcaaccct 19

<210> 168
<211> 20
<212> DNA
<213> Artificial

<220>
<223> oligonucleotide

<400> 168
ccgaagtcgg ggttttgcgg 20

<210> 169
<211> 20
<212> DNA
<213> Artificial

<220>
<223> oligonucleotide

<400> 169
gatagccgaa accacctttc 20

<210> 170
<211> 20
<212> DNA
<213> Artificial

<220>
<223> oligonucleotide

<400> 170

gccgaaacca cctttcaaac 20

<210> 171
<211> 20
<212> DNA
<213> Artificial

<220>
<223> oligonucleotide

<400> 171
gtgatagccg aaaccacctt 20

<210> 172
<211> 20
<212> DNA
<213> Artificial

<220>
<223> oligonucleotide

<400> 172
agtgatagcc gaaaccacct 20

<210> 173
<211> 20
<212> DNA
<213> Artificial

<220>
<223> oligonucleotide

<400> 173
tttaacggga tgcgttcgac 20

<210> 174
<211> 20
<212> DNA
<213> Artificial

<220>
<223> oligonucleotide

<400> 174
aagtgatagc cgaaaccacc 20

<210> 175
<211> 20
<212> DNA
<213> Artificial

<220>
<223> oligonucleotide

<400> 175
ggttgaatac cgtcaacgtc 20

<210> 176

<211> 20
<212> DNA
<213> Artificial

<220>
<223> oligonucleotide

<400> 176
gcacagtatg tcaagacctg 20

<210> 177
<211> 20
<212> DNA
<213> Artificial

<220>
<223> oligonucleotide

<400> 177
catccgatgt gcaagcactt 20

<210> 178
<211> 20
<212> DNA
<213> Artificial

<220>
<223> oligonucleotide

<400> 178
tcatccgatg tgcaagcact 20

<210> 179
<211> 20
<212> DNA
<213> Artificial

<220>
<223> oligonucleotide

<400> 179
ccgatgtgca agcacttcat 20

<210> 180
<211> 20
<212> DNA
<213> Artificial

<220>
<223> oligonucleotide

<400> 180
ccactcatcc gatgtgcaag 20

<210> 181
<211> 20
<212> DNA
<213> Artificial

<220>
<223> oligonucleotide

<400> 181
gccacagttc gccactcatc 20

<210> 182
<211> 20
<212> DNA
<213> Artificial

<220>
<223> oligonucleotide

<400> 182
cctccgcgtt tgtcaccggc 20

<210> 183
<211> 20
<212> DNA
<213> Artificial

<220>
<223> oligonucleotide

<400> 183
accagttcgc cacagttcgc 20

<210> 184
<211> 20
<212> DNA
<213> Artificial

<220>
<223> oligonucleotide

<400> 184
cactcatccg atgtgcaagc 20

<210> 185
<211> 20
<212> DNA
<213> Artificial

<220>
<223> oligonucleotide

<400> 185
ccagttcgcc acagttcgcc 20

<210> 186
<211> 20
<212> DNA
<213> Artificial

<220>
<223> oligonucleotide

<400> 186
ctcatccgat gtgcaagcac 20

<210> 187
<211> 20
<212> DNA
<213> Artificial

<220>
<223> oligonucleotide

<400> 187
tccgatgtgc aagcacttca 20

<210> 188
<211> 20
<212> DNA
<213> Artificial

<220>
<223> oligonucleotide

<400> 188
cgccactcat ccgatgtgca 20

<210> 189
<211> 20
<212> DNA
<213> Artificial

<220>
<223> oligonucleotide

<400> 189
cagttcgcca cagttcgcca 20

<210> 190
<211> 20
<212> DNA
<213> Artificial

<220>
<223> oligonucleotide

<400> 190
gccactcatc cgatgtgcaa 20

<210> 191
<211> 20
<212> DNA
<213> Artificial

<220>
<223> oligonucleotide

<400> 191
cgccacagtt cgccactcat 20

<210> 192
<211> 20
<212> DNA
<213> Artificial

<220>
<223> oligonucleotide

<400> 192
atccgatgtg caagcacttc 20

<210> 193
<211> 20
<212> DNA
<213> Artificial

<220>
<223> oligonucleotide

<400> 193
gttcgccaca gttcgccact 20

<210> 194
<211> 20
<212> DNA
<213> Artificial

<220>
<223> oligonucleotide

<400> 194
tcctccgcgt ttgtcaccgg 20

<210> 195
<211> 20
<212> DNA
<213> Artificial

<220>
<223> oligonucleotide

<400> 195
cgecagggtt catcctgagc 20

<210> 196
<211> 20
<212> DNA
<213> Artificial

<220>
<223> oligonucleotide

<400> 196
agttcgccac agttcgccac 20

<210> 197
<211> 20
<212> DNA

<213> Artificial

<220>
<223> oligonucleotide

<400> 197
tcgccacagt tcgccactca 20

<210> 198
<211> 20
<212> DNA
<213> Artificial

<220>
<223> oligonucleotide

<400> 198
ttaacgggat gcgttcgact 20

<210> 199
<211> 20
<212> DNA
<213> Artificial

<220>
<223> oligonucleotide

<400> 199
tcgccactca tccgatgtgc 20

<210> 200
<211> 20
<212> DNA
<213> Artificial

<220>
<223> oligonucleotide

<400> 200
ccacagttcg ccactcatcc 20

<210> 201
<211> 20
<212> DNA
<213> Artificial

<220>
<223> oligonucleotide

<400> 201
gatttaacgg gatgcgttcg 20

<210> 202
<211> 20
<212> DNA
<213> Artificial

<220>

<223> oligonucleotide

<400> 202
taacgggatg cgttcgactt 20

<210> 203
<211> 20
<212> DNA
<213> Artificial

<220>
<223> oligonucleotide

<400> 203
aacgggatgc gttcgacttg 20

<210> 204
<211> 20
<212> DNA
<213> Artificial

<220>
<223> oligonucleotide

<400> 204
cgaaggttac cgaaccgact 20

<210> 205
<211> 20
<212> DNA
<213> Artificial

<220>
<223> oligonucleotide

<400> 205
ccgaaggtta ccgaaccgac 20

<210> 206
<211> 20
<212> DNA
<213> Artificial

<220>
<223> oligonucleotide

<400> 206
cccgaaggtt accgaaccga 20

<210> 207
<211> 20
<212> DNA
<213> Artificial

<220>
<223> oligonucleotide

<400> 207

ttcctccgcg tttgtcaccg 20

<210> 208
<211> 20
<212> DNA
<213> Artificial

<220>
<223> oligonucleotide

<400> 208
ccgccagggt tcatcctgag 20

<210> 209
<211> 20
<212> DNA
<213> Artificial

<220>
<223> oligonucleotide

<400> 209
tccttccaga agtgatagcc 20

<210> 210
<211> 20
<212> DNA
<213> Artificial

<220>
<223> oligonucleotide

<400> 210
caccagttcg ccacagttcg 20

<210> 211
<211> 20
<212> DNA
<213> Artificial

<220>
<223> oligonucleotide

<400> 211
acgggatgcg ttcgacttgc 20

<210> 212
<211> 20
<212> DNA
<213> Artificial

<220>
<223> oligonucleotide

<400> 212
gtccttccag aagtgatagc 20

<210> 213

<211> 20
<212> DNA
<213> Artificial

<220>
<223> oligonucleotide

<400> 213
gccagggttc atcctgagcc 20

<210> 214
<211> 20
<212> DNA
<213> Artificial

<220>
<223> oligonucleotide

<400> 214
actcatccga tgtgcaagca 20

<210> 215
<211> 20
<212> DNA
<213> Artificial

<220>
<223> oligonucleotide

<400> 215
atcattgcct tggtgaaccg 20

<210> 216
<211> 20
<212> DNA
<213> Artificial

<220>
<223> oligonucleotide

<400> 216
tccgcgtttg tcaccggcag 20

<210> 217
<211> 20
<212> DNA
<213> Artificial

<220>
<223> oligonucleotide

<400> 217
tgaaccgtta ctccaccaac 20

<210> 218
<211> 20
<212> DNA
<213> Artificial

<220>
<223> oligonucleotide

<400> 218
gaagtgatag ccgaaaccac 20

<210> 219
<211> 20
<212> DNA
<213> Artificial

<220>
<223> oligonucleotide

<400> 219
ccgcgtttgt caccggcagt 20

<210> 220
<211> 20
<212> DNA
<213> Artificial

<220>
<223> oligonucleotide

<400> 220
ttcgccactc atccgatgtg 20

<210> 221
<211> 20
<212> DNA
<213> Artificial

<220>
<223> oligonucleotide

<400> 221
catttaacgg gatgcgttcg 20

<210> 222
<211> 20
<212> DNA
<213> Artificial

<220>
<223> oligonucleotide

<400> 222
cacagttcgc cactcatccg 20

<210> 223
<211> 20
<212> DNA
<213> Artificial

<220>
<223> oligonucleotide

<400> 223
ttcgccacag ttcgccactc 20

<210> 224
<211> 20
<212> DNA
<213> Artificial

<220>
<223> oligonucleotide

<400> 224
ctccgcgttt gtcaccggca 20

<210> 225
<211> 20
<212> DNA
<213> Artificial

<220>
<223> oligonucleotide

<400> 225
acgccgccag ggttcatcct 20

<210> 226
<211> 20
<212> DNA
<213> Artificial

<220>
<223> oligonucleotide

<400> 226
ccttccagaa gtgatagccg 20

<210> 227
<211> 20
<212> DNA
<213> Artificial

<220>
<223> oligonucleotide

<400> 227
tcattgcctt ggtgaaccgt 20

<210> 228
<211> 20
<212> DNA
<213> Artificial

<220>
<223> oligonucleotide

<400> 228
cacagtatgt caagacctgg 20

<210> 229
<211> 20
<212> DNA
<213> Artificial

<220>
<223> oligonucleotide

<400> 229
ttggtgaacc gttactccac 20

<210> 230
<211> 20
<212> DNA
<213> Artificial

<220>
<223> oligonucleotide

<400> 230
cttggtgaac cgttactcca 20

<210> 231
<211> 20
<212> DNA
<213> Artificial

<220>
<223> oligonucleotide

<400> 231
gtgaaccgtt actccaccaa 20

<210> 232
<211> 20
<212> DNA
<213> Artificial

<220>
<223> oligonucleotide

<400> 232
ggctcccgaa ggttaccgaa 20

<210> 233
<211> 20
<212> DNA
<213> Artificial

<220>
<223> oligonucleotide

<400> 233
gaaggttacc gaaccgactt 20

<210> 234
<211> 20
<212> DNA

<213> Artificial

<220>
<223> oligonucleotide

<400> 234
tggctcccga aggttaccga 20

<210> 235
<211> 20
<212> DNA
<213> Artificial

<220>
<223> oligonucleotide

<400> 235
taatacgccg cgggtccttc 20

<210> 236
<211> 20
<212> DNA
<213> Artificial

<220>
<223> oligonucleotide

<400> 236
gaaccgttac tccaccaact 20

<210> 237
<211> 20
<212> DNA
<213> Artificial

<220>
<223> oligonucleotide

<400> 237
tacgccgcgg gtccttccag 20

<210> 238
<211> 20
<212> DNA
<213> Artificial

<220>
<223> oligonucleotide

<400> 238
tcaccagttc gccacagttc 20

<210> 239
<211> 20
<212> DNA
<213> Artificial

<220>

<223> oligonucleotide

<400> 239
ccttggtgaa ccgttactcc 20

<210> 240
<211> 20
<212> DNA
<213> Artificial

<220>
<223> oligonucleotide

<400> 240
ctcaccagtt cgccacagtt 20

<210> 241
<211> 20
<212> DNA
<213> Artificial

<220>
<223> oligonucleotide

<400> 241
cgccgccagg gttcatcctg 20

<210> 242
<211> 20
<212> DNA
<213> Artificial

<220>
<223> oligonucleotide

<400> 242
ccttggtgaa ccattactcc 20

<210> 243
<211> 20
<212> DNA
<213> Artificial

<220>
<223> oligonucleotide

<400> 243
tggtgaacca ttactccacc 20

<210> 244
<211> 20
<212> DNA
<213> Artificial

<220>
<223> oligonucleotide

<400> 244

gccgccaggg ttcatcctga 20

<210> 245
<211> 20
<212> DNA
<213> Artificial

<220>
<223> oligonucleotide

<400> 245
ggtgaaccat tactccacca 20

<210> 246
<211> 20
<212> DNA
<213> Artificial

<220>
<223> oligonucleotide

<400> 246
ccagggttca tcctgagcca 20

<210> 247
<211> 20
<212> DNA
<213> Artificial

<220>
<223> oligonucleotide

<400> 247
aatacgccgc gggtccttcc 20

<210> 248
<211> 20
<212> DNA
<213> Artificial

<220>
<223> oligonucleotide

<400> 248
cacgccgcca gggttcatcc 20

<210> 249
<211> 20
<212> DNA
<213> Artificial

<220>
<223> oligonucleotide

<400> 249
agttcgccac tcatccgatg 20

<210> 250

<211> 20
<212> DNA
<213> Artificial

<220>
<223> oligonucleotide

<400> 250
cgggatgcgt tcgacttgca 20

<210> 251
<211> 20
<212> DNA
<213> Artificial

<220>
<223> oligonucleotide

<400> 251
cattgccttg gtgaaccgtt 20

<210> 252
<211> 20
<212> DNA
<213> Artificial

<220>
<223> oligonucleotide

<400> 252
gcacgccgcc agggttcatc 20

<210> 253
<211> 20
<212> DNA
<213> Artificial

<220>
<223> oligonucleotide

<400> 253
cttcctccgc gtttgtcacc 20

<210> 254
<211> 20
<212> DNA
<213> Artificial

<220>
<223> oligonucleotide

<400> 254
tggtgaaccg ttactccacc 20

<210> 255
<211> 20
<212> DNA
<213> Artificial

<220>
<223> oligonucleotide

<400> 255
cettcctccg cgtttgtcac 20

<210> 256
<211> 20
<212> DNA
<213> Artificial

<220>
<223> oligonucleotide

<400> 256
acgccgcggg tccttccaga 20

<210> 257
<211> 20
<212> DNA
<213> Artificial

<220>
<223> oligonucleotide

<400> 257
ggtgaaccgt tactccacca 20

<210> 258
<2.11> 20
<212> DNA
<213> Artificial

<220>
<223> oligonucleotide

<400> 258
gggtccttcc agaagtgata 20

<210> 259
<211> 20
<212> DNA
<213> Artificial

<220>
<223> oligonucleotide

<400> 259
cttccagaag tgatagccga 20

<210> 260
<211> 20
<212> DNA
<213> Artificial

<220>
<223> oligonucleotide

<400> 260
gccttggtga accattactc 20

<210> 261
<211> 20
<212> DNA
<213> Artificial

<220>
<223> oligonucleotide

<400> 261
acagttcgcc actcatccga 20

<210> 262
<211> 20
<212> DNA
<213> Artificial

<220>
<223> oligonucleotide

<400> 262
accttcctcc gcgtttgtca 20

<210> 263
<211> 20
<212> DNA
<213> Artificial

<220>
<223> oligonucleotide

<400> 263
cgaaccgact ttgggtgttg 20

<210> 264
<211> 20
<212> DNA
<213> Artificial

<220>
<223> oligonucleotide

<400> 264
gaaccgactt tgggtgttgc 20

<210> 265
<211> 20
<212> DNA
<213> Artificial

<220>
<223> oligonucleotide

<400> 265
aggttaccga accgactttg 20

<210> 266
<211> 20
<212> DNA
<213> Artificial

<220>
<223> oligonucleotide

<400> 266
accgaaccga ctttgggtgt 20

<210> 267
<211> 20
<212> DNA
<213> Artificial

<220>
<223> oligonucleotide

<400> 267
ttaccgaacc gactttgggt 20

<210> 268
<211> 20
<212> DNA
<213> Artificial

<220>
<223> oligonucleotide

<400> 268
taccgaaccg actttgggtg 20

<210> 269
<211> 20
<212> DNA
<213> Artificial

<220>
<223> oligonucleotide

<400> 269
gttaccgaac cgactttggg 20

<210> 270
<211> 21
<212> DNA
<213> Artificial

<220>
<223> oligonucleotide

<400> 270
cctttctggt atggtaccgt c 21

<210> 271
<211> 20
<212> DNA

<213> Artificial

<220>
<223> oligonucleotide

<400> 271
tgcaccgcgg ayccatctct 20

<210> 272
<211> 20
<212> DNA
<213> Artificial

<220>
<223> oligonucleotide

<400> 272
agttgcagtc cagtaagccg 20

<210> 273
<211> 20
<212> DNA
<213> Artificial

<220>
<223> oligonucleotide

<400> 273
gttgcagtcc agtaagccgc 20

<210> 274
<211> 20
<212> DNA
<213> Artificial

<220>
<223> oligonucleotide

<400> 274
cagttgcagt ccagtaagcc 20

<210> 275
<211> 20
<212> DNA
<213> Artificial

<220>
<223> oligonucleotide

<400> 275
tgcagtccag taagccgcct 20

<210> 276
<211> 20
<212> DNA
<213> Artificial

<220>

<223> oligonucleotide

<400> 276
tcagttgcag tccagtaagc 20

<210> 277
<211> 20
<212> DNA
<213> Artificial

<220>
<223> oligonucleotide

<400> 277
ttgcagtcca gtaagccgcc 20

<210> 278
<211> 20
<212> DNA
<213> Artificial

<220>
<223> oligonucleotide

<400> 278
gcagtccagt aagccgcctt 20

<210> 279
<211> 20
<212> DNA
<213> Artificial

<220>
<223> oligonucleotide

<400> 279
gtcagttgca gtccagtaag 20

<210> 280
<211> 20
<212> DNA
<213> Artificial

<220>
<223> oligonucleotide

<400> 280
ctctaggtga cgccgaagcg 20

<210> 281
<211> 20
<212> DNA
<213> Artificial

<220>
<223> oligonucleotide

<400> 281

atctctaggt gacgccgaag 20

<210> 282
<211> 20
<212> DNA
<213> Artificial

<220>
<223> oligonucleotide

<400> 282
tctaggtgac gccgaagcgc 20

<210> 283
<211> 20
<212> DNA
<213> Artificial

<220>
<223> oligonucleotide

<400> 283
tctctaggtg acgccgaagc 20

<210> 284
<211> 20
<212> DNA
<213> Artificial

<220>
<223> oligonucleotide

<400> 284
ccatctctag gtgacgccga 20

<210> 285
<211> 20
<212> DNA
<213> Artificial

<220>
<223> oligonucleotide

<400> 285
catctctagg tgacgccgaa 20

<210> 286
<211> 20
<212> DNA
<213> Artificial

<220>
<223> oligonucleotide

<400> 286
taggtgacgc cgaagcgcct 20

<210> 287

<211> 20
<212> DNA
<213> Artificial

<220>
<223> oligonucleotide

<400> 287
ctaggtgacg ccgaagcgcc 20

<210> 288
<211> 20
<212> DNA
<213> Artificial

<220>
<223> oligonucleotide

<400> 288
cttagacggc tccttcctaa 20

<210> 289
<211> 20
<212> DNA
<213> Artificial

<220>
<223> oligonucleotide

<400> 289
ccttagacgg ctccttccta 20

<210> 290
<211> 20
<212> DNA
<213> Artificial

<220>
<223> oligonucleotide

<400> 290
acgtcagttg cagtccagta 20

<210> 291
<211> 20
<212> DNA
<213> Artificial

<220>
<223> oligonucleotide

<400> 291
cgtcagttgc agtccagtaa 20

<210> 292
<211> 20
<212> DNA
<213> Artificial

<220>
<223> oligonucleotide

<400> 292
acgccgaagc gcctttttaac 20

<210> 293
<211> 20
<212> DNA
<213> Artificial

<220>
<223> oligonucleotide

<400> 293
gacgccgaag cgccttttaa 20

<210> 294
<211> 20
<212> DNA
<213> Artificial

<220>
<223> oligonucleotide

<400> 294
gccgaagcgc cttttaactt 20

<210> 295
<211> 20
<212> DNA
<213> Artificial

<220>
<223> oligonucleotide

<400> 295
cgccgaagcg ccttttaact 20

<210> 296
<211> 20
<212> DNA
<213> Artificial

<220>
<223> oligonucleotide

<400> 296
gtgacgccga agcgcctttt 20

<210> 297
<211> 20
<212> DNA
<213> Artificial

<220>
<223> oligonucleotide

<400> 297
tgacgccgaa gcgcctttta 20

<210> 298
<211> 20
<212> DNA
<213> Artificial

<220>
<223> oligonucleotide

<400> 298
agacggctcc ttcctaaaag 20

<210> 299
<211> 20
<212> DNA
<213> Artificial

<220>
<223> oligonucleotide

<400> 299
acggctcctt cctaaaaggt 20

<210> 300
<211> 20
<212> DNA
<213> Artificial

<220>
<223> oligonucleotide

<400> 300
gacggctcct tcctaaaagg 20

<210> 301
<211> 20
<212> DNA
<213> Artificial

<220>
<223> oligonucleotide

<400> 301
ccttcctaaa aggttaggcc 20

<210> 302
<211> 20
<212> DNA
<213> Artificial

<220>
<223> oligonucleotide

<400> 302
ggtgacgcca aagcgccttt 20

<210> 303
<211> 20
<212> DNA
<213> Artificial

<220>
<223> oligonucleotide

<400> 303
aggtgacgcc aaagcgcctt 20

<210> 304
<211> 20
<212> DNA
<213> Artificial

<220>
<223> oligonucleotide

<400> 304
taggtgacgc caaagcgcct 20

<210> 305
<211> 20
<212> DNA
<213> Artificial

<220>
<223> oligonucleotide

<400> 305
ctctaggtga cgccaaagcg 20

<210> 306
<211> 20
<212> DNA
<213> Artificial

<220>
<223> oligonucleotide

<400> 306
tctaggtgac gccaaagcgc 20

<210> 307
<211> 20
<212> DNA
<213> Artificial

<220>
<223> oligonucleotide

<400> 307
ctaggtgacg ccaaagcgcc 20

<210> 308
<211> 20
<212> DNA

<213> Artificial

<220>
<223> oligonucleotide

<400> 308
acgccaaagc gccttttaac 20

<210> 309
<211> 20
<212> DNA
<213> Artificial

<220>
<223> oligonucleotide

<400> 309
cgccaaagcg ccttttaact 20

<210> 310
<211> 20
<212> DNA
<213> Artificial

<220>
<223> oligonucleotide

<400> 310
tgacgccaaa gcgcctttta 20

<210> 311
<211> 20
<212> DNA
<213> Artificial

<220>
<223> oligonucleotide

<400> 311
tctctaggtg acgccaaagc 20

<210> 312
<211> 20
<212> DNA
<213> Artificial

<220>
<223> oligonucleotide

<400> 312
gtgacgccaa agcgcctttt 20

<210> 313
<211> 20
<212> DNA
<213> Artificial

<220>

<223> oligonucleotide

<400> 313
gacgccaaag cgccttttaa 20

<210> 314
<211> 20
<212> DNA
<213> Artificial

<220>
<223> oligonucleotide

<400> 314
atctctaggt gacgccaaag 20

<210> 315
<211> 20
<212> DNA
<213> Artificial

<220>
<223> oligonucleotide

<400> 315
catctctagg tgacgccaaa 20

<210> 316
<211> 20
<212> DNA
<213> Artificial

<220>
<223> oligonucleotide

<400> 316
tccatctcta ggtgacgcca 20

<210> 317
<211> 20
<212> DNA
<213> Artificial

<220>
<223> oligonucleotide

<400> 317
ccatctctag gtgacgccaa 20

<210> 318
<211> 20
<212> DNA
<213> Artificial

<220>
<223> oligonucleotide

<400> 318

ctgccttaga cggctccccc 20

<210> 319
<211> 20
<212> DNA
<213> Artificial

<220>
<223> oligonucleotide

<400> 319
cctgccttag acggctcccc 20

<210> 320
<211> 20
<212> DNA
<213> Artificial

<220>
<223> oligonucleotide

<400> 320
gtgtcatgcg acactgagtt 20

<210> 321
<211> 20
<212> DNA
<213> Artificial

<220>
<223> oligonucleotide

<400> 321
tgtgtcatgc gacactgagt 20

<210> 322
<211> 20
<212> DNA
<213> Artificial

<220>
<223> oligonucleotide

<400> 322
ctttgtgtca tgcgacactg 20

<210> 323
<211> 20
<212> DNA
<213> Artificial

<220>
<223> oligonucleotide

<400> 323
ttgtgtcatg cgacactgag 20

<210> 324

<211> 20
<212> DNA
<213> Artificial

<220>
<223> oligonucleotide

<400> 324
tgccttagac ggctccccct 20

<210> 325
<211> 20
<212> DNA
<213> Artificial

<220>
<223> oligonucleotide

<400> 325
agacggctcc ccctaaaagg 20

<210> 326
<211> 20
<212> DNA
<213> Artificial

<220>
<223> oligonucleotide

<400> 326
tagacggctc cccctaaaag 20

<210> 327
<211> 20
<212> DNA
<213> Artificial

<220>
<223> oligonucleotide

<400> 327
gccttagacg gctcccccta 20

<210> 328
<211> 20
<212> DNA
<213> Artificial

<220>
<223> oligonucleotide

<400> 328
gctcccccta aaaggttagg 20

<210> 329
<211> 20
<212> DNA
<213> Artificial

<220>
<223> oligonucleotide

<400> 329
ggctccccct aaaaggttag 20

<210> 330
<211> 20
<212> DNA
<213> Artificial

<220>
<223> oligonucleotide

<400> 330
ctccccctaa aaggttaggc 20

<210> 331
<211> 20
<212> DNA
<213> Artificial

<220>
<223> oligonucleotide

<400> 331
tccccctaaa aggttaggcc 20

<210> 332
<211> 20
<212> DNA
<213> Artificial

<220>
<223> oligonucleotide

<400> 332
ccctaaaagg ttaggccacc 20

<210> 333
<211> 20
<212> DNA
<213> Artificial

<220>
<223> oligonucleotide

<400> 333
cccctaaaag gttaggccac 20

<210> 334
<211> 20
<212> DNA
<213> Artificial

<220>
<223> oligonucleotide

<400> 334
cggctccccc taaaaggtta 20

<210> 335
<211> 20
<212> DNA
<213> Artificial

<220>
<223> oligonucleotide

<400> 335
ccccctaaaa ggttaggcca 20

<210> 336
<211> 20
<212> DNA
<213> Artificial

<220>
<223> oligonucleotide

<400> 336
cttagacggc tccccctaaa 20

<210> 337
<211> 20
<212> DNA
<213> Artificial

<220>
<223> oligonucleotide

<400> 337
ttagacggct ccccctaaaa 20

<210> 338
<211> 20
<212> DNA
<213> Artificial

<220>
<223> oligonucleotide

<400> 338
gggttcgcaa ctcgttgtat 20

<210> 339
<211> 20
<212> DNA
<213> Artificial

<220>
<223> oligonucleotide

<400> 339
ccttagacgg ctccccctaa 20

<210> 340
<211> 20
<212> DNA
<213> Artificial

<220>
<223> oligonucleotide

<400> 340
acggctcccc ctaaaaggtt 20

<210> 341
<211> 20
<212> DNA
<213> Artificial

<220>
<223> oligonucleotide

<400> 341
gacggctccc cctaaaaggt 20

<210> 342
<211> 20
<212> DNA
<213> Artificial

<220>
<223> oligonucleotide

<400> 342
acgccgcaag accatcctct 20

<210> 343
<211> 20
<212> DNA
<213> Artificial

<220>
<223> oligonucleotide

<400> 343
ctaatacgcc gcaagaccat 20

<210> 344
<211> 20
<212> DNA
<213> Artificial

<220>
<223> oligonucleotide

<400> 344
tacgccgcaa gaccatcctc 20

<210> 345
<211> 20
<212> DNA

<213> Artificial

<220>
<223> oligonucleotide

<400> 345
gttacgatct agcaagccgc 20

<210> 346
<211> 20
<212> DNA
<213> Artificial

<220>
<223> oligonucleotide

<400> 346
aatacgccgc aagaccatcc 20

<210> 347
<211> 20
<212> DNA
<213> Artificial

<220>
<223> oligonucleotide

<400> 347
cgccgcaaga ccatcctcta 20

<210> 348
<211> 20
<212> DNA
<213> Artificial

<220>
<223> oligonucleotide

<400> 348
gctaatacgc cgcaagacca 20

<210> 349
<211> 20
<212> DNA
<213> Artificial

<220>
<223> oligonucleotide

<400> 349
accatcctct agcgatccaa 20

<210> 350
<211> 20
<212> DNA
<213> Artificial

<220>

<223> oligonucleotide

<400> 350
taatacgccg caagaccatc 20

<210> 351
<211> 20
<212> DNA
<213> Artificial

<220>
<223> oligonucleotide

<400> 351
agccatccct ttctggtaag 20

<210> 352
<211> 20
<212> DNA
<213> Artificial

<220>
<223> oligonucleotide

<400> 352
atacgccgca agaccatcct 20

<210> 353
<211> 20
<212> DNA
<213> Artificial

<220>
<223> oligonucleotide

<400> 353
agttacgatc tagcaagccg 20

<210> 354
<211> 20
<212> DNA
<213> Artificial

<220>
<223> oligonucleotide

<400> 354
agctaatacg ccgcaagacc 20

<210> 355
<211> 20
<212> DNA
<213> Artificial

<220>
<223> oligonucleotide

<400> 355

gccgcaagac catcctctag 20

<210> 356
<211> 20
<212> DNA
<213> Artificial

<220>
<223> oligonucleotide

<400> 356
ttacgatcta gcaagccgct 20

<210> 357
<211> 20
<212> DNA
<213> Artificial

<220>
<223> oligonucleotide

<400> 357
gaccatcctc tagcgatcca 20

<210> 358
<211> 20
<212> DNA
<213> Artificial

<220>
<223> oligonucleotide

<400> 358
ttgctacgtc actaggaggc 20

<210> 359
<211> 20
<212> DNA
<213> Artificial

<220>
<223> oligonucleotide

<400> 359
acgtcactag gaggcggaaa 20

<210> 360
<211> 20
<212> DNA
<213> Artificial

<220>
<223> oligonucleotide

<400> 360
tttgctacgt cactaggagg 20

<210> 361

<211> 20
<212> DNA
<213> Artificial

<220>
<223> oligonucleotide

<400> 361
gccatccctt tctggtaagg 20

<210> 362
<211> 20
<212> DNA
<213> Artificial

<220>
<223> oligonucleotide

<400> 362
tacgtcacta ggaggcggaa 20

<210> 363
<211> 20
<212> DNA
<213> Artificial

<220>
<223> oligonucleotide

<400> 363
cgtcactagg aggcggaaac 20

<210> 364
<211> 20
<212> DNA
<213> Artificial

<220>
<223> oligonucleotide

<400> 364
aagaccatcc tctagcgatc 20

<210> 365
<211> 20
<212> DNA
<213> Artificial

<220>
<223> oligonucleotide

<400> 365
gcacgtattt agccatccct 20

<210> 366
<211> 20
<212> DNA
<213> Artificial

<220>
<223> oligonucleotide

<400> 366
ctctagcgat ccaaaaggac 20

<210> 367
<211> 20
<212> DNA
<213> Artificial

<220>
<223> oligonucleotide

<400> 367
cctctagcga tccaaaagga 20

<210> 368
<211> 20
<212> DNA
<213> Artificial

<220>
<223> oligonucleotide

<400> 368
ccatcctcta gcgatccaaa 20

<210> 369
<211> 20
<212> DNA
<213> Artificial

<220>
<223> oligonucleotide

<400> 369
ggcacgtatt tagccatccc 20

<210> 370
<211> 20
<212> DNA
<213> Artificial

<220>
<223> oligonucleotide

<400> 370
tacgatctag caagccgctt 20

<210> 371
<211> 20
<212> DNA
<213> Artificial

<220>
<223> oligonucleotide

<400> 371
cagttacgat ctagcaagcc 20

<210> 372
<211> 20
<212> DNA
<213> Artificial

<220>
<223> oligonucleotide

<400> 372
ccgcaagacc atcctctagc 20

<210> 373
<211> 20
<212> DNA
<213> Artificial

<220>
<223> oligonucleotide

<400> 373
ccatcccttt ctggtaaggt 20

<210> 374
<211> 20
<212> DNA
<213> Artificial

<220>
<223> oligonucleotide

<400> 374
agaccatcct ctagcgatcc 20

<210> 375
<211> 20
<212> DNA
<213> Artificial

<220>
<223> oligonucleotide

<400> 375
caagaccatc ctctagcgat 20

<210> 376
<211> 20
<212> DNA
<213> Artificial

<220>
<223> oligonucleotide

<400> 376
gctacgtcac taggaggcgg 20

<210> 377
<211> 20
<212> DNA
<213> Artificial

<220>
<223> oligonucleotide

<400> 377
tgctacgtca ctaggaggcg 20

<210> 378
<211> 20
<212> DNA
<213> Artificial

<220>
<223> oligonucleotide

<400> 378
ctacgtcact aggaggcgga 20

<210> 379
<211> 20
<212> DNA
<213> Artificial

<220>
<223> oligonucleotide

<400> 379
cctcaacgtc agttacgatc 20

<210> 380
<211> 20
<212> DNA
<213> Artificial

<220>
<223> oligonucleotide

<400> 380
gtcactagga ggcggaaacc 20

<210> 381
<211> 20
<212> DNA
<213> Artificial

<220>
<223> oligonucleotide

<400> 381
tcctctagcg atccaaaagg 20

<210> 382
<211> 20
<212> DNA

<213> Artificial

<220>
<223> oligonucleotide

<400> 382
tggcacgtat ttagccatcc 20

<210> 383
<211> 20
<212> DNA
<213> Artificial

<220>
<223> oligonucleotide

<400> 383
acgatctagc aagccgcttt 20

<210> 384
<211> 20
<212> DNA
<213> Artificial

<220>
<223> oligonucleotide

<400> 384
gccagtctct caactcggct 20

<210> 385
<211> 20
<212> DNA
<213> Artificial

<220>
<223> oligonucleotide

<400> 385
aagctaatac gccgcaagac 20

<210> 386
<211> 20
<212> DNA
<213> Artificial

<220>
<223> oligonucleotide

<400> 386
gtttgctacg tcactaggag 20

<210> 387
<211> 20
<212> DNA
<213> Artificial

<220>

<223> oligonucleotide

<400> 387
cgccactcta gtcattgcct 20

<210> 388
<211> 20
<212> DNA
<213> Artificial

<220>
<223> oligonucleotide

<400> 388
ggccagccag tctctcaact 20

<210> 389
<211> 20
<212> DNA
<213> Artificial

<220>
<223> oligonucleotide

<400> 389
cagccagtct ctcaactcgg 20

<210> 390
<211> 20
<212> DNA
<213> Artificial

<220>
<223> oligonucleotide

<400> 390
cccgaagatc aattcagcgg 20

<210> 391
<211> 20
<212> DNA
<213> Artificial

<220>
<223> oligonucleotide

<400> 391
ccggccagtc tctcaactcg 20

<210> 392
<211> 20
<212> DNA
<213> Artificial

<220>
<223> oligonucleotide

<400> 392

ccagccagtc tctcaactcg 20

<210> 393
<211> 20
<212> DNA
<213> Artificial

<220>
<223> oligonucleotide

<400> 393
tcattgcctc acttcacccg 20

<210> 394
<211> 20
<212> DNA
<213> Artificial

<220>
<223> oligonucleotide

<400> 394
gccagccagt ctctcaactc 20

<210> 395
<211> 20
<212> DNA
<213> Artificial

<220>
<223> oligonucleotide

<400> 395
cacccgaaga tcaattcagc 20

<210> 396
<211> 20
<212> DNA
<213> Artificial

<220>
<223> oligonucleotide

<400> 396
gtcattgcct cacttcaccc 20

<210> 397
<211> 20
<212> DNA
<213> Artificial

<220>
<223> oligonucleotide

<400> 397
cattgcctca cttcacccga 20

<210> 398

<211> 20
<212> DNA
<213> Artificial

<220>
<223> oligonucleotide

<400> 398
attgcctcac ttcacccgaa 20

<210> 399
<211> 20
<212> DNA
<213> Artificial

<220>
<223> oligonucleotide

<400> 399
cgaagatcaa ttcagcggct 20

<210> 400
<211> 20
<212> DNA
<213> Artificial

<220>
<223> oligonucleotide

<400> 400
agtcattgcc tcacttcacc 20

<210> 401
<211> 20
<212> DNA
<213> Artificial

<220>
<223> oligonucleotide

<400> 401
tcgccactct agtcattgcc 20

<210> 402
<211> 20
<212> DNA
<213> Artificial

<220>
<223> oligonucleotide

<400> 402
ttgcctcact tcacccgaag 20

<210> 403
<211> 20
<212> DNA
<213> Artificial

<220>
<223> oligonucleotide

<400> 403
cggccagtct ctcaactcgg 20

<210> 404
<211> 20
<212> DNA
<213> Artificial

<220>
<223> oligonucleotide

<400> 404
ctggcacgta tttagccatc 20

<210> 405
<211> 20
<212> DNA
<213> Artificial

<220>
<223> oligonucleotide

<400> 405
acccgaagat caattcagcg 20

<210> 406
<211> 20
<212> DNA
<213> Artificial

<220>
<223> oligonucleotide

<400> 406
tctagcgatc caaaaggacc 20

<210> 407
<211> 20
<212> DNA
<213> Artificial

<220>
<223> oligonucleotide

<400> 407
ctagcgatcc aaaaggacct 20

<210> 408
<211> 20
<212> DNA
<213> Artificial

<220>
<223> oligonucleotide

<400> 408
gcacccatcg tttacggtat 20

<210> 409
<211> 20
<212> DNA
<213> Artificial

<220>
<223> oligonucleotide

<400> 409
cacccatcgt ttacggtatg 20

<210> 410
<211> 20
<212> DNA
<213> Artificial

<220>
<223> oligonucleotide

<400> 410
gccactctag tcattgcctc 20

<210> 411
<211> 20
<212> DNA
<213> Artificial

<220>
<223> oligonucleotide

<400> 411
cgtttgctac gtcactagga 20

<210> 412
<211> 20
<212> DNA
<213> Artificial

<220>
<223> oligonucleotide

<400> 412
gcctcaacgt cagttacgat 20

<210> 413
<211> 20
<212> DNA
<213> Artificial

<220>
<223> oligonucleotide

<400> 413
gccggccagt ctctcaactc 20

<210> 414
<211> 20
<212> DNA
<213> Artificial

<220>
<223> oligonucleotide

<400> 414
tcactaggag gcggaaacct 20

<210> 415
<211> 20
<212> DNA
<213> Artificial

<220>
<223> oligonucleotide

<400> 415
agcctcaacg tcagttacga 20

<210> 416
<211> 20
<212> DNA
<213> Artificial

<220>
<223> oligonucleotide

<400> 416
agccagtctc tcaactcggc 20

<210> 417
<211> 20
<212> DNA
<213> Artificial

<220>
<223> oligonucleotide

<400> 417
ggccagtctc tcaactcggc 20

<210> 418
<211> 20
<212> DNA
<213> Artificial

<220>
<223> oligonucleotide

<400> 418
caagctaata cgccgcaaga 20

<210> 419
<211> 20
<212> DNA

<213> Artificial

<220>
<223> oligonucleotide

<400> 419
ttcgccactc tagtcattgc 20

<210> 420
<211> 20
<212> DNA
<213> Artificial

<220>
<223> oligonucleotide

<400> 420
ccgaagatca attcagcggc 20

<210> 421
<211> 20
<212> DNA
<213> Artificial

<220>
<223> oligonucleotide

<400> 421
cgcaagacca tcctctagcg 20

<210> 422
<211> 20
<212> DNA
<213> Artificial

<220>
<223> oligonucleotide

<400> 422
gcaagaccat cctctagcga 20

<210> 423
<211> 20
<212> DNA
<213> Artificial

<220>
<223> oligonucleotide

<400> 423
gcgtttgcta cgtcactagg 20

<210> 424
<211> 20
<212> DNA
<213> Artificial

<220>

<223> oligonucleotide

<400> 424
ccactctagt cattgcctca 20

<210> 425
<211> 20
<212> DNA
<213> Artificial

<220>
<223> oligonucleotide

<400> 425
cactctagtc attgcctcac 20

<210> 426
<211> 20
<212> DNA
<213> Artificial

<220>
<223> oligonucleotide

<400> 426
ccagtctctc aactcggcta 20

<210> 427
<211> 20
<212> DNA
<213> Artificial

<220>
<223> oligonucleotide

<400> 427
ttaccttagg caccggcctc 20

<210> 428
<211> 20
<212> DNA
<213> Artificial

<220>
<223> oligonucleotide

<400> 428
acaagctaat acgccgcaag 20

<210> 429
<211> 20
<212> DNA
<213> Artificial

<220>
<223> oligonucleotide

<400> 429

tttaccttag gcaccggcct 20

<210> 430
<211> 20
<212> DNA
<213> Artificial

<220>
<223> oligonucleotide

<400> 430
ttttacctta ggcaccggcc 20

<210> 431
<211> 20
<212> DNA
<213> Artificial

<220>
<223> oligonucleotide

<400> 431
attttacctt aggcaccggc 20

<210> 432
<211> 20
<212> DNA
<213> Artificial

<220>
<223> oligonucleotide

<400> 432
gattttacct taggcaccgg 20

<210> 433
<211> 20
<212> DNA
<213> Artificial

<220>
<223> oligonucleotide

<400> 433
ctcacttcac ccgaagatca 20

<210> 434
<211> 20
<212> DNA
<213> Artificial

<220>
<223> oligonucleotide

<400> 434
acgccaccag cgttcatcct 20

<210> 435

<211> 20
<212> DNA
<213> Artificial

<220>
<223> oligonucleotide

<400> 435
gccaagcgac tttgggtact 20

<210> 436
<211> 20
<212> DNA
<213> Artificial

<220>
<223> oligonucleotide

<400> 436
cggaaaattc cctactgcag 20

<210> 437
<211> 20
<212> DNA
<213> Artificial

<220>
<223> oligonucleotide

<400> 437
cgatctagca agccgctttc 20

<210> 438
<211> 20
<212> DNA
<213> Artificial

<220>
<223> oligonucleotide

<400> 438
ggtaccgtca agctgaaaac 20

<210> 439
<211> 20
<212> DNA
<213> Artificial

<220>
<223> oligonucleotide

<400> 439
tgcctcactt cacccgaaga 20

<210> 440
<211> 20
<212> DNA
<213> Artificial

<220>
<223> oligonucleotide

<400> 440
ggccggccag tctctcaact 20

<210> 441
<211> 20
<212> DNA
<213> Artificial

<220>
<223> oligonucleotide

<400> 441
ggtaaggtac cgtcaagctg 20

<210> 442
<211> 20
<212> DNA
<213> Artificial

<220>
<223> oligonucleotide

<400> 442
gtaaggtacc gtcaagctga 20

<210> 443
<211> 20
<212> DNA
<213> Artificial

<220>
<223> oligonucleotide

<400> 443
ccgcaagacc atcctctagg 20

<210> 444
<211> 20
<212> DNA
<213> Artificial

<220>
<223> oligonucleotide

<400> 444
atttagccat ccctttctgg 20

<210> 445
<211> 18
<212> DNA
<213> Artificial

<220>
<223> oligonucleotide

<400> 445
aacccttcat cacacacg 18

<210> 446
<211> 18
<212> DNA
<213> Artificial

<220>
<223> oligonucleotide

<400> 446
cgaaaccctt catcacac 18

<210> 447
<211> 18
<212> DNA
<213> Artificial

<220>
<223> oligonucleotide

<400> 447
acccttcatc acacacgc 18

<210> 448
<211> 18
<212> DNA
<213> Artificial

<220>
<223> oligonucleotide

<400> 448
taccgtcaca cactgaac 18

<210> 449
<211> 18
<212> DNA
<213> Artificial

<220>
<223> oligonucleotide

<400> 449
agataccgtc acacactg 18

<210> 450
<211> 18
<212> DNA
<213> Artificial

<220>
<223> oligonucleotide

<400> 450
cactcaaggg cggaaacc 18

<210> 451
<211> 18
<212> DNA
<213> Artificial

<220>
<223> oligonucleotide

<400> 451
accgtcacac actgaaca 18

<210> 452
<211> 18
<212> DNA
<213> Artificial

<220>
<223> oligonucleotide

<400> 452
cgtcacacac tgaacagt 18

<210> 453
<211> 18
<212> DNA
<213> Artificial

<220>
<223> oligonucleotide

<400> 453 18
ccgaaaccct tcatcaca 18

<210> 454
<211> 18
<212> DNA
<213> Artificial

<220>
<223> oligonucleotide

<400> 454 18
ccgtcacaca ctgaacag 18

<210> 455
<211> 18
<212> DNA
<213> Artificial

<220>
<223> oligonucleotide

<400> 455
gataccgtca cacactga 18

<210> 456
<211> 18
<212> DNA

<213> Artificial

<220>
<223> oligonucleotide

<400> 456 18
ggtaagatac cgtcacac 18

<210> 457
<211> 18
<212> DNA
<213> Artificial

<220>
<223> oligonucleotide

<400> 457 18
cccttcatca cacacgcg 18

<210> 458
<211> 18
<212> DNA
<213> Artificial

<220>
<223> oligonucleotide

<400> 458
acagtgtttt acgagccg 18

<210> 459
<211> 18
<212> DNA
<213> Artificial

<220>
<223> oligonucleotide

<400> 459
cagtgtttta cgagccga 18

<210> 460
<211> 18
<212> DNA
<213> Artificial

<220>
<223> oligonucleotide

<400> 460
acaaagcgtt cgacttgc 18

<210> 461
<211> 18
<212> DNA
<213> Artificial

<220>

<223> oligonucleotide

<400> 461
cggataacgc ttggaaca 18

<210> 462
<211> 18
<212> DNA
<213> Artificial

<220>
<223> oligonucleotide

<400> 462
agggcggaaa ccctcgaa 18

<210> 463
<211> 18
<212> DNA
<213> Artificial

<220>
<223> oligonucleotide

<400> 463
gggcggaaac cctcgaac 18

<210> 464
<211> 18
<212> DNA
<213> Artificial

<220>
<223> oligonucleotide

<400> 464
ggcggaaacc ctcgaaca 18

<210> 465
<211> 18
<212> DNA
<213> Artificial

<220>
<223> oligonucleotide

<400> 465
tgagggcttt cacttcag 18

<210> 466
<211> 18
<212> DNA
<213> Artificial

<220>
<223> oligonucleotide

<400> 466

agggctttca cttcagac 18

<210> 467
<211> 18
<212> DNA
<213> Artificial

<220>
<223> oligonucleotide

<400> 467
gagggctttc acttcaga 18

<210> 468
<211> 18
<212> DNA
<213> Artificial

<220>
<223> oligonucleotide

<400> 468
actgcactca agtcatcc 18

<210> 469
<211> 18
<212> DNA
<213> Artificial

<220>
<223> oligonucleotide

<400> 469
ccggataacg cttggaac 18

<210> 470
<211> 18
<212> DNA
<213> Artificial

<220>
<223> oligonucleotide

<400> 470
tccggataac gcttggaa 18

<210> 471
<211> 18
<212> DNA
<213> Artificial

<220>
<223> oligonucleotide

<400> 471
tatcccctgc taagaggt 18

<210> 472

<211> 18
<212> DNA
<213> Artificial

<220>
<223> oligonucleotide

<400> 472
cctgctaaga ggtaggtt 18

<210> 473
<211> 18
<212> DNA
<213> Artificial

<220>
<223> oligonucleotide

<400> 473
ccctgctaag aggtaggt 18

<210> 474
<211> 18
<212> DNA
<213> Artificial

<220>
<223> oligonucleotide

<400> 474
cccctgctaa gaggtagg 18

<210> 475
<211> 18
<212> DNA
<213> Artificial

<220>
<223> oligonucleotide

<400> 475
tcccctgcta agaggtag 18

<210> 476
<211> 18
<212> DNA
<213> Artificial

<220>
<223> oligonucleotide

<400> 476
atcccctgct aagaggta 18

<210> 477
<211> 18
<212> DNA
<213> Artificial

<220>
<223> oligonucleotide

<400> 477
ccgttccttt ctggtaag 18

<210> 478
<211> 18
<212> DNA
<213> Artificial

<220>
<223> oligonucleotide

<400> 478
gccgttcctt tctggtaa 18

<210> 479
<211> 18
<212> DNA
<213> Artificial

<220>
<223> oligonucleotide

<400> 479
agccgttcct ttctggta 18

<210> 480
<211> 18
<212> DNA
<213> Artificial

<220>
<223> oligonucleotide

<400> 480
gcacgtattt agccgttc 18

<210> 481
<211> 18
<212> DNA
<213> Artificial

<220>
<223> oligonucleotide

<400> 481
cacgtattta gccgttcc 18

<210> 482
<211> 18
<212> DNA
<213> Artificial

<220>
<223> oligonucleotide

<400> 482
ggcacgtatt tagccgtt 18

<210> 483
<211> 18
<212> DNA
<213> Artificial

<220>
<223> oligonucleotide

<400> 483
cactttcctc tactgcac 18

<210> 484
<211> 18
<212> DNA
<213> Artificial

<220>
<223> oligonucleotide

<400> 484
ccactttcct ctactgca 18

<210> 485
<211> 18
<212> DNA
<213> Artificial

<220>
<223> oligonucleotide

<400> 485
tccactttcc tctactgc 18

<210> 486
<211> 18
<212> DNA
<213> Artificial

<220>
<223> oligonucleotide

<400> 486
ctttcctcta ctgcactc 18

<210> 487
<211> 18
<212> DNA
<213> Artificial

<220>
<223> oligonucleotide

<400> 487
tagccgttcc tttctggt 18

<210> 488
<211> 18
<212> DNA
<213> Artificial

<220>
<223> oligonucleotide

<400> 488
ttagccgttc ctttctgg 18

<210> 489
<211> 18
<212> DNA
<213> Artificial

<220>
<223> oligonucleotide

<400> 489
ttatcccctg ctaagagg 18

<210> 490
<211> 18
<212> DNA
<213> Artificial

<220>
<223> oligonucleotide

<400> 490
gttatcccct gctaagag 18

<210> 491
<211> 18
<212> DNA
<213> Artificial

<220>
<223> oligonucleotide

<400> 491
cccgttcgcc actctttg 18

<210> 492
<211> 18
<212> DNA
<213> Artificial

<220>
<223> oligonucleotide

<400> 492
agctgagggc tttcactt 18

<210> 493
<211> 18
<212> DNA

<213> Artificial

<220>
<223> oligonucleotide

<400> 493
gagctgaggg ctttcact 18

<210> 494
<211> 18
<212> DNA
<213> Artificial

<220>
<223> oligonucleotide

<400> 494
gctgagggct ttcacttc 18

<210> 495
<211> 18
<212> DNA
<213> Artificial

<220>
<223> oligonucleotide

<400> 495
ctgagggctt tcacttca 18

<210> 496
<211> 18
<212> DNA
<213> Artificial

<220>
<223> oligonucleotide

<400> 496
cccgtgtccc gaaggaac 18

<210> 497
<211> 18
<212> DNA
<213> Artificial

<220>
<223> oligonucleotide

<400> 497
gcacgagtat gtcaagac 18

<210> 498
<211> 18
<212> DNA
<213> Artificial

<220>

<223> oligonucleotide

<400> 498
gtatcccgtg tcccgaag 18

<210> 499
<211> 18
<212> DNA
<213> Artificial

<220>
<223> oligonucleotide

<400> 499
tcccgtgtcc cgaaggaa 18

<210> 500
<211> 18
<212> DNA
<213> Artificial

<220>
<223> oligonucleotide

<400> 500
atcccgtgtc ccgaagga 18

<210> 501
<211> 18
<212> DNA
<213> Artificial

<220>
<223> oligonucleotide

<400> 501
tatcccgtgt cccgaagg 18

<210> 502
<211> 18
<212> DNA
<213> Artificial

<220>
<223> oligonucleotide

<400> 502
cttaccttag gaagcgcc 18

<210> 503
<211> 18
<212> DNA
<213> Artificial

<220>
<223> oligonucleotide

<400> 503

ttaccttagg aagcgccc 18

<210> 504
<211> 18
<212> DNA
<213> Artificial

<220>
<223> oligonucleotide

<400> 504
cctgtatccc gtgtcccg 18

<210> 505
<211> 18
<212> DNA
<213> Artificial

<220>
<223> oligonucleotide

<400> 505
ccacctgtat cccgtgtc 18

<210> 506
<211> 18
<212> DNA
<213> Artificial

<220>
<223> oligonucleotide

<400> 506
cacctgtatc ccgtgtcc 18

<210> 507
<211> 18
<212> DNA
<213> Artificial

<220>
<223> oligonucleotide

<400> 507
acctgtatcc cgtgtccc 18

<210> 508
<211> 18
<212> DNA
<213> Artificial

<220>
<223> oligonucleotide

<400> 508
ctgtatcccg tgtcccga 18

<210> 509

<211> 18
<212> DNA
<213> Artificial

<220>
<223> oligonucleotide

<400> 509
tgtatcccgt gtcccgaa 18

<210> 510
<211> 18
<212> DNA
<213> Artificial

<220>
<223> oligonucleotide

<400> 510
cacgagtatg tcaagacc 18

<210> 511
<211> 18
<212> DNA
<213> Artificial

<220>
<223> oligonucleotide

<400> 511
cggtcttacc ttaggaag 18

<210> 512
<211> 18
<212> DNA
<213> Artificial

<220>
<223> oligonucleotide

<400> 512
taggaagcgc cctccttg 18

<210> 513
<211> 18
<212> DNA
<213> Artificial

<220>
<223> oligonucleotide

<400> 513
aggaagcgcc ctccttgc 18

<210> 514
<211> 18
<212> DNA
<213> Artificial

<220>
<223> oligonucleotide

<400> 514
ttaggaagcg ccctcctt 18

<210> 515
<211> 18
<212> DNA
<213> Artificial

<220>
<223> oligonucleotide

<400> 515
cttaggaagc gccctcct 18

<210> 516
<211> 18
<212> DNA
<213> Artificial

<220>
<223> oligonucleotide

<400> 516
ccttaggaag cgccctcc 18

<210> 517
<211> 18
<212> DNA
<213> Artificial

<220>
<223> oligonucleotide

<400> 517
accttaggaa gcgccctc 18

<210> 518
<211> 18
<212> DNA
<213> Artificial

<220>
<223> oligonucleotide

<400> 518
tgcacacaat ggttgagc 18

<210> 519
<211> 18
<212> DNA
<213> Artificial

<220>
<223> oligonucleotide

<400> 519
taccttagga agcgccct 18

<210> 520
<211> 18
<212> DNA
<213> Artificial

<220>
<223> oligonucleotide

<400> 520
accacctgta tcccgtgt 18

<210> 521
<211> 18
<212> DNA
<213> Artificial

<220>
<223> oligonucleotide

<400> 521
gcaccacctg tatcccgt 18

<210> 522
<211> 18
<212> DNA
<213> Artificial

<220>
<223> oligonucleotide

<400> 522
caccacctgt atcccgtg 18

<210> 523
<211> 18
<212> DNA
<213> Artificial

<220>
<223> oligonucleotide

<400> 523
gcggttaggc aacctact 18

<210> 524
<211> 18
<212> DNA
<213> Artificial

<220>
<223> oligonucleotide

<400> 524
tgcggttagg caacctac 18

<210> 525
<211> 18
<212> DNA
<213> Artificial

<220>
<223> oligonucleotide

<400> 525
ttgcggttag gcaaccta 18

<210> 526
<211> 18
<212> DNA
<213> Artificial

<220>
<223> oligonucleotide

<400> 526
ggtcttacct taggaagc 18

<210> 527
<211> 18
<212> DNA
<213> Artificial

<220>
<223> oligonucleotide

<400> 527
gctaatacaa cgcgggat 18

<210> 528
<211> 18
<212> DNA
<213> Artificial

<220>
<223> oligonucleotide

<400> 528
ctaatacaac gcgggatc 18

<210> 529
<211> 18
<212> DNA
<213> Artificial

<220>
<223> oligonucleotide

<400> 529
atacaacgcg ggatcatc 18

<210> 530
<211> 18
<212> DNA

<213> Artificial

<220>
<223> oligonucleotide

<400> 530
cggttaggca acctactt 18

<210> 531
<211> 18
<212> DNA
<213> Artificial

<220>
<223> oligonucleotide

<400> 531
tgcaccacct gtatcccg 18

<210> 532
<211> 18
<212> DNA
<213> Artificial

<220>
<223> oligonucleotide

<400> 532
gaagcgccct ccttgcgg 18

<210> 533
<211> 18
<212> DNA
<213> Artificial

<220>
<223> oligonucleotide

<400> 533
ggaagcgccc tccttgcg 18

<210> 534
<211> 18
<212> DNA
<213> Artificial

<220>
<223> oligonucleotide

<400> 534
cgtccctttc tggttaga 18

<210> 535
<211> 18
<212> DNA
<213> Artificial

<220>

<223> oligonucleotide

<400> 535
agctaataca acgcggga 18

<210> 536
<211> 18
<212> DNA
<213> Artificial

<220>
<223> oligonucleotide

<400> 536
tagctaatac aacgcggg 18

<210> 537
<211> 18
<212> DNA
<213> Artificial

<220>
<223> oligonucleotide

<400> 537
ctagctaata caacgcgg 18

<210> 538
<211> 18
<212> DNA
<213> Artificial

<220>
<223> oligonucleotide

<400> 538
ggctatgtat catcgcct 18

<210> 539
<211> 18
<212> DNA
<213> Artificial

<220>
<223> oligonucleotide

<400> 539
gagccactgc cttttaca 18

<210> 540
<211> 18
<212> DNA
<213> Artificial

<220>
<223> oligonucleotide

<400> 540

gtcggctatg tatcatcg 18

<210> 541
<211> 18
<212> DNA
<213> Artificial

<220>
<223> oligonucleotide

<400> 541
ggtcggctat gtatcatc 18

<210> 542
<211> 18
<212> DNA
<213> Artificial

<220>
<223> oligonucleotide

<400> 542
caggtcggct atgtatca 18

<210> 543
<211> 18
<212> DNA
<213> Artificial

<220>
<223> oligonucleotide

<400> 543 18
cggctatgta tcatcgcc 18

<210> 544
<211> 18
<212> DNA
<213> Artificial

<220>
<223> oligonucleotide

<400> 544 18
tcggctatgt atcatcgc 18

<210> 545
<211> 18
<212> DNA
<213> Artificial

<220>
<223> oligonucleotide

<400> 545
gtcttacctt aggaagcg 18

<210> 546

<211> 18
<212> DNA
<213> Artificial

<220>
<223> oligonucleotide

<400> 546 18
tcttacctta ggaagcgc 18

<210> 547
<211> 20
<212> DNA
<213> Artificial

<220>
<223> oligonucleotide

<400> 547 20
gtacaaaccg cctacacgcc 20

<210> 548
<211> 20
<212> DNA
<213> Artificial

<220>
<223> oligonucleotide

<400> 548 20
tgtacaaacc gcctacacgc 20

<210> 549
<211> 20
<212> DNA
<213> Artificial

<220>
<223> oligonucleotide

<400> 549
gatcagcacg atgtcgccat 20

<210> 550
<211> 20
<212> DNA
<213> Artificial

<220>
<223> oligonucleotide

<400> 550
ctgtacaaac cgcctacacg 20

<210> 551
<211> 20
<212> DNA
<213> Artificial

<220>
<223> oligonucleotide

<400> 551
gagatcagca cgatgtcgcc 20

<210> 552
<211> 20
<212> DNA
<213> Artificial

<220>
<223> oligonucleotide

<400> 552
agatcagcac gatgtcgcca 20

<210> 553
<211> 20
<212> DNA
<213> Artificial

<220>
<223> oligonucleotide

<400> 553 20
atcagcacga tgtcgccatc 20

<210> 554
<211> 20
<212> DNA
<213> Artificial

<220>
<223> oligonucleotide

<400> 554
tcagcacgat gtcgccatct 20

<210> 555
<211> 20
<212> DNA
<213> Artificial

<220>
<223> oligonucleotide

<400> 555
actgtacaaa ccgcctacac 20

<210> 556
<211> 20
<212> DNA
<213> Artificial

<220>
<223> oligonucleotide

<400> 556
ccgccactaa ggccgaaacc 20

<210> 557
<211> 20
<212> DNA
<213> Artificial

<220>
<223> oligonucleotide

<400> 557
cagcacgatg tcgccatcta 20

<210> 558
<211> 20
<212> DNA
<213> Artificial

<220>
<223> oligonucleotide

<400> 558
tacaaaccgc ctacacgccc 20

<210> 559
<211> 20
<212> DNA
<213> Artificial

<220>
<223> oligonucleotide

<400> 559
agcacgatgt cgccatctag 20

<210> 560
<211> 20
<212> DNA
<213> Artificial

<220>
<223> oligonucleotide

<400> 560
cggcttttag agatcagcac 20

<210> 561
<211> 20
<212> DNA
<213> Artificial

<220>
<223> oligonucleotide

<400> 561
tccgccacta aggccgaaac 20

<210> 562
<211> 20
<212> DNA
<213> Artificial

<220>
<223> oligonucleotide

<400> 562
gactgtacaa accgcctaca 20

<210> 563
<211> 20
<212> DNA
<213> Artificial

<220>
<223> oligonucleotide

<400> 563
gtccgccact aaggccgaaa 20

<210> 564
<211> 20
<212> DNA
<213> Artificial

<220>
<223> oligonucleotide

<400> 564
ggggatttca catctgactg 20

<210> 565
<211> 20
<212> DNA
<213> Artificial

<220>
<223> oligonucleotide

<400> 565 20
catacaagcc ctggtaaggt 20

<210> 566
<211> 20
<212> DNA
<213> Artificial

<220>
<223> oligonucleotide

<400> 566
acaagccctg gtaaggttct 20

<210> 567
<211> 20
<212> DNA

<213> Artificial

<220>
<223> oligonucleotide

<400> 567
acaaaccgcc tacacgccct 20

<210> 568
<211> 20
<212> DNA
<213> Artificial

<220>
<223> oligonucleotide

<400> 568
ctgactgtac aaaccgccta 20

<210> 569
<211> 20
<212> DNA
<213> Artificial

<220>
<223> oligonucleotide

<400> 569
tgactgtaca aaccgcctac 20

<210> 570
<211> 20
<212> DNA
<213> Artificial

<220>
<223> oligonucleotide

<400> 570
acgatgtcgc catctagctt 20

<210> 571
<211> 20
<212> DNA
<213> Artificial

<220>
<223> oligonucleotide

<400> 571
cacgatgtcg ccatctagct 20

<210> 572
<211> 20
<212> DNA
<213> Artificial

<220>

<223> oligonucleotide

<400> 572
cgatgtcgcc atctagcttc 20

<210> 573
<211> 20
<212> DNA
<213> Artificial

<220>
<223> oligonucleotide

<400> 573
gcacgatgtc gccatctagc 20

<210> 574
<211> 20
<212> DNA
<213> Artificial

<220>
<223> oligonucleotide

<400> 574
gatgtcgcca tctagcttcc 20

<210> 575
<211> 20
<212> DNA
<213> Artificial

<220>
<223> oligonucleotide

<400> 575
atgtcgccat ctagcttccc 20

<210> 576
<211> 20
<212> DNA
<213> Artificial

<220>
<223> oligonucleotide

<400> 576
tgtcgccatc tagcttccca 20

<210> 577
<211> 20
<212> DNA
<213> Artificial

<220>
<223> oligonucleotide

<400> 577

gccatctagc ttcccactgt 20

<210> 578
<211> 20
<212> DNA
<213> Artificial

<220>
<223> oligonucleotide

<400> 578
tcgccatcta gcttcccact 20

<210> 579
<211> 20
<212> DNA
<213> Artificial

<220>
<223> oligonucleotide

<400> 579
cgccatctag cttcccactg 20

<210> 580
<211> 20
<212> DNA
<213> Artificial

<220>
<223> oligonucleotide

<400> 580
gtcgccatct agcttcccac 20

<210> 581
<211> 20
<212> DNA
<213> Artificial

<220>
<223> oligonucleotide

<400> 581
tacaagccct ggtaaggttc 20

<210> 582
<211> 20
<212> DNA
<213> Artificial

<220>
<223> oligonucleotide

<400> 582
gccactaagg ccgaaacctt 20

<210> 583

<211> 20
<212> DNA
<213> Artificial

<220>
<223> oligonucleotide

<400> 583
actaaggccg aaaccttcgt 20

<210> 584
<211> 20
<212> DNA
<213> Artificial

<220>
<223> oligonucleotide

<400> 584
ctaaggccga aaccttcgtg 20

<210> 585
<211> 20
<212> DNA
<213> Artificial

<220>
<223> oligonucleotide

<400> 585
cactaaggcc gaaaccttcg 20

<210> 586
<211> 20
<212> DNA
<213> Artificial

<220>
<223> oligonucleotide

<400> 586
aaggccgaaa ccttcgtgcg 20

<210> 587
<211> 20
<212> DNA
<213> Artificial

<220>
<223> oligonucleotide

<400> 587
ccactaaggc cgaaaccttc 20

<210> 588
<211> 20
<212> DNA
<213> Artificial

<220>
<223> oligonucleotide

<400> 588
taaggccgaa accttcgtgc 20

<210> 589
<211> 20
<212> DNA
<213> Artificial

<220>
<223> oligonucleotide

<400> 589
aggccgaaac cttcgtgcga 20

<210> 590
<211> 20
<212> DNA
<213> Artificial

<220>
<223> oligonucleotide

<400> 590
tctgactgta caaaccgcct 20

<210> 591
<211> 20
<212> DNA
<213> Artificial

<220>
<223> oligonucleotide

<400> 591
catctgactg tacaaaccgc 20

<210> 592
<211> 20
<212> DNA
<213> Artificial

<220>
<223> oligonucleotide

<400> 592
atctgactgt acaaaccgcc 20

<210> 593
<211> 20
<212> DNA
<213> Artificial

<220>
<223> oligonucleotide

<400> 593
cttcgtgcga cttgcatgtg 20

<210> 594
<211> 20
<212> DNA
<213> Artificial

<220>
<223> oligonucleotide

<400> 594
ccttcgtgcg acttgcatgt 20

<210> 595
<211> 20
<212> DNA
<213> Artificial

<220>
<223> oligonucleotide

<400> 595
ctctctagag tgcccaccca 20

<210> 596
<211> 20
<212> DNA
<213> Artificial

<220>
<223> oligonucleotide

<400> 596
tctctagagt gcccacccaa 20

<210> 597
<211> 20
<212> DNA
<213> Artificial

<220>
<223> oligonucleotide

<400> 597
acgtatcaaa tgcagctccc 20

<210> 598
<211> 20
<212> DNA
<213> Artificial

<220>
<223> oligonucleotide

<400> 598
cgtatcaaat gcagctccca 20

<210> 599
<211> 20
<212> DNA
<213> Artificial

<220>
<223> oligonucleotide

<400> 599
cgccactaag gccgaaacct 20

<210> 600
<211> 20
<212> DNA
<213> Artificial

<220>
<223> oligonucleotide

<400> 600
ccgaaacctt cgtgcgactt 20

<210> 601
<211> 20
<212> DNA
<213> Artificial

<220>
<223> oligonucleotide

<400> 601
gccgaaacct tcgtgcgact 20

<210> 602
<211> 20
<212> DNA
<213> Artificial

<220>
<223> oligonucleotide

<400> 602
aaccttcgtg cgacttgcat 20

<210> 603
<211> 20
<212> DNA
<213> Artificial

<220>
<223> oligonucleotide

<400> 603
cgaaaccttc gtgcgacttg 20

<210> 604
<211> 20
<212> DNA

<213> Artificial

<220>
<223> oligonucleotide

<400> 604
accttcgtgc gacttgcatg 20

<210> 605
<211> 20
<212> DNA
<213> Artificial

<220>
<223> oligonucleotide

<400> 605
gaaaccttcg tgcgacttgc 20

<210> 606
<211> 20
<212> DNA
<213> Artificial

<220>
<223> oligonucleotide

<400> 606
ggccgaaacc ttcgtgcgac 20

<210> 607
<211> 20
<212> DNA
<213> Artificial

<220>
<223> oligonucleotide

<400> 607
aaaccttcgt gcgacttgca 20

<210> 608
<211> 20
<212> DNA
<213> Artificial

<220>
<223> oligonucleotide

<400> 608
cacgtatcaa atgcagctcc 20

<210> 609
<211> 20
<212> DNA
<213> Artificial

<220>

<223> oligonucleotide

<400> 609
gctcaccggc ttaaggtcaa 20

<210> 610
<211> 20
<212> DNA
<213> Artificial

<220>
<223> oligonucleotide

<400> 610
cgctcaccgg cttaaggtca 20

<210> 611
<211> 20
<212> DNA
<213> Artificial

<220>
<223> oligonucleotide

<400> 611
tcgctcaccg gcttaaggtc 20

<210> 612
<211> 20
<212> DNA
<213> Artificial

<220>
<223> oligonucleotide

<400> 612
ctcaccggct taaggtcaaa 20

<210> 613
<211> 20
<212> DNA
<213> Artificial

<220>
<223> oligonucleotide

<400> 613
cccgaccgtg gtcggctgcg 20

<210> 614
<211> 20
<212> DNA
<213> Artificial

<220>
<223> oligonucleotide

<400> 614

gctcaccggc ttaaggtcaa 20

<210> 615
<211> 20
<212> DNA
<213> Artificial

<220>
<223> oligonucleotide

<400> 615
cgctcaccgg cttaaggtca 20

<210> 616
<211> 20
<212> DNA
<213> Artificial

<220>
<223> oligonucleotide

<400> 616
tcgctcaccg gcttaaggtc 20

<210> 617
<211> 20
<212> DNA
<213> Artificial

<220>
<223> oligonucleotide

<400> 617
ctcaccggct taaggtcaaa 20

<210> 618
<211> 20
<212> DNA
<213> Artificial

<220>
<223> oligonucleotide

<400> 618
cccgaccgtg gtcggctgcg 20

<210> 619
<211> 20
<212> DNA
<213> Artificial

<220>
<223> oligonucleotide

<400> 619
tcaccggctt aaggtcaaac 20

<210> 620

<211> 20
<212> DNA
<213> Artificial

<220>
<223> oligonucleotide

<400> 620
caaccctctc tcacactcta 20

<210> 621
<211> 20
<212> DNA
<213> Artificial

<220>
<223> oligonucleotide

<400> 621
acaaccctct ctcacactct 20

<210> 622
<211> 20
<212> DNA
<213> Artificial

<220>
<223> oligonucleotide

<400> 622
ccacaaccct ctctcacact 20

<210> 623
<211> 20
<212> DNA
<213> Artificial

<220>
<223> oligonucleotide

<400> 623 20
aaccctctct cacactctag 20

<210> 624
<211> 20
<212> DNA
<213> Artificial

<220>
<223> oligonucleotide

<400> 624 20
cacaaccctc tctcacactc 20

<210> 625
<211> 20
<212> DNA
<213> Artificial

<220>
<223> oligonucleotide

<400> 625 20
tccacaaccc tctctcacac 20

<210> 626
<211> 20
<212> DNA
<213> Artificial

<220>
<223> oligonucleotide

<400> 626
ttccacaacc ctctctcaca 20

<210> 627
<211> 20
<212> DNA
<213> Artificial

<220>
<223> oligonucleotide

<400> 627 20
accctctctc acactctagt 20

<210> 628
<211> 20
<212> DNA
<213> Artificial

<220>
<223> oligonucleotide

<400> 628
gagccaggtt gccgccttcg 20

<210> 629
<211> 20
<212> DNA
<213> Artificial

<220>
<223> oligonucleotide

<400> 629
aggtcaaacc aactcccatg 20

<210> 630
<211> 20
<212> DNA
<213> Artificial

<220>
<223> oligonucleotide

<400> 630
atgagccagg ttgccgcctt 20

<210> 631
<211> 20
<212> DNA
<213> Artificial

<220>
<223> oligonucleotide

<400> 631
tgagccaggt tgccgccttc 20

<210> 632
<211> 20
<212> DNA
<213> Artificial

<220>
<223> oligonucleotide

<400> 632
aggctcctcc acaggcgact 20

<210> 633
<211> 20
<212> DNA
<213> Artificial

<220>
<223> oligonucleotide

<400> 633
caggctcctc cacaggcgac 20

<210> 634
<211> 20
<212> DNA
<213> Artificial

<220>
<223> oligonucleotide

<400> 634
gcaggctcct ccacaggcga 20

<210> 635
<211> 20
<212> DNA
<213> Artificial

<220>
<223> oligonucleotide

<400> 635
ttcgctcacc ggcttaaggt 20

<210> 636
<211> 20
<212> DNA
<213> Artificial

<220>
<223> oligonucleotide

<400> 636
gttcgctcac cggcttaagg 20

<210> 637
<211> 20
<212> DNA
<213> Artificial

<220>
<223> oligonucleotide

<400> 637
ggttcgctca ccggcttaag 20

<210> 638
<211> 20
<212> DNA
<213> Artificial

<220>
<223> oligonucleotide

<400> 638
attccacaac cctctctcac 20

<210> 639
<211> 20
<212> DNA
<213> Artificial

<220>
<223> oligonucleotide

<400> 639
tgacccgacc gtggtcggct 20

<210> 640
<211> 20
<212> DNA
<213> Artificial

<220>
<223> oligonucleotide

<400> 640
ccctctctca cactctagtc 20

<210> 641
<211> 20
<212> DNA

<213> Artificial

<220>
<223> oligonucleotide

<400> 641
gaattccaca accctctctc 20

<210> 642
<211> 20
<212> DNA
<213> Artificial

<220>
<223> oligonucleotide

<400> 642
agccaggttg ccgccttcgc 20

<210> 643
<211> 20
<212> DNA
<213> Artificial

<220>
<223> oligonucleotide

<400> 643
gccaggttgc cgccttcgcc 20

<210> 644
<211> 20
<212> DNA
<213> Artificial

<220>
<223> oligonucleotide

<400> 644
ggaattccac aaccctctct 20

<210> 645
<211> 20
<212> DNA
<213> Artificial

<220>
<223> oligonucleotide

<400> 645
gggaattcca caaccctctc 20

<210> 646
<211> 20
<212> DNA
<213> Artificial

<220>

<223> oligonucleotide

<400> 646
aacgcaggct cctccacagg 20

<210> 647
<211> 20
<212> DNA
<213> Artificial

<220>
<223> oligonucleotide

<400> 647
cggcttaagg tcaaaccaac 20

<210> 648
<211> 20
<212> DNA
<213> Artificial

<220>
<223> oligonucleotide

<400> 648
ccggcttaag gtcaaaccaa 20

<210> 649
<211> 20
<212> DNA
<213> Artificial

<220>
<223> oligonucleotide

<400> 649
caccggctta aggtcaaacc 20

<210> 650
<211> 20
<212> DNA
<213> Artificial

<220>
<223> oligonucleotide

<400> 650
accggcttaa ggtcaaacca 20

<210> 651
<211> 20
<212> DNA
<213> Artificial

<220>
<223> oligonucleotide

<400> 651

acccaacatc cagcacacat 20

<210> 652
<211> 20
<212> DNA
<213> Artificial

<220>
<223> oligonucleotide

<400> 652
tcgctgaccc gaccgtggtc 20

<210> 653
<211> 20
<212> DNA
<213> Artificial

<220>
<223> oligonucleotide

<400> 653
cgctgacccg accgtggtcg 20

<210> 654
<211> 20
<212> DNA
<213> Artificial

<220>
<223> oligonucleotide

<400> 654
gacccgaccg tggtcggctg 20

<210> 655
<211> 20
<212> DNA
<213> Artificial

<220>
<223> oligonucleotide

<400> 655
gctgacccga ccgtggtcgg 20

<210> 656
<211> 20
<212> DNA
<213> Artificial

<220>
<223> oligonucleotide

<400> 656
ctgacccgac cgtggtcggc 20

<210> 657

<211> 20
<212> DNA
<213> Artificial

<220>
<223> oligonucleotide

<400> 657
caggcgactt gcgcctttga 20

<210> 658
<211> 20
<212> DNA
<213> Artificial

<220>
<223> oligonucleotide

<400> 658
tcatgcggta ttagctccag 20

<210> 659
<211> 20
<212> DNA
<213> Artificial

<220>
<223> oligonucleotide

<400> 659
actagctaat cgaacgcagg 20

<210> 660
<211> 20
<212> DNA
<213> Artificial

<220>
<223> oligonucleotide

<400> 660
catgcggtat tagctccagt 20

<210> 661
<211> 20
<212> DNA
<213> Artificial

<220>
<223> oligonucleotide

<400> 661
cgcaggctcc tccacaggcg 20

<210> 662
<211> 20
<212> DNA
<213> Artificial

<220>
<223> oligonucleotide

<400> 662
acgcaggctc ctccacaggc 20

<210> 663
<211> 20
<212> DNA
<213> Artificial

<220>
<223> oligonucleotide

<400> 663
ctcaggtgtc atgcggtatt 20

<210> 664
<211> 20
<212> DNA
<213> Artificial

<220>
<223> oligonucleotide

<400> 664
cgcctttgac cctcaggtgt 20

<210> 665
<211> 20
<212> DNA
<213> Artificial

<220>
<223> oligonucleotide

<400> 665
accctcaggt gtcatgcggt 20

<210> 666
<211> 20
<212> DNA
<213> Artificial

<220>
<223> oligonucleotide

<400> 666
cctcaggtgt catgcggtat 20

<210> 667
<211> 20
<212> DNA
<213> Artificial

<220>
<223> oligonucleotide

<400> 667
tttgaccctc aggtgtcatg 20

<210> 668
<211> 20
<212> DNA
<213> Artificial

<220>
<223> oligonucleotide

<400> 668
gaccctcagg tgtcatgcgg 20

<210> 669
<211> 20
<212> DNA
<213> Artificial

<220>
<223> oligonucleotide

<400> 669
tgaccctcag gtgtcatgcg 20

<210> 670
<211> 20
<212> DNA
<213> Artificial

<220>
<223> oligonucleotide

<400> 670
gcctttgacc ctcaggtgtc 20

<210> 671
<211> 20
<212> DNA
<213> Artificial

<220>
<223> oligonucleotide

<400> 671
ttgaccctca ggtgtcatgc 20

<210> 672
<211> 20
<212> DNA
<213> Artificial

<220>
<223> oligonucleotide

<400> 672
ccctcaggtg tcatgcggta 20

<210> 673
<211> 20
<212> DNA
<213> Artificial

<220>
<223> oligonucleotide

<400> 673
cctttgaccc tcaggtgtca 20

<210> 674
<211> 20
<212> DNA
<213> Artificial

<220>
<223> oligonucleotide

<400> 674
ctttgaccct caggtgtcat 20

<210> 675
<211> 20
<212> DNA
<213> Artificial

<220>
<223> oligonucleotide

<400> 675
agttatcccc cacccatgga 20

<210> 676
<211> 20
<212> DNA
<213> Artificial

<220>
<223> oligonucleotide

<400> 676
ccagctatcg atcatcgcct 20

<210> 677
<211> 20
<212> DNA
<213> Artificial

<220>
<223> oligonucleotide

<400> 677
accagctatc gatcatcgcc 20

<210> 678
<211> 20
<212> DNA

<213> Artificial

<220>
<223> oligonucleotide

<400> 678
cagctatcga tcatcgcctt 20

<210> 679
<211> 20
<212> DNA
<213> Artificial

<220>
<223> oligonucleotide

<400> 679
agctatcgat catcgccttg 20

<210> 680
<211> 20
<212> DNA
<213> Artificial

<220>
<223> oligonucleotide

<400> 680
gctatcgatc atcgccttgg 20

<210> 681
<211> 20
<212> DNA
<213> Artificial

<220>
<223> oligonucleotide

<400> 681
ctatcgatca tcgccttggt 20

<210> 682
<211> 20
<212> DNA
<213> Artificial

<220>
<223> oligonucleotide

<400> 682
ttcgtgcgac ttgcatgtgt 20

<210> 683
<211> 20
<212> DNA
<213> Artificial

<220>

<223> oligonucleotide

<400> 683
tcgatcatcg ccttggtagg 20

<210> 684
<211> 20
<212> DNA
<213> Artificial

<220>
<223> oligonucleotide

<400> 684
atcgatcatc gccttggtag 20

<210> 685
<211> 20
<212> DNA
<213> Artificial

<220>
<223> oligonucleotide

<400> 685
cacaggcgac ttgcgccttt 20

<210> 686
<211> 20
<212> DNA
<213> Artificial

<220>
<223> oligonucleotide

<400> 686
ccacaggcga cttgcgcctt 20

<210> 687
<211> 20
<212> DNA
<213> Artificial

<220>
<223> oligonucleotide

<400> 687
tccacaggcg acttgcgcct 20

<210> 688
<211> 20
<212> DNA
<213> Artificial

<220>
<223> oligonucleotide

<400> 688

tcctccacag gcgacttgcg 20

<210> 689
<211> 20
<212> DNA
<213> Artificial

<220>
<223> oligonucleotide

<400> 689
cctccacagg cgacttgcgc 20

<210> 690
<211> 20
<212> DNA
<213> Artificial

<220>
<223> oligonucleotide

<400> 690
ctccacaggc gacttgcgcc 20

<210> 691
<211> 20
<212> DNA
<213> Artificial

<220>
<223> oligonucleotide

<400> 691
acaggcgact tgcgcctttg 20

<210> 692
<211> 20
<212> DNA
<213> Artificial

<220>
<223> oligonucleotide

<400> 692
gctcaccggc ttaaggtcaa 20

<210> 693
<211> 20
<212> DNA
<213> Artificial

<220>
<223> oligonucleotide

<400> 693
cgctcaccgg cttaaggtca 20

<210> 694

<211> 20
<212> DNA
<213> Artificial

<220>
<223> oligonucleotide

<400> 694
tcgctcaccg gcttaaggtc 20

<210> 695
<211> 20
<212> DNA
<213> Artificial

<220>
<223> oligonucleotide

<400> 695
ctcaccggct taaggtcaaa 20

<210> 696
<211> 20
<212> DNA
<213> Artificial

<220>
<223> oligonucleotide

<400> 696
cccgaccgtg gtcggctgcg 20

<210> 697
<211> 20
<212> DNA
<213> Artificial

<220>
<223> oligonucleotide

<400> 697
tcaccggctt aaggtcaaac 20

<210> 698
<211> 20
<212> DNA
<213> Artificial

<220>
<223> oligonucleotide

<400> 698
caaccctctc tcacactcta 20

<210> 699
<211> 20
<212> DNA
<213> Artificial

<220>
<223> oligonucleotide

<400> 699
acaaccctct ctcacactct 20

<210> 700
<211> 20
<212> DNA
<213> Artificial

<220>
<223> oligonucleotide

<400> 700
ccacaaccct ctctcacact 20

<210> 701
<211> 20
<212> DNA
<213> Artificial

<220>
<223> oligonucleotide

<400> 701
aaccctctct cacactctag 20

<210> 702
<211> 20
<212> DNA
<213> Artificial

<220>
<223> oligonucleotide

<400> 702
cacaaccctc tctcacactc 20

<210> 703
<211> 20
<212> DNA
<213> Artificial

<220>
<223> oligonucleotide

<400> 703
tccacaaccc tctctcacac 20

<210> 704
<211> 20
<212> DNA
<213> Artificial

<220>
<223> oligonucleotide

<400> 704
ttccacaacc ctctctcaca 20

<210> 705
<211> 20
<212> DNA
<213> Artificial

<220>
<223> oligonucleotide

<400> 705
accctctctc acactctagt 20

<210> 706
<211> 20
<212> DNA
<213> Artificial

<220>
<223> oligonucleotide

<400> 706
gagccaggtt gccgccttcg 20

<210> 707
<211> 20
<212> DNA
<213> Artificial

<220>
<223> oligonucleotide

<400> 707
aggtcaaacc aactcccatg 20

<210> 708
<211> 20
<212> DNA
<213> Artificial

<220>
<223> oligonucleotide

<400> 708
atgagccagg ttgccgcctt 20

<210> 709
<211> 20
<212> DNA
<213> Artificial

<220>
<223> oligonucleotide

<400> 709
tgagccaggt tgccgccttc 20

<210> 710
<211> 20
<212> DNA
<213> Artificial

<220>
<223> oligonucleotide

<400> 710
aggctcctcc acaggcgact 20

<210> 711
<211> 20
<212> DNA
<213> Artificial

<220>
<223> oligonucleotide

<400> 711
caggctcctc cacaggcgac 20

<210> 712
<211> 20
<212> DNA
<213> Artificial

<220>
<223> oligonucleotide

<400> 712
gcaggctcct ccacaggcga 20

<210> 713
<211> 20
<212> DNA
<213> Artificial

<220>
<223> oligonucleotide

<400> 713
ttcgctcacc ggcttaaggt 20

<210> 714
<211> 20
<212> DNA
<213> Artificial

<220>
<223> oligonucleotide

<400> 714
gttcgctcac cggcttaagg 20

<210> 715
<211> 20
<212> DNA

<213> Artificial

<220>
<223> oligonucleotide

<400> 715
ggttcgctca ccggcttaag 20

<210> 716
<211> 20
<212> DNA
<213> Artificial

<220>
<223> oligonucleotide

<400> 716
attccacaac cctctctcac 20

<210> 717
<211> 20
<212> DNA
<213> Artificial

<220>
<223> oligonucleotide

<400> 717
tgacccgacc gtggtcggct 20

<210> 718
<211> 20
<212> DNA
<213> Artificial

<220>
<223> oligonucleotide

<400> 718
ccctctctca cactctagtc 20

<210> 719
<211> 20
<212> DNA
<213> Artificial

<220>
<223> oligonucleotide

<400> 719
gaattccaca accctctctc 20

<210> 720
<211> 20
<212> DNA
<213> Artificial

<220>

<223> oligonucleotide

<400> 720
agccaggttg ccgccttcgc 20

<210> 721
<211> 20
<212> DNA
<213> Artificial

<220>
<223> oligonucleotide

<400> 721
gccaggttgc cgccttcgcc 20

<210> 722
<211> 20
<212> DNA
<213> Artificial

<220>
<223> oligonucleotide

<400> 722
ggaattccac aaccctctct 20

<210> 723
<211> 20
<212> DNA
<213> Artificial

<220>
<223> oligonucleotide

<400> 723
gggaattcca caaccctctc 20

<210> 724
<211> 20
<212> DNA
<213> Artificial

<220>
<223> oligonucleotide

<400> 724
aacgcaggct cctccacagg 20

<210> 725
<211> 20
<212> DNA
<213> Artificial

<220>
<223> oligonucleotide

<400> 725

cggcttaagg tcaaaccaac 20

<210> 726
<211> 20
<212> DNA
<213> Artificial

<220>
<223> oligonucleotide

<400> 726
ccggcttaag gtcaaaccaa 20

<210> 727
<211> 20
<212> DNA
<213> Artificial

<220>
<223> oligonucleotide

<400> 727
caccggctta aggtcaaacc 20

<210> 728
<211> 20
<212> DNA
<213> Artificial

<220>
<223> oligonucleotide

<400> 728
accggcttaa ggtcaaacca 20

<210> 729
<211> 20
<212> DNA
<213> Artificial

<220>
<223> oligonucleotide

<400> 729
acccaacatc cagcacacat 20

<210> 730
<211> 20
<212> DNA
<213> Artificial

<220>
<223> oligonucleotide

<400> 730
tcgctgaccc gaccgtggtc 20

<210> 731

<211> 20
<212> DNA
<213> Artificial

<220>
<223> oligonucleotide

<400> 731
cgctgacccg accgtggtcg 20

<210> 732
<211> 20
<212> DNA
<213> Artificial

<220>
<223> oligonucleotide

<400> 732
gacccgaccg tggtcggctg 20

<210> 733
<211> 20
<212> DNA
<213> Artificial

<220>
<223> oligonucleotide

<400> 733
gctgacccga ccgtggtcgg 20

<210> 734
<211> 20
<212> DNA
<213> Artificial

<220>
<223> oligonucleotide

<400> 734
ctgacccgac cgtggtcggc 20

<210> 735
<211> 20
<212> DNA
<213> Artificial

<220>
<223> oligonucleotide

<400> 735
caggcgactt gcgcctttga 20

<210> 736
<211> 20
<212> DNA
<213> Artificial

<220>
<223> oligonucleotide

<400> 736
tcatgcggta ttagctccag 20

<210> 737
<211> 20
<212> DNA
<213> Artificial

<220>
<223> oligonucleotide

<400> 737 20
actagctaat cgaacgcagg 20

<210> 738
<211> 20
<212> DNA
<213> Artificial

<220>
<223> oligonucleotide

<400> 738 20
catgcggtat tagctccagt 20

<210> 739
<211> 20
<212> DNA
<213> Artificial

<220>
<223> oligonucleotide

<400> 739 20
cgcaggctcc tccacaggcg 20

<210> 740
<211> 20
<212> DNA
<213> Artificial

<220>
<223> oligonucleotide

<400> 740
acgcaggctc ctccacaggc 20

<210> 741
<211> 20
<212> DNA
<213> Artificial

<220>
<223> oligonucleotide

<400> 741
ctcaggtgtc atgcggtatt 20

<210> 742
<211> 20
<212> DNA
<213> Artificial

<220>
<223> oligonucleotide

<400> 742
cgcctttgac cctcaggtgt 20

<210> 743
<211> 20
<212> DNA
<213> Artificial

<220>
<223> oligonucleotide

<400> 743
accctcaggt gtcatgcggt 20

<210> 744
<211> 20
<212> DNA
<213> Artificial

<220>
<223> oligonucleotide

<400> 744
cctcaggtgt catgcggtat 20

<210> 745
<211> 20
<212> DNA
<213> Artificial

<220>
<223> oligonucleotide

<400> 745
tttgaccctc aggtgtcatg 20

<210> 746
<211> 20
<212> DNA
<213> Artificial

<220>
<223> oligonucleotide

<400> 746
gaccctcagg tgtcatgcgg 20

<210> 747
<211> 20
<212> DNA
<213> Artificial

<220>
<223> oligonucleotide

<400> 747
tgaccctcag gtgtcatgcg 20

<210> 748
<211> 20
<212> DNA
<213> Artificial

<220>
<223> oligonucleotide

<400> 748
gcctttgacc ctcaggtgtc 20

<210> 749
<211> 20
<212> DNA
<213> Artificial

<220>
<223> oligonucleotide

<400> 749
ttgaccctca ggtgtcatgc 20

<210> 750
<211> 20
<212> DNA
<213> Artificial

<220>
<223> oligonucleotide

<400> 750
ccctcaggtg tcatgcggta 20

<210> 751
<211> 20
<212> DNA
<213> Artificial

<220>
<223> oligonucleotide

<400> 751
cctttgaccc tcaggtgtca 20

<210> 752
<211> 20
<212> DNA

<213> Artificial

<220>
<223> oligonucleotide

<400> 752
ctttgaccct caggtgtcat 20

<210> 753
<211> 20
<212> DNA
<213> Artificial

<220>
<223> oligonucleotide

<400> 753 20
agttatcccc cacccatgga 20

<210> 754
<211> 20
<212> DNA
<213> Artificial

<220>
<223> oligonucleotide

<400> 754
ccagctatcg atcatcgcct 20

<210> 755
<211> 20
<212> DNA
<213> Artificial

<220>
<223> oligonucleotide

<400> 755 20
accagctatc gatcatcgcc 20

<210> 756
<211> 20
<212> DNA
<213> Artificial

<220>
<223> oligonucleotide

<400> 756
cagctatcga tcatcgcctt 20

<210> 757
<211> 20
<212> DNA
<213> Artificial

<220>

<223> oligonucleotide

<400> 757 20
agctatcgat catcgccttg 20

<210> 758
<211> 20
<212> DNA
<213> Artificial

<220>
<223> oligonucleotide

<400> 758
gctatcgatc atcgccttgg 20

<210> 759
<211> 20
<212> DNA
<213> Artificial

<220>
<223> oligonucleotide

<400> 759
ctatcgatca tcgccttggt 20

<210> 760
<211> 20
<212> DNA
<213> Artificial

<220>
<223> oligonucleotide

<400> 760
ttcgtgcgac ttgcatgtgt 20

<210> 761
<211> 20
<212> DNA
<213> Artificial

<220>
<223> oligonucleotide

<400> 761
tcgatcatcg ccttggtagg 20

<210> 762
<211> 20
<212> DNA
<213> Artificial

<220>
<223> oligonucleotide

<400> 762

atcgatcatc gccttggtag 20

<210> 763
<211> 20
<212> DNA
<213> Artificial

<220>
<223> oligonucleotide

<400> 763
cacaggcgac ttgcgccttt 20

<210> 764
<211> 20
<212> DNA
<213> Artificial

<220>
<223> oligonucleotide

<400> 764
ccacaggcga cttgcgcctt 20

<210> 765
<211> 20
<212> DNA
<213> Artificial

<220>
<223> oligonucleotide

<400> 765
tccacaggcg acttgcgcct 20

<210> 766
<211> 20
<212> DNA
<213> Artificial

<220>
<223> oligonucleotide

<400> 766
tcctccacag gcgacttgcg 20

<210> 767
<211> 20
<212> DNA
<213> Artificial

<220>
<223> oligonucleotide

<400> 767
cctccacagg cgacttgcgc 20

<210> 768

<211> 20
<212> DNA
<213> Artificial

<220>
<223> oligonucleotide

<400> 768
ctccacaggc gacttgcgcc 20

<210> 769
<211> 20
<212> DNA
<213> Artificial

<220>
<223> oligonucleotide

<400> 769
acaggcgact tgcgcctttg 20

<210> 770
<211> 20
<212> DNA
<213> Artificial

<220>
<223> oligonucleotide

<400> 770
tcaccggctt aaggtcaaac 20

<210> 771
<211> 20
<212> DNA
<213> Artificial

<220>
<223> oligonucleotide

<400> 771
caaccctctc tcacactcta 20

<210> 772
<211> 20
<212> DNA
<213> Artificial

<220>
<223> oligonucleotide

<400> 772
acaaccctct ctcacactct 20

<210> 773
<211> 20
<212> DNA
<213> Artificial

<220>
<223> oligonucleotide

<400> 773
ccacaaccct ctctcacact 20

<210> 774
<211> 20
<212> DNA
<213> Artificial

<220>
<223> oligonucleotide

<400> 774
aaccctctct cacactctag 20

<210> 775
<211> 20
<212> DNA
<213> Artificial

<220>
<223> oligonucleotide

<400> 775
cacaaccctc tctcacactc 20

<210> 776
<211> 20
<212> DNA
<213> Artificial

<220>
<223> oligonucleotide

<400> 776
tccacaaccc tctctcacac 20

<210> 777
<211> 20
<212> DNA
<213> Artificial

<220>
<223> oligonucleotide

<400> 777
ttccacaacc ctctctcaca 20

<210> 778
<211> 20
<212> DNA
<213> Artificial

<220>
<223> oligonucleotide

<400> 778
accctctctc acactctagt 20

<210> 779
<211> 20
<212> DNA
<213> Artificial

<220>
<223> oligonucleotide

<400> 779
gagccaggtt gccgccttcg 20

<210> 780
<211> 20
<212> DNA
<213> Artificial

<220>
<223> oligonucleotide

<400> 780
aggtcaaacc aactcccatg 20

<210> 781
<211> 20
<212> DNA
<213> Artificial

<220>
<223> oligonucleotide

<400> 781
atgagccagg ttgccgcctt 20

<210> 782
<211> 20
<212> DNA
<213> Artificial

<220>
<223> oligonucleotide

<400> 782
tgagccaggt tgccgccttc 20

<210> 783
<211> 20
<212> DNA
<213> Artificial

<220>
<223> oligonucleotide

<400> 783
aggctcctcc acaggcgact 20

<210> 784
<211> 20
<212> DNA
<213> Artificial

<220>
<223> oligonucleotide

<400> 784
caggctcctc cacaggcgac 20

<210> 785
<211> 20
<212> DNA
<213> Artificial

<220>
<223> oligonucleotide

<400> 785
gcaggctcct ccacaggcga 20

<210> 786
<211> 20
<212> DNA
<213> Artificial

<220>
<223> oligonucleotide

<400> 786
ttcgctcacc ggcttaaggt 20

<210> 787
<211> 20
<212> DNA
<213> Artificial

<220>
<223> oligonucleotide

<400> 787
gttcgctcac cggcttaagg 20

<210> 788
<211> 20
<212> DNA
<213> Artificial

<220>
<223> oligonucleotide

<400> 788
ggttcgctca ccggcttaag 20

<210> 789
<211> 20
<212> DNA

<213> Artificial

<220>
<223> oligonucleotide

<400> 789
attccacaac cctctctcac 20

<210> 790
<211> 20
<212> DNA
<213> Artificial

<220>
<223> oligonucleotide

<400> 790
tgacccgacc gtggtcggct 20

<210> 791
<211> 20
<212> DNA
<213> Artificial

<220>
<223> oligonucleotide

<400> 791
ccctctctca cactctagtc 20

<210> 792
<211> 20
<212> DNA
<213> Artificial

<220>
<223> oligonucleotide

<400> 792
gaattccaca accctctctc 20

<210> 793
<211> 20
<212> DNA
<213> Artificial

<220>
<223> oligonucleotide

<400> 793
agccaggttg ccgccttcgc 20

<210> 794
<211> 20
<212> DNA
<213> Artificial

<220>

<223> oligonucleotide

<400> 794
gccaggttgc cgccttcgcc 20

<210> 795
<211> 20
<212> DNA
<213> Artificial

<220>
<223> oligonucleotide

<400> 795
ggaattccac aaccctctct 20

<210> 796
<211> 20
<212> DNA
<213> Artificial

<220>
<223> oligonucleotide

<400> 796
gggaattcca caaccctctc 20

<210> 797
<211> 20
<212> DNA
<213> Artificial

<220>
<223> oligonucleotide

<400> 797
aacgcaggct cctccacagg 20

<210> 798
<211> 20
<212> DNA
<213> Artificial

<220>
<223> oligonucleotide

<400> 798
cggcttaagg tcaaaccaac 20

<210> 799
<211> 20
<212> DNA
<213> Artificial

<220>
<223> oligonucleotide

<400> 799

ccggcttaag gtcaaaccaa 20

<210> 800
<211> 20
<212> DNA
<213> Artificial

<220>
<223> oligonucleotide

<400> 800
caccggctta aggtcaaacc 20

<210> 801
<211> 20
<212> DNA
<213> Artificial

<220>
<223> oligonucleotide

<400> 801
accggcttaa ggtcaaacca 20

<210> 802
<211> 20
<212> DNA
<213> Artificial

<220>
<223> oligonucleotide

<400> 802
acccaacatc cagcacacat 20

<210> 803
<211> 20
<212> DNA
<213> Artificial

<220>
<223> oligonucleotide

<400> 803
tcgctgaccc gaccgtggtc 20

<210> 804
<211> 20
<212> DNA
<213> Artificial

<220>
<223> oligonucleotide

<400> 804
cgctgacccg accgtggtcg 20

<210> 805

<211> 20
<212> DNA
<213> Artificial

<220>
<223> oligonucleotide

<400> 805
gacccgaccg tggtcggctg 20

<210> 806
<211> 20
<212> DNA
<213> Artificial

<220>
<223> oligonucleotide

<400> 806
gctgacccga ccgtggtcgg 20

<210> 807
<211> 20
<212> DNA
<213> Artificial

<220>
<223> oligonucleotide

<400> 807
ctgacccgac cgtggtcggc 20

<210> 808
<211> 20
<212> DNA
<213> Artificial

<220>
<223> oligonucleotide

<400> 808
caggcgactt gcgcctttga 20

<210> 809
<211> 20
<212> DNA
<213> Artificial

<220>
<223> oligonucleotide

<400> 809
tcatgcggta ttagctccag 20

<210> 810
<211> 20
<212> DNA
<213> Artificial

<220>
<223> oligonucleotide

<400> 810
actagctaat cgaacgcagg 20

<210> 811
<211> 20
<212> DNA
<213> Artificial

<220>
<223> oligonucleotide

<400> 811
catgcggtat tagctccagt 20

<210> 812
<211> 20
<212> DNA
<213> Artificial

<220>
<223> oligonucleotide

<400> 812
cgcaggctcc tccacaggcg 20

<210> 813
<211> 20
<212> DNA
<213> Artificial

<220>
<223> oligonucleotide

<400> 813
acgcaggctc ctccacaggc 20

<210> 814
<211> 20
<212> DNA
<213> Artificial

<220>
<223> oligonucleotide

<400> 814
ctcaggtgtc atgcggtatt 20

<210> 815
<211> 20
<212> DNA
<213> Artificial

<220>
<223> oligonucleotide

<400> 815
cgcctttgac cctcaggtgt 20

<210> 816
<211> 20
<212> DNA
<213> Artificial

<220>
<223> oligonucleotide

<400> 816
accctcaggt gtcatgcggt 20

<210> 817
<211> 20
<212> DNA
<213> Artificial

<220>
<223> oligonucleotide

<400> 817
cctcaggtgt catgcggtat 20

<210> 818
<211> 20
<212> DNA
<213> Artificial

<220>
<223> oligonucleotide

<400> 818
tttgaccctc aggtgtcatg 20

<210> 819
<211> 20
<212> DNA
<213> Artificial

<220>
<223> oligonucleotide

<400> 819
gaccctcagg tgtcatgcgg 20

<210> 820
<211> 20
<212> DNA
<213> Artificial

<220>
<223> oligonucleotide

<400> 820
tgaccctcag gtgtcatgcg 20

<210> 821
<211> 20
<212> DNA
<213> Artificial

<220>
<223> oligonucleotide

<400> 821
gcctttgacc ctcaggtgtc 20

<210> 822
<211> 20
<212> DNA
<213> Artificial

<220>
<223> oligonucleotide

<400> 822
ttgaccctca ggtgtcatgc 20

<210> 823
<211> 20
<212> DNA
<213> Artificial

<220>
<223> oligonucleotide

<400> 823
ccctcaggtg tcatgcggta 20

<210> 824
<211> 20
<212> DNA
<213> Artificial

<220>
<223> oligonucleotide

<400> 824
cctttgaccc tcaggtgtca 20

<210> 825
<211> 20
<212> DNA
<213> Artificial

<220>
<223> oligonucleotide

<400> 825
ctttgaccct caggtgtcat 20

<210> 826
<211> 20
<212> DNA

<213> Artificial

<220>
<223> oligonucleotide

<400> 826
agttatcccc cacccatgga 20

<210> 827
<211> 20
<212> DNA
<213> Artificial

<220>
<223> oligonucleotide

<400> 827
ccagctatcg atcatcgcct 20

<210> 828
<211> 20
<212> DNA
<213> Artificial

<220>
<223> oligonucleotide

<400> 828
accagctatc gatcatcgcc 20

<210> 829
<211> 20
<212> DNA
<213> Artificial

<220>
<223> oligonucleotide

<400> 829
cagctatcga tcatcgcctt 20

<210> 830
<211> 20
<212> DNA
<213> Artificial

<220>
<223> oligonucleotide

<400> 830
agctatcgat catcgccttg 20

<210> 831
<211> 20
<212> DNA
<213> Artificial

<220>

<223> oligonucleotide

<400> 831
gctatcgatc atcgccttgg 20

<210> 832
<211> 20
<212> DNA
<213> Artificial

<220>
<223> oligonucleotide

<400> 832
ctatcgatca tcgccttggt 20

<210> 833
<211> 20
<212> DNA
<213> Artificial

<220>
<223> oligonucleotide

<400> 833
ttcgtgcgac ttgcatgtgt 20

<210> 834
<211> 20
<212> DNA
<213> Artificial

<220>
<223> oligonucleotide

<400> 834
tcgatcatcg ccttggtagg 20

<210> 835
<211> 20
<212> DNA
<213> Artificial

<220>
<223> oligonucleotide

<400> 835
atcgatcatc gccttggtag 20

<210> 836
<211> 20
<212> DNA
<213> Artificial

<220>
<223> oligonucleotide

<400> 836

cacaggcgac ttgcgccttt 20

<210> 837
<211> 20
<212> DNA
<213> Artificial

<220>
<223> oligonucleotide

<400> 837
ccacaggcga cttgcgcctt 20

<210> 838
<211> 20
<212> DNA
<213> Artificial

<220>
<223> oligonucleotide

<400> 838
tccacaggcg acttgcgcct 20

<210> 839
<211> 20
<212> DNA
<213> Artificial

<220>
<223> oligonucleotide

<400> 839
tcctccacag gcgacttgcg 20

<210> 840
<211> 20
<212> DNA
<213> Artificial

<220>
<223> oligonucleotide

<400> 840
cctccacagg cgacttgcgc 20

<210> 841
<211> 20
<212> DNA
<213> Artificial

<220>
<223> oligonucleotide

<400> 841
ctccacaggc gacttgcgcc 20

<210> 842

<211> 20
<212> DNA
<213> Artificial

<220>
<223> oligonucleotide

<400> 842
acaggcgact tgcgcctttg 20

<210> 843
<211> 20
<212> DNA
<213> Artificial

<220>
<223> oligonucleotide

<400> 843
agccccggtt tcccggcgtt 20

<210> 844
<211> 20
<212> DNA
<213> Artificial

<220>
<223> oligonucleotide

<400> 844
cgcctttcct ttttcctcca 20

<210> 845
<211> 20
<212> DNA
<213> Artificial

<220>
<223> oligonucleotide

<400> 845
gccccggttt cccggcgtta 20

<210> 846
<211> 20
<212> DNA
<213> Artificial

<220>
<223> oligonucleotide

<400> 846
gccgcctttc ctttttcctc 20

<210> 847
<211> 20
<212> DNA
<213> Artificial

<220>
<223> oligonucleotide

<400> 847
tagccccggt ttcccggcgt 20

<210> 848
<211> 20
<212> DNA
<213> Artificial

<220>
<223> oligonucleotide

<400> 848
ccgggtaccg tcaaggcgcc 20

<210> 849
<211> 20
<212> DNA
<213> Artificial

<220>
<223> oligonucleotide

<400> 849
aagcccgcctt tcctttttcc 20

<210> 850
<211> 20
<212> DNA
<213> Artificial

<220>
<223> oligonucleotide

<400> 850
ccccggtttc ccggcgttat 20

<210> 851
<211> 20
<212> DNA
<213> Artificial

<220>
<223> oligonucleotide

<400> 851
ccggcgttat cccagtctta 20

<210> 852
<211> 20
<212> DNA
<213> Artificial

<220>
<223> oligonucleotide

<400> 852
agccgccttt cctttttcct 20

<210> 853
<211> 20
<212> DNA
<213> Artificial

<220>
<223> oligonucleotide

<400> 853
ccgcctttcc tttttcctcc 20

<210> 854
<211> 20
<212> DNA
<213> Artificial

<220>
<223> oligonucleotide

<400> 854
ttagccccgg tttcccggcg 20

<210> 855
<211> 20
<212> DNA
<213> Artificial

<220>
<223> oligonucleotide

<400> 855
cccggcgtta tcccagtctt 20

<210> 856
<211> 20
<212> DNA
<213> Artificial

<220>
<223> oligonucleotide

<400> 856
gccgggtacc gtcaaggcgc 20

<210> 857
<211> 20
<212> DNA
<213> Artificial

<220>
<223> oligonucleotide

<400> 857
ggccgggtac cgtcaaggcg 20

<210> 858
<211> 20
<212> DNA
<213> Artificial

<220>
<223> oligonucleotide

<400> 858
tcccggcgtt atcccagtct 20

<210> 859
<211> 20
<212> DNA
<213> Artificial

<220>
<223> oligonucleotide

<400> 859
tggccgggta ccgtcaaggc 20

<210> 860
<211> 20
<212> DNA
<213> Artificial

<220>
<223> oligonucleotide

<400> 860
gaagccgcct ttcctttttc 20

<210> 861
<211> 20
<212> DNA
<213> Artificial

<220>
<223> oligonucleotide

<400> 861
cccggtttcc cggcgttatc 20

<210> 862
<211> 20
<212> DNA
<213> Artificial

<220>
<223> oligonucleotide

<400> 862
cggcgttatc ccagtcttac 20

<210> 863
<211> 20
<212> DNA

<213> Artificial

<220>
<223> oligonucleotide

<400> 863
ggcgttatcc cagtcttaca 20

<210> 864
<211> 20
<212> DNA
<213> Artificial

<220>
<223> oligonucleotide

<400> 864
gcgttatccc agtcttacag 20

<210> 865
<211> 20
<212> DNA
<213> Artificial

<220>
<223> oligonucleotide

<400> 865
cgggtaccgt caaggcgccg 20

<210> 866
<211> 20
<212> DNA
<213> Artificial

<220>
<223> oligonucleotide

<400> 866
attagccccg gtttcccggc 20

<210> 867
<211> 20
<212> DNA
<213> Artificial

<220>
<223> oligonucleotide

<400> 867
aaggggaagg ccctgtctcc 20

<210> 868
<211> 20
<212> DNA
<213> Artificial

<220>

<223> oligonucleotide

<400> 868
ggccctgtct ccagggaggt 20

<210> 869
<211> 20
<212> DNA
<213> Artificial

<220>
<223> oligonucleotide

<400> 869
aggccctgtc tccagggagg 20

<210> 870
<211> 20
<212> DNA
<213> Artificial

<220>
<223> oligonucleotide

<400> 870
aaggccctgt ctccagggag 20

<210> 871
<211> 20
<212> DNA
<213> Artificial

<220>
<223> oligonucleotide

<400> 871
gccctgtctc cagggaggtc 20

<210> 872
<211> 20
<212> DNA
<213> Artificial

<220>
<223> oligonucleotide

<400> 872
cgttatccca gtcttacagg 20

<210> 873
<211> 20
<212> DNA
<213> Artificial

<220>
<223> oligonucleotide

<400> 873

gggtaccgtc aaggcgccgc 20

<210> 874
<211> 20
<212> DNA
<213> Artificial

<220>
<223> oligonucleotide

<400> 874
cggcaacaga gttttacgac 20

<210> 875
<211> 20
<212> DNA
<213> Artificial

<220>
<223> oligonucleotide

<400> 875
ggggaaggcc ctgtctccag 20

<210> 876
<211> 20
<212> DNA
<213> Artificial

<220>
<223> oligonucleotide

<400> 876
aggggaaggc cctgtctcca 20

<210> 877
<211> 20
<212> DNA
<213> Artificial

<220>
<223> oligonucleotide

<400> 877
gcagccgaag ccgcctttcc 20

<210> 878
<211> 20
<212> DNA
<213> Artificial

<220>
<223> oligonucleotide

<400> 878
ttcttccccg gcaacagagt 20

<210> 879

<211> 20
<212> DNA
<213> Artificial

<220>
<223> oligonucleotide

<400> 879
cggcacttgt tcttccccgg 20

<210> 880
<211> 20
<212> DNA
<213> Artificial

<220>
<223> oligonucleotide

<400> 880
gttcttcccc ggcaacagag 20

<210> 881
<211> 20
<212> DNA
<213> Artificial

<220>
<223> oligonucleotide

<400> 881
ggcacttgtt cttccccggc 20

<210> 882
<211> 20
<212> DNA
<213> Artificial

<220>
<223> oligonucleotide

<400> 882
gcacttgttc ttccccggca 20

<210> 883
<211> 20
<212> DNA
<213> Artificial

<220>
<223> oligonucleotide

<400> 883
cacttgttct tccccggcaa 20

<210> 884
<211> 20
<212> DNA
<213> Artificial

<220>
<223> oligonucleotide

<400> 884
tcttccccgg caacagagtt 20

<210> 885
<211> 20
<212> DNA
<213> Artificial

<220>
<223> oligonucleotide

<400> 885
ttgttcttcc ccggcaacag 20

<210> 886
<211> 20
<212> DNA
<213> Artificial

<220>
<223> oligonucleotide

<400> 886
acttgttcrtt ccccggcaac 20

<210> 887
<211> 20
<212> DNA
<213> Artificial

<220>
<223> oligonucleotide

<400> 887
tgttcttccc cggcaacaga 20

<210> 888
<211> 20
<212> DNA
<213> Artificial

<220>
<223> oligonucleotide

<400> 888
cttgttcttc cccggcaaca 20

<210> 889
<211> 20
<212> DNA
<213> Artificial

<220>
<223> oligonucleotide

<400> 889
acggcacttg ttcttccccg 20

<210> 890
<211> 20
<212> DNA
<213> Artificial

<220>
<223> oligonucleotide

<400> 890
gtccgccgct aaccttttaa 20

<210> 891
<211> 20
<212> DNA
<213> Artificial

<220>
<223> oligonucleotide

<400> 891
ctggccgggt accgtcaagg 20

<210> 892
<211> 20
<212> DNA
<213> Artificial

<220>
<223> oligonucleotide

<400> 892
tctggccggg taccgtcaag 20

<210> 893
<211> 20
<212> DNA
<213> Artificial

<220>
<223> oligonucleotide

<400> 893
ttctggccgg gtaccgtcaa 20

<210> 894
<211> 20
<212> DNA
<213> Artificial

<220>
<223> oligonucleotide

<400> 894
caatgctggc aactaaggtc 20

<210> 895
<211> 20
<212> DNA
<213> Artificial

<220>
<223> oligonucleotide

<400> 895
cgtccgccgc taacctttta 20

<210> 896
<211> 20
<212> DNA
<213> Artificial

<220>
<223> oligonucleotide

<400> 896
cgaagccgcc tttccttttt 20

<210> 897
<211> 20
<212> DNA
<213> Artificial

<220>
<223> oligonucleotide

<400> 897
ccgaagccgc ctttcctttt 20

<210> 898
<211> 20
<212> DNA
<213> Artificial

<220>
<223> oligonucleotide

<400> 898
gccgaagccg cctttccttt 20

<210> 899
<211> 20
<212> DNA
<213> Artificial

<220>
<223> oligonucleotide

<400> 899
agccgaagcc gcctttcctt 20

<210> 900
<211> 20
<212> DNA

<213> Artificial

<220>
<223> oligonucleotide

<400> 900
accgtcaagg cgccgccctg 20

<210> 901
<211> 20
<212> DNA
<213> Artificial

<220>
<223> oligonucleotide

<400> 901
ccgtggcttt ctggccgggt 20

<210> 902
<211> 20
<212> DNA
<213> Artificial

<220>
<223> oligonucleotide

<400> 902
gctttctggc cgggtaccgt 20

<210> 903
<211> 20
<212> DNA
<213> Artificial

<220>
<223> oligonucleotide

<400> 903
gccgtggctt tctggccggg 20

<210> 904
<211> 20
<212> DNA
<213> Artificial

<220>
<223> oligonucleotide

<400> 904
ggctttctgg ccgggtaccg 20

<210> 905
<211> 20
<212> DNA
<213> Artificial

<220>

<223> oligonucleotide

<400> 905
ctttctggcc gggtaccgtc 20

<210> 906
<211> 20
<212> DNA
<213> Artificial

<220>
<223> oligonucleotide

<400> 906
tggctttctg gccgggtacc 20

<210> 907
<211> 20
<212> DNA
<213> Artificial

<220>
<223> oligonucleotide

<400> 907
gtggctttct ggccgggtac 20

<210> 908
<211> 20
<212> DNA
<213> Artificial

<220>
<223> oligonucleotide

<400> 908
cgtggctttc tggccgggta 20

<210> 909
<211> 20
<212> DNA
<213> Artificial

<220>
<223> oligonucleotide

<400> 909
tttctggccg ggtaccgtca 20

<210> 910
<211> 20
<212> DNA
<213> Artificial

<220>
<223> oligonucleotide

<400> 910

gggaaggccc tgtctccagg 20

<210> 911
<211> 20
<212> DNA
<213> Artificial

<220>
<223> oligonucleotide

<400> 911
cgaaggggaa ggccctgtct 20

<210> 912
<211> 20
<212> DNA
<213> Artificial

<220>
<223> oligonucleotide

<400> 912
ccgaagggga aggccctgtc 20

<210> 913
<211> 20
<212> DNA
<213> Artificial

<220>
<223> oligonucleotide

<400> 913
gaaggggaag gccctgtctc 20

<210> 914
<211> 20
<212> DNA
<213> Artificial

<220>
<223> oligonucleotide

<400> 914
ggcgccgccc tgttcgaacg 20

<210> 915
<211> 20
<212> DNA
<213> Artificial

<220>
<223> oligonucleotide

<400> 915
aggcgccgcc ctgttcgaac 20

<210> 916

<211> 20
<212> DNA
<213> Artificial

<220>
<223> oligonucleotide

<400> 916
aaggcgccgc cctgttcgaa 20

<210> 917
<211> 20
<212> DNA
<213> Artificial

<220>
<223> oligonucleotide

<400> 917
cccggcaaca gagttttacg 20

<210> 918
<211> 20
<212> DNA
<213> Artificial

<220>
<223> oligonucleotide

<400> 918 20
ccccggcaac agagttttac 20

<210> 919
<211> 20
<212> DNA
<213> Artificial

<220>
<223> oligonucleotide

<400> 919 20
ccatctgtaa gtggcagccg 20

<210> 920
<211> 20
<212> DNA
<213> Artificial

<220>
<223> oligonucleotide

<400> 920 20
tctgtaagtg gcagccgaag 20

<210> 921
<211> 20
<212> DNA
<213> Artificial

<220>
<223> oligonucleotide

<400> 921 20
ctgtaagtgg cagccgaagc 20

<210> 922
<211> 20
<212> DNA
<213> Artificial

<220>
<223> oligonucleotide

<400> 922 20
cccatctgta agtggcagcc 20

<210> 923
<211> 20
<212> DNA
<213> Artificial

<220>
<223> oligonucleotide

<400> 923
tgtaagtggc agccgaagcc 20

<210> 924
<211> 20
<212> DNA
<213> Artificial

<220>
<223> oligonucleotide

<400> 924
catctgtaag tggcagccga 20

<210> 925
<211> 20
<212> DNA
<213> Artificial

<220>
<223> oligonucleotide

<400> 925
atctgtaagt ggcagccgaa 20

<210> 926
<211> 20
<212> DNA
<213> Artificial

<220>
<223> oligonucleotide

<400> 926
cagccgaagc cgcctttcct 20

<210> 927
<211> 20
<212> DNA
<213> Artificial

<220>
<223> oligonucleotide

<400> 927
ggcaacagag ttttacgacc 20

<210> 928
<211> 20
<212> DNA
<213> Artificial

<220>
<223> oligonucleotide

<400> 928
ccggcaacag agttttacga 20

<210> 929
<211> 20
<212> DNA
<213> Artificial

<220>
<223> oligonucleotide

<400> 929
ttccccggca acagagtttt 20

<210> 930
<211> 20
<212> DNA
<213> Artificial

<220>
<223> oligonucleotide

<400> 930
cttccccggc aacagagttt 20

<210> 931
<211> 20
<212> DNA
<213> Artificial

<220>
<223> oligonucleotide

<400> 931
tccccggcaa cagagtttta 20

<210> 932
<211> 20
<212> DNA
<213> Artificial

<220>
<223> oligonucleotide

<400> 932
ccgtccgccg ctaacctttt 20

<210> 933
<211> 20
<212> DNA
<213> Artificial

<220>
<223> oligonucleotide

<400> 933
cttcctccga cttacgccgg 20

<210> 934
<211> 20
<212> DNA
<213> Artificial

<220>
<223> oligonucleotide

<400> 934
cctccgactt acgccggcag 20

<210> 935
<211> 20
<212> DNA
<213> Artificial

<220>
<223> oligonucleotide

<400> 935
ttcctccgac ttacgccggc 20

<210> 936
<211> 20
<212> DNA
<213> Artificial

<220>
<223> oligonucleotide

<400> 936
tcctccgact tacgccggca 20

<210> 937
<211> 20
<212> DNA

<213> Artificial

<220>
<223> oligonucleotide

<400> 937
tccgacttac gccggcagtc 20

<210> 938
<211> 20
<212> DNA
<213> Artificial

<220>
<223> oligonucleotide

<400> 938
ccgacttacg ccggcagtca 20

<210> 939
<211> 20
<212> DNA
<213> Artificial

<220>
<223> oligonucleotide

<400> 939
gccttcctcc gacttacgcc 20

<210> 940
<211> 20
<212> DNA
<213> Artificial

<220>
<223> oligonucleotide

<400> 940
ccttcctccg acttacgccg 20

<210> 941
<211> 20
<212> DNA
<213> Artificial

<220>
<223> oligonucleotide

<400> 941
gctctccccg agcaacagag 20

<210> 942
<211> 20
<212> DNA
<213> Artificial

<220>

<223> oligonucleotide

<400> 942
ctctccccga gcaacagagc 20

<210> 943
<211> 20
<212> DNA
<213> Artificial

<220>
<223> oligonucleotide

<400> 943
cgctctcccc gagcaacaga 20

<210> 944
<211> 20
<212> DNA
<213> Artificial

<220>
<223> oligonucleotide

<400> 944
ctccgactta cgccggcagt 20

<210> 945
<211> 20
<212> DNA
<213> Artificial

<220>
<223> oligonucleotide

<400> 945
tctccccgag caacagagct 20

<210> 946
<211> 20
<212> DNA
<213> Artificial

<220>
<223> oligonucleotide

<400> 946
cgacttacgc cggcagtcac 20

<210> 947
<211> 20
<212> DNA
<213> Artificial

<220>
<223> oligonucleotide

<400> 947

tcggcactgg ggtgtgtccc 20

<210> 948
<211> 20
<212> DNA
<213> Artificial

<220>
<223> oligonucleotide

<400> 948 20
ggcactgggg tgtgtccccc 20

<210> 949
<211> 20
<212> DNA
<213> Artificial

<220>
<223> oligonucleotide

<400> 949
ctggggtgtg tccccccaac 20

<210> 950
<211> 20
<212> DNA
<213> Artificial

<220>
<223> oligonucleotide

<400> 950
cactggggtg tgtcccccca 20

<210> 951
<211> 20
<212> DNA
<213> Artificial

<220>
<223> oligonucleotide

<400> 951 20
actggggtgt gtccccccaa 20

<210> 952
<211> 20
<212> DNA
<213> Artificial

<220>
<223> oligonucleotide

<400> 952 20
gcactggggt gtgtcccccc 20

<210> 953

<211> 20
<212> DNA
<213> Artificial

<220>
<223> oligonucleotide

<400> 953
tggggtgtgt ccccccaaca 20

<210> 954
<211> 20
<212> DNA
<213> Artificial

<220>
<223> oligonucleotide

<400> 954 20
cactccagac ttgctcgacc 20

<210> 955
<211> 20
<212> DNA
<213> Artificial

<220>
<223> oligonucleotide

<400> 955 20
tcactccaga cttgctcgac 20

<210> 956
<211> 20
<212> DNA
<213> Artificial

<220>
<223> oligonucleotide

<400> 956
cggcactggg gtgtgtcccc 20

<210> 957
<211> 20
<212> DNA
<213> Artificial

<220>
<223> oligonucleotide

<400> 957
cgccttcctc cgacttacgc 20

<210> 958
<211> 20
<212> DNA
<213> Artificial

<220>
<223> oligonucleotide

<400> 958
ctccccgagc aacagagctt 20

<210> 959
<211> 20
<212> DNA
<213> Artificial

<220>
<223> oligonucleotide

<400> 959
actccagact tgctcgaccg 20

<210> 960
<211> 20
<212> DNA
<213> Artificial

<220>
<223> oligonucleotide

<400> 960
cccatgccgc tctccccgag 20

<210> 961
<211> 20
<212> DNA
<213> Artificial

<220>
<223> oligonucleotide

<400> 961
ccatgccgct ctccccgagc 20

<210> 962
<211> 20
<212> DNA
<213> Artificial

<220>
<223> oligonucleotide

<400> 962
ccccatgccg ctctccccga 20

<210> 963
<211> 20
<212> DNA
<213> Artificial

<220>
<223> oligonucleotide

<400> 963
tcactcggta ccgtctcgca 20

<210> 964
<211> 20
<212> DNA
<213> Artificial

<220>
<223> oligonucleotide

<400> 964
catgccgctc tccccgagca 20

<210> 965
<211> 20
<212> DNA
<213> Artificial

<220>
<223> oligonucleotide

<400> 965
atgccgctct ccccgagcaa 20

<210> 966
<211> 20
<212> DNA
<213> Artificial

<220>
<223> oligonucleotide

<400> 966
ttcggcactg gggtgtgtcc 20

<210> 967
<211> 20
<212> DNA
<213> Artificial

<220>
<223> oligonucleotide

<400> 967
tgccgctctc cccgagcaac 20

<210> 968
<211> 20
<212> DNA
<213> Artificial

<220>
<223> oligonucleotide

<400> 968
ttcactccag acttgctcga 20

<210> 969
<211> 20
<212> DNA
<213> Artificial

<220>
<223> oligonucleotide

<400> 969
cccgcaagaa gatgcctcct 20

<210> 970
<211> 20
<212> DNA
<213> Artificial

<220>
<223> oligonucleotide

<400> 970
agaagatgcc tcctcgcggg 20

<210> 971
<211> 20
<212> DNA
<213> Artificial

<220>
<223> oligonucleotide

<400> 971
aagaagatgc ctcctcgcgg 20

<210> 972
<211> 20
<212> DNA
<213> Artificial

<220>
<223> oligonucleotide

<400> 972
cgcaagaaga tgcctcctcg 20

<210> 973
<211> 20
<212> DNA
<213> Artificial

<220>
<223> oligonucleotide

<400> 973
aagatgcctc ctcgcgggcg 20

<210> 974
<211> 20
<212> DNA

<213> Artificial

<220>
<223> oligonucleotide

<400> 974
ccgcaagaag atgcctcctc 20

<210> 975
<211> 20
<212> DNA
<213> Artificial

<220>
<223> oligonucleotide

<400> 975
gaagatgcct cctcgcgggc 20

<210> 976
<211> 20
<212> DNA
<213> Artificial

<220>
<223> oligonucleotide

<400> 976
ccccgcaaga agatgcctcc 20

<210> 977
<211> 20
<212> DNA
<213> Artificial

<220>
<223> oligonucleotide

<400> 977
caagaagatg cctcctcgcg 20

<210> 978
<211> 20
<212> DNA
<213> Artificial

<220>
<223> oligonucleotide

<400> 978
tccttcggca ctggggtgtg 20

<210> 979
<211> 20
<212> DNA
<213> Artificial

<220>

<223> oligonucleotide

<400> 979
ccgctctccc cgagcaacag 20

<210> 980
<211> 20
<212> DNA
<213> Artificial

<220>
<223> oligonucleotide

<400> 980
tgcctcctcg cgggcgtatc 20

<210> 981
<211> 20
<212> DNA
<213> Artificial

<220>
<223> oligonucleotide

<400> 981
gacttacgcc ggcagtcacc 20

<210> 982
<211> 20
<212> DNA
<213> Artificial

<220>
<223> oligonucleotide

<400> 982
ggctcctctc tcagcggccc 20

<210> 983
<211> 20
<212> DNA
<213> Artificial

<220>
<223> oligonucleotide

<400> 983
ccttcggcac tggggtgtgt 20

<210> 984
<211> 20
<212> DNA
<213> Artificial

<220>
<223> oligonucleotide

<400> 984

ggggtgtgtc cccccaacac 20

<210> 985
<211> 20
<212> DNA
<213> Artificial

<220>
<223> oligonucleotide

<400> 985
gccgctctcc ccgagcaaca 20

<210> 986
<211> 20
<212> DNA
<213> Artificial

<220>
<223> oligonucleotide

<400> 986
agatgcctcc tcgcgggcgt 20

<210> 987
<211> 20
<212> DNA
<213> Artificial

<220>
<223> oligonucleotide

<400> 987
cactcggtac cgtctcgcat 20

<210> 988
<211> 20
<212> DNA
<213> Artificial

<220>
<223> oligonucleotide

<400> 988
ctcactcggt accgtctcgc 20

<210> 989
<211> 20
<212> DNA
<213> Artificial

<220>
<223> oligonucleotide

<400> 989
gcaagaagat gcctcctcgc 20

<210> 990

<211> 20
<212> DNA
<213> Artificial

<220>
<223> oligonucleotide

<400> 990
ctccagactt gctcgaccgc 20

<210> 991
<211> 20
<212> DNA
<213> Artificial

<220>
<223> oligonucleotide

<400> 991
ttacgccggc agtcacctgt 20

<210> 992
<211> 20
<212> DNA
<213> Artificial

<220>
<223> oligonucleotide

<400> 992
cttcggcact ggggtgtgtc 20

<210> 993
<211> 20
<212> DNA
<213> Artificial

<220>
<223> oligonucleotide

<400> 993
ctcgcgggcg tatccggcat 20

<210> 994
<211> 20
<212> DNA
<213> Artificial

<220>
<223> oligonucleotide

<400> 994
gcctcctcgc gggcgtatcc 20

<210> 995
<211> 20
<212> DNA
<213> Artificial

<220>
<223> oligonucleotide

<400> 995
actcggtacc gtctcgcatg 20

<210> 996
<211> 20
<212> DNA
<213> Artificial

<220>
<223> oligonucleotide

<400> 996
gatgcctcct cgcgggcgta 20

<210> 997
<211> 20
<212> DNA
<213> Artificial

<220>
<223> oligonucleotide

<400> 997
gggtgtgtcc ccccaacacc 20

<210> 998
<211> 20
<212> DNA
<213> Artificial

<220>
<223> oligonucleotide

<400> 998
acttacgccg gcagtcacct 20

<210> 999
<211> 20
<212> DNA
<213> Artificial

<220>
<223> oligonucleotide

<400> 999
cttacgccgg cagtcacctg 20

<210> 1000
<211> 20
<212> DNA
<213> Artificial

<220>
<223> oligonucleotide

<400> 1000
atgcctcctc gcgggcgtat 20

<210> 1001
<211> 20
<212> DNA
<213> Artificial

<220>
<223> oligonucleotide

<400> 1001
gcgccgcggg ctcctctctc 20

<210> 1002
<211> 20
<212> DNA
<213> Artificial

<220>
<223> oligonucleotide

<400> 1002
ggtgtgtccc cccaacacct 20

<210> 1003
<211> 20
<212> DNA
<213> Artificial

<220>
<223> oligonucleotide

<400> 1003
gtgtgtcccc ccaacaccta 20

<210> 1004
<211> 20
<212> DNA
<213> Artificial

<220>
<223> oligonucleotide

<400> 1004
cctcgcgggc gtatccggca 20

<210> 1005
<211> 20
<212> DNA
<213> Artificial

<220>
<223> oligonucleotide

<400> 1005
cctcactcgg taccgtctcg 20

<210> 1006
<211> 20
<212> DNA
<213> Artificial

<220>
<223> oligonucleotide

<400> 1006
tcctcactcg gtaccgtctc 20

<210> 1007
<211> 20
<212> DNA
<213> Artificial

<220>
<223> oligonucleotide

<400> 1007
tcgcgggcgt atccggcatt 20

<210> 1008
<211> 20
<212> DNA
<213> Artificial

<220>
<223> oligonucleotide

<400> 1008
tttcactcca gacttgctcg 20

<210> 1009
<211> 20
<212> DNA
<213> Artificial

<220>
<223> oligonucleotide

<400> 1009
tacgccggca gtcacctgtg 20

<210> 1010
<211> 20
<212> DNA
<213> Artificial

<220>
<223> oligonucleotide

<400> 1010
tccagacttg ctcgaccgcc 20

<210> 1011
<211> 20
<212> DNA

<213> Artificial

<220>
<223> oligonucleotide

<400> 1011
ctcggtaccg tctcgcatgg 20

<210> 1012
<211> 20
<212> DNA
<213> Artificial

<220>
<223> oligonucleotide

<400> 1012
cgcgggcgta tccggcatta 20

<210> 1013
<211> 20
<212> DNA
<213> Artificial

<220>
<223> oligonucleotide

<400> 1013
gcgtatccgg cattagcgcc 20

<210> 1014
<211> 20
<212> DNA
<213> Artificial

<220>
<223> oligonucleotide

<400> 1014
gggctcctct ctcagcggcc 20

<210> 1015
<211> 20
<212> DNA
<213> Artificial

<220>
<223> oligonucleotide

<400> 1015
tccccgagca acagagcttt 20

<210> 1016
<211> 20
<212> DNA
<213> Artificial

<220>

<223> oligonucleotide

<400> 1016
ccccgagcaa cagagcttta 20

<210> 1017
<211> 20
<212> DNA
<213> Artificial

<220>
<223> oligonucleotide

<400> 1017
ccgagcaaca gagctttaca 20

<210> 1018
<211> 20
<212> DNA
<213> Artificial

<220>
<223> oligonucleotide

<400> 1018
ccatcccatg gttgagccat 20

<210> 1019
<211> 20
<212> DNA
<213> Artificial

<220>
<223> oligonucleotide

<400> 1019
gtgtcccccc aacacctagc 20

<210> 1020
<211> 20
<212> DNA
<213> Artificial

<220>
<223> oligonucleotide

<400> 1020
gcgggcgtat ccggcattag 20

<210> 1021
<211> 20
<212> DNA
<213> Artificial

<220>
<223> oligonucleotide

<400> 1021 20

cgagcggctt tttgggtttc 20

<210> 1022
<211> 20
<212> DNA
<213> Artificial

<220>
<223> oligonucleotide

<400> 1022
ctttcactcc agacttgctc 20

<210> 1023
<211> 20
<212> DNA
<213> Artificial

<220>
<223> oligonucleotide

<400> 1023
ttccttcggc actggggtgt 20

<210> 1024
<211> 20
<212> DNA
<213> Artificial

<220>
<223> oligonucleotide

<400> 1024
ccgccttcct ccgacttacg 20

<210> 1025
<211> 20
<212> DNA
<213> Artificial

<220>
<223> oligonucleotide

<400> 1025
cccgccttcc tccgacttac 20

<210> 1026
<211> 20
<212> DNA
<213> Artificial

<220>
<223> oligonucleotide

<400> 1026
cctcctcgcg ggcgtatccg 20

<210> 1027

<211> 20
<212> DNA
<213> Artificial

<220>
<223> oligonucleotide

<400> 1027
tcctcgcggg cgtatccggc 20

<210> 1028
<211> 20
<212> DNA
<213> Artificial

<220>
<223> oligonucleotide

<400> 1028
cattagcgcc cgtttccggg 20

<210> 1029
<211> 20
<212> DNA
<213> Artificial

<220>
<223> oligonucleotide

<400> 1029
gcattagcgc ccgtttccgg 20

<210> 1030
<211> 20
<212> DNA
<213> Artificial

<220>
<223> oligonucleotide

<400> 1030
ggcattagcg cccgtttccg 20

<210> 1031
<211> 20
<212> DNA
<213> Artificial

<220>
<223> oligonucleotide

<400> 1031
gtctcgcatg gggctttcca 20

<210> 1032
<211> 20
<212> DNA
<213> Artificial

<220>
<223> oligonucleotide

<400> 1032
gccatggact ttcactccag 20

<210> 1033
<211> 20
<212> DNA
<213> Artificial

<220>
<223> oligonucleotide

<400> 1033
catggacttt cactccagac 20

<210> 1034
<211> 22
<212> DNA
<213> Artificial

<220>
<223> oligonucleotide

<400> 1034
ccttcctccg gcttacgccg gc 22

<210> 1035
<211> 22
<212> DNA
<213> Artificial

<220>
<223> oligonucleotide

<400> 1035
ccttcctccg acttgcgccg gc 22

<210> 1036
<211> 22
<212> DNA
<213> Artificial

<220>
<223> oligonucleotide

<400> 1036
ccttcctccg actttcaccg gc 22

<210> 1037
<211> 20
<212> DNA
<213> Artificial

<220>
<223> oligonucleotide

<400> 1037
accgtctcac aaggagcttt 20

<210> 1038
<211> 20
<212> DNA
<213> Artificial

<220>
<223> oligonucleotide

<400> 1038
taccgtctca caaggagctt 20

<210> 1039
<211> 20
<212> DNA
<213> Artificial

<220>
<223> oligonucleotide

<400> 1039
gtaccgtctc acaaggagct 20

<210> 1040
<211> 20
<212> DNA
<213> Artificial

<220>
<223> oligonucleotide

<400> 1040
gcctacccgt gtattatccg 20

<210> 1041
<211> 20
<212> DNA
<213> Artificial

<220>
<223> oligonucleotide

<400> 1041
ccgtctcaca aggagctttc 20

<210> 1042
<211> 20
<212> DNA
<213> Artificial

<220>
<223> oligonucleotide

<400> 1042 20
ctacccgtgt attatccggc 20

<210> 1043
<211> 20
<212> DNA
<213> Artificial

<220>
<223> oligonucleotide

<400> 1043 20
ggtaccgtct cacaaggagc 20

<210> 1044
<211> 20
<212> DNA
<213> Artificial

<220>
<223> oligonucleotide

<400> 1044 20
cgtctcacaa ggagctttcc 20

<210> 1045
<211> 20
<212> DNA
<213> Artificial

<220>
<223> oligonucleotide

<400> 1045 20
tctcacaagg agctttccac 20

<210> 1046
<211> 20
<212> DNA
<213> Artificial

<220>
<223> oligonucleotide

<400> 1046 20
tacccgtgta ttatccggca 20

<210> 1047
<211> 20
<212> DNA
<213> Artificial

<220>
<223> oligonucleotide

<400> 1047
gtctcacaag gagctttcca 20

<210> 1048
<211> 20
<212> DNA

<213> Artificial

<220>
<223> oligonucleotide

<400> 1048
acccgtgtat tatccggcat 20

<210> 1049
<211> 20
<212> DNA
<213> Artificial

<220>
<223> oligonucleotide

<400> 1049
ctcggtaccg tctcacaagg 20

<210> 1050
<211> 20
<212> DNA
<213> Artificial

<220>
<223> oligonucleotide

<400> 1050
cggtaccgtc tcacaaggag 20

<210> 1051
<211> 20
<212> DNA
<213> Artificial

<220>
<223> oligonucleotide

<400> 1051
actcggtacc gtctcacaag 20

<210> 1052
<211> 20
<212> DNA
<213> Artificial

<220>
<223> oligonucleotide

<400> 1052
cggctggctc cataacggtt 20

<210> 1053
<211> 20
<212> DNA
<213> Artificial

<220>

<223> oligonucleotide

<400> 1053
acaagtagat gcctacccgt 20

<210> 1054
<211> 20
<212> DNA
<213> Artificial

<220>
<223> oligonucleotide

<400> 1054
tggctccata acggttacct 20

<210> 1055
<211> 20
<212> DNA
<213> Artificial

<220>
<223> oligonucleotide

<400> 1055
caagtagatg cctacccgtg 20

<210> 1056
<211> 20
<212> DNA
<213> Artificial

<220>
<223> oligonucleotide

<400> 1056
cacaagtaga tgcctacccg 20

<210> 1057
<211> 20
<212> DNA
<213> Artificial

<220>
<223> oligonucleotide

<400> 1057
ggctccataa cggttacctc 20

<210> 1058
<211> 20
<212> DNA
<213> Artificial

<220>
<223> oligonucleotide

<400> 1058

acacaagtag atgcctaccc 20

<210> 1059
<211> 20
<212> DNA
<213> Artificial

<220>
<223> oligonucleotide

<400> 1059
ctggctccat aacggttacc 20

<210> 1060
<211> 20
<212> DNA
<213> Artificial

<220>
<223> oligonucleotide

<400> 1060
gctggctcca taacggttac 20

<210> 1061
<211> 20
<212> DNA
<213> Artificial

<220>
<223> oligonucleotide

<400> 1061
ggctggctcc ataacggtta 20

<210> 1062
<211> 20
<212> DNA
<213> Artificial

<220>
<223> oligonucleotide

<400> 1062 20
gctccataac ggttacctca 20

<210> 1063
<211> 20
<212> DNA
<213> Artificial

<220>
<223> oligonucleotide

<400> 1063
aagtagatgc ctacccgtgt 20

<210> 1064

<211> 20
<212> DNA
<213> Artificial

<220>
<223> oligonucleotide

<400> 1064
ctccataacg gttacctcac 20

<210> 1065
<211> 20
<212> DNA
<213> Artificial

<220>
<223> oligonucleotide

<400> 1065
tgcctacccg tgtattatcc 20

<210> 1066
<211> 20
<212> DNA
<213> Artificial

<220>
<223> oligonucleotide

<400> 1066
tcggtaccgt ctcacaagga 20

<210> 1067
<211> 20
<212> DNA
<213> Artificial

<220>
<223> oligonucleotide

<400> 1067
ctcacaagga gctttccact 20

<210> 1068
<211> 20
<212> DNA
<213> Artificial

<220>
<223> oligonucleotide

<400> 1068
gtagatgcct acccgtgtat 20

<210> 1069
<211> 20
<212> DNA
<213> Artificial

<220>
<223> oligonucleotide

<400> 1069
cctacccgtg tattatccgg 20

<210> 1070
<211> 20
<212> DNA
<213> Artificial

<220>
<223> oligonucleotide

<400> 1070
cactcggtac cgtctcacaa 20

<210> 1071
<211> 20
<212> DNA
<213> Artificial

<220>
<223> oligonucleotide

<400> 1071
ctcagcgatg cagttgcatc 20

<210> 1072
<211> 20
<212> DNA
<213> Artificial

<220>
<223> oligonucleotide

<400> 1072
agtagatgcc tacccgtgta 20

<210> 1073
<211> 20
<212> DNA
<213> Artificial

<220>
<223> oligonucleotide

<400> 1073
gcggctggct ccataacggt 20

<210> 1074
<211> 20
<212> DNA
<213> Artificial

<220>
<223> oligonucleotide

<400> 1074
ccaaagcaat cccaaggttg 20

<210> 1075
<211> 20
<212> DNA
<213> Artificial

<220>
<223> oligonucleotide

<400> 1075
tccataacgg ttacctcacc 20

<210> 1076
<211> 20
<212> DNA
<213> Artificial

<220>
<223> oligonucleotide

<400> 1076
cccgtgtatt atccggcatt 20

<210> 1077
<211> 20
<212> DNA
<213> Artificial

<220>
<223> oligonucleotide

<400> 1077
tctcagcgat gcagttgcat 20

<210> 1078
<211> 20
<212> DNA
<213> Artificial

<220>
<223> oligonucleotide

<400> 1078
ccataacggt tacctcaccg 20

<210> 1079
<211> 20
<212> DNA
<213> Artificial

<220>
<223> oligonucleotide

<400> 1079 20
tcagcgatgc agttgcatct 20

<210> 1080
<211> 20
<212> DNA
<213> Artificial

<220>
<223> oligonucleotide

<400> 1080
ggcggctggc tccataacgg 20

<210> 1081
<211> 20
<212> DNA
<213> Artificial

<220>
<223> oligonucleotide

<400> 1081
aagcaatccc aaggttgagc 20

<210> 1082
<211> 20
<212> DNA
<213> Artificial

<220>
<223> oligonucleotide

<400> 1082 20
tcactcggta ccgtctcaca 20

<210> 1083
<211> 20
<212> DNA
<213> Artificial

<220>
<223> oligonucleotide

<400> 1083 20
ccgagtgtta ttccagtctg 20

<210> 1084
<211> 20
<212> DNA
<213> Artificial

<220>
<223> oligonucleotide

<400> 1084 20
cacaaggagc tttccactct 20

<210> 1085
<211> 20
<212> DNA

<213> Artificial

<220>
<223> oligonucleotide

<400> 1085 20
acaaggagct ttccactctc 20

<210> 1086
<211> 20
<212> DNA
<213> Artificial

<220>
<223> oligonucleotide

<400> 1086 20
tcacaaggag ctttccactc 20

<210> 1087
<211> 20
<212> DNA
<213> Artificial

<220>
<223> oligonucleotide

<400> 1087
cagcgatgca gttgcatctt 20

<210> 1088
<211> 20
<212> DNA
<213> Artificial

<220>
<223> oligonucleotide

<400> 1088
caaggagctt tccactctcc 20

<210> 1089
<211> 20
<212> DNA
<213> Artificial

<220>
<223> oligonucleotide

<400> 1089
ccagtctgaa aggcagattg 20

<210> 1090
<211> 20
<212> DNA
<213> Artificial

<220>

<223> oligonucleotide

<400> 1090
cagtctgaaa ggcagattgc 20

<210> 1091
<211> 20
<212> DNA
<213> Artificial

<220>
<223> oligonucleotide

<400> 1091
cggcggctgg ctccataacg 20

<210> 1092
<211> 20
<212> DNA
<213> Artificial

<220>
<223> oligonucleotide

<400> 1092
cctctctcag cgatgcagtt 20

<210> 1093
<211> 20
<212> DNA
<213> Artificial

<220>
<223> oligonucleotide

<400> 1093 20
ctctctcagc gatgcagttg 20

<210> 1094
<211> 20
<212> DNA
<213> Artificial

<220>
<223> oligonucleotide

<400> 1094 20
tctctcagcg atgcagttgc 20

<210> 1095
<211> 20
<212> DNA
<213> Artificial

<220>
<223> oligonucleotide

<400> 1095 20

ctctcagcga tgcagttgca 20

<210> 1096
<211> 20
<212> DNA
<213> Artificial

<220>
<223> oligonucleotide

<400> 1096 20
caatcccaag gttgagcctt 20

<210> 1097
<211> 20
<212> DNA
<213> Artificial

<220>
<223> oligonucleotide

<400> 1097 20
aatcccaagg ttgagccttg 20

<210> 1098
<211> 20
<212> DNA
<213> Artificial

<220>
<223> oligonucleotide

<400> 1098
agcaatccca aggttgagcc 20

<210> 1099
<211> 20
<212> DNA
<213> Artificial

<220>
<223> oligonucleotide

<400> 1099
ctcactcggt accgtctcac 20

<210> 1100
<211> 20
<212> DNA
<213> Artificial

<220>
<223> oligonucleotide

<400> 1100
gcaatcccaa ggttgagcct 20

<210> 1101

<211> 20
<212> DNA
<213> Artificial

<220>
<223> oligonucleotide

<400> 1101
gccttggact ttcacttcag 20

<210> 1102
<211> 20
<212> DNA
<213> Artificial

<220>
<223> oligonucleotide

<400> 1102
cataacggtt acctcaccga 20

<210> 1103
<211> 20
<212> DNA
<213> Artificial

<220>
<223> oligonucleotide

<400> 1103 20
ctcctctctc agcgatgcag 20

<210> 1104
<211> 20
<212> DNA
<213> Artificial

<220>
<223> oligonucleotide

<400> 1104
tcggcggctg gctccataac 20

<210> 1105
<211> 20
<212> DNA
<213> Artificial

<220>
<223> oligonucleotide

<400> 1105
agtctgaaag gcagattgcc 20

<210> 1106
<211> 20
<212> DNA
<213> Artificial

<220>
<223> oligonucleotide

<400> 1106
tcctctctca gcgatgcagt 20

<210> 1107
<211> 20
<212> DNA
<213> Artificial

<220>
<223> oligonucleotide

<400> 1107
cccaaggttg agccttggac 20

<210> 1108
<211> 20
<212> DNA
<213> Artificial

<220>
<223> oligonucleotide

<400> 1108
ataacggtta cctcaccgac 20

<210> 1109
<211> 20
<212> DNA
<213> Artificial

<220>
<223> oligonucleotide

<400> 1109
tcccaaggtt gagccttgga 20

<210> 1110
<211> 20
<212> DNA
<213> Artificial

<220>
<223> oligonucleotide

<400> 1110
attatccggc attagcaccc 20

<210> 1111
<211> 20
<212> DNA
<213> Artificial

<220>
<223> oligonucleotide

<400> 1111
ctacgtgctg gtaacacaga 20

<210> 1112
<211> 20
<212> DNA
<213> Artificial

<220>
<223> oligonucleotide

<400> 1112
gccgctagcc ccgaagggct 20

<210> 1113
<211> 20
<212> DNA
<213> Artificial

<220>
<223> oligonucleotide

<400> 1113
ctagccccga agggctcgct 20

<210> 1114
<211> 20
<212> DNA
<213> Artificial

<220>
<223> oligonucleotide

<400> 1114
cgctagcccc gaagggctcg 20

<210> 1115
<211> 20
<212> DNA
<213> Artificial

<220>
<223> oligonucleotide

<400> 1115
agccccgaag ggctcgctcg 20

<210> 1116
<211> 20
<212> DNA
<213> Artificial

<220>
<223> oligonucleotide

<400> 1116
ccgctagccc cgaagggctc 20

<210> 1117
<211> 20
<212> DNA
<213> Artificial

<220>
<223> oligonucleotide

<400> 1117
tagccccgaa gggctcgctc 20

<210> 1118
<211> 20
<212> DNA
<213> Artificial

<220>
<223> oligonucleotide

<400> 1118
gctagccccg aagggctcgc 20

<210> 1119
<211> 20
<212> DNA
<213> Artificial

<220>
<223> oligonucleotide

<400> 1119
gccccgaagg gctcgctcga 20

<210> 1120
<211> 20
<212> DNA
<213> Artificial

<220>
<223> oligonucleotide

<400> 1120
atcccaaggt tgagccttgg 20

<210> 1121
<211> 20
<212> DNA
<213> Artificial

<220>
<223> oligonucleotide

<400> 1121
gagccttgga ctttcacttc 20

<210> 1122
<211> 20
<212> DNA

<213> Artificial

<220>
<223> oligonucleotide

<400> 1122
caaggttgag ccttggactt 20

<210> 1123
<211> 20
<212> DNA
<213> Artificial

<220>
<223> oligonucleotide

<400> 1123
gagctttcca ctctccttgt 20

<210> 1124
<211> 20
<212> DNA
<213> Artificial

<220>
<223> oligonucleotide

<400> 1124
ccaaggttga gccttggact 20

<210> 1125
<211> 20
<212> DNA
<213> Artificial

<220>
<223> oligonucleotide

<400> 1125
cgggctcctc tctcagcgat 20

<210> 1126
<211> 20
<212> DNA
<213> Artificial

<220>
<223> oligonucleotide

<400> 1126
ggagctttcc actctccttg 20

<210> 1127
<211> 20
<212> DNA
<213> Artificial

<220>

<223> oligonucleotide

<400> 1127 20
gggctcctct ctcagcgatg 20

<210> 1128
<211> 20
<212> DNA
<213> Artificial

<220>
<223> oligonucleotide

<400> 1128 20
tctccttgtc gctctccccg 20

<210> 1129
<211> 20
<212> DNA
<213> Artificial

<220>
<223> oligonucleotide

<400> 1129 20
tccttgtcgc tctccccgag 20

<210> 1130
<211> 20
<212> DNA
<213> Artificial

<220>
<223> oligonucleotide

<400> 1130 20
agctttccac tctccttgtc 20

<210> 1131
<211> 20
<212> DNA
<213> Artificial

<220>
<223> oligonucleotide

<400> 1131 20
ccactctcct tgtcgctctc 20

<210> 1132
<211> 20
<212> DNA
<213> Artificial

<220>
<223> oligonucleotide

<400> 1132

ggctcctctc tcagcgatgc 20

<210> 1133
<211> 20
<212> DNA
<213> Artificial

<220>
<223> oligonucleotide

<400> 1133
ccttgtcgct ctccccgagc 20

<210> 1134
<211> 20
<212> DNA
<213> Artificial

<220>
<223> oligonucleotide

<400> 1134
cactctcctt gtcgctctcc 20

<210> 1135
<211> 20
<212> DNA
<213> Artificial

<220>
<223> oligonucleotide

<400> 1135
actctccttg tcgctctccc 20

<210> 1136
<211> 20
<212> DNA
<213> Artificial

<220>
<223> oligonucleotide

<400> 1136
ctctccttgt cgctctcccc 20

<210> 1137
<211> 20
<212> DNA
<213> Artificial

<220>
<223> oligonucleotide

<400> 1137
gcgggctcct ctctcagcga 20

<210> 1138

<211> 20
<212> DNA
<213> Artificial

<220>
<223> oligonucleotide

<400> 1138
ggctccatca tggttacctc 20

<210> 1139
<211> 22
<212> DNA
<213> Artificial

<220>
<223> oligonucleotide

<400> 1139
ccgtctccta aggagctttc ca 22

<210> 1140
<211> 22
<212> DNA
<213> Artificial

<220>
<223> oligonucleotide

<400> 1140
tccctcctta acggttacct ca 22

<210> 1141
<211> 22
<212> DNA
<213> Artificial

<220>
<223> oligonucleotide

<400> 1141
tggctccata awggttacct ca 22

<210> 1142
<211> 20
<212> DNA
<213> Artificial

<220>
<223> oligonucleotide

<400> 1142
cttcctccgg cttgcgccgg 20

<210> 1143
<211> 20
<212> DNA
<213> Artificial

<220>
<223> oligonucleotide

<400> 1143
cgctcttccc gaktgactga 20

<210> 1144
<211> 20
<212> DNA
<213> Artificial

<220>
<223> oligonucleotide

<400> 1144
cctcgggctc ctccatcwgc 20

## Beschreibung

Die Erfindung betrifft ein Verfahren zum spezifischen Schnellnachweis getränkeschädlicher Mikroorganismen durch in situ-Hybridisierung. Weiter betrifft die Erfindung spezifische Oligonukleotidsonden, die im Rahmen des Nachweisverfahrens eingesetzt werden sowie Kits, die diese Oligonukleotidsonden enthalten.

Unter dem Oberbegriff "Alkoholfreie Getränke" (AfG) werden Getränkegruppen wie Fruchtsäfte, Fruchtnektare, Fruchtkonzentrate, Fruchtpürees, Erfrischungsgetränke und Wässer zusammengefasst.

Generell können alkoholfreie Getränke aufgrund ihrer sehr vielseitigen Zusammensetzung aus Nähr- und Wuchsstoffen als potenziell gefährdet durch das Wachstum eines breiten Spektrums von Mikroorganismen eingestuft werden.

Nach heutigem Kenntnisstand werden hauptsächlich Hefen, Schimmelpilze, Milchsäurebakterien, Essigsäurebakterien, Bazillen und Alicyclobazillen im AfG-Bereich vorgefunden und somit als "getränkeschädliche Mikroorganismen" beschrieben.

Die Kontaminationen mit diesen Mikroorganismen führen in der Regel nicht zu gesundheitlichen Schäden des Konsumenten, sie gehen aber meist mit Trübungen, Geschmacks- und Geruchsveränderungen des Endprodukts einher und führen durch einen daraus resultierenden Imageverlust zu hohen wirtschaftlichen Einbußen für die produzierende Industrie.

In Fruchtsäften und Fruchtnektaren können sich aufgrund der meist natürlicherweise hohen Konzentration an Fruchtsäuren und einem damit verbundenen niedrigen pH-Wert (pH-Bereich 2,5 bis 4,5) i.d.R. nur acidophile oder acidotolerante Mikroorganismen (z.B. Milchsäurebakterien, Alicyclobazillen, säuretolerante Hefe- und Schimmelpilzarten) vermehren und somit zu einer Schädigung dieser Getränke führen.

Eine Maßnahme zur Einschränkung des Verderbs durch Mikroorganismen stellt die Carbonisierung von Getränken dar. Dieses Verfahren wird sehr häufig bei der Herstellung von Erfrischungsgetränken eingesetzt. Durch die Zugabe von $CO_2$ wird im Produkt ein nahezu anaerobes Milieu geschaffen und nur mikroaerophile, fakultativ anaerobe und anaerobe Mikroorganismen (z.B. Milchsäurebakterien, Essigsäurebakterien und Hefen) sind in der Lage, dieses Milieu zu tolerieren.

Stille Getränke werden in den meisten Fällen einem Pasteurisierungsprozess unterzogen, um eine lange Stabilität und Qualität dieser Produkte zu gewährleisten. Durch die Pasteurisierung sollen möglichst umfassend alle vegetativen Mikroorganismen abgetötet werden. Allerdings findet dadurch keine Eliminierung der durch Bazillen und Alicyclobazillen gebildeten Sporen statt. Zudem sind auch einige Schimmelpilzarten in der Lage, diesen Prozess ohne Schaden zu überstehen und nachfolgend Produktschäden hervorzurufen.

Ein entscheidender Faktor in der Gewährleistung der biologischen Qualität von Getränken ist die Fahndung nach der Ursache der Kontamination, um diese endgültig zu beseitigen.

Im Allgemeinen werden dabei zwei Kontaminationswege unterschieden: Als Primärkontamination werden Kontaminationen bezeichnet, bei denen Mikroorganismen durch die Rohstoffe oder durch Verunreinigungen im Prozess in das Produkt eingetragen werden.

Sekundärkontaminationen sind Kontaminationen, die nach der eigentlichen Produktion des Getränks im Abfüllbereich auftreten.

Die Herausforderung, die sich durch diese verschiedenen Faktoren an die mikrobiologische Qualitätskontrolle stellt, besteht darin, umfassend und schnell alle im Produkt vorhandenen Keime zu identifizieren, um möglichst rasch entsprechende Gegenmaßnahmen einleiten zu können.

Bislang erfolgt der konventionelle Nachweis von AfG-Schädlingen durch mehrtägige Anreicherung der Untersuchungsprobe in einem Selektivmedium und anschließende Lichtmikroskopie. Zudem müssen zur genauen Bestimmung des AfG-Verderbers weitere physiologische Tests (wie Gram-Färbung, Zuckerverwertungsreihen) durchgeführt werden.

Die Nachteile dieser ausschließlich kultivierungsabhängigen Methode liegen in der langen Analysedauer, welche erhebliche logistische Kosten in den getränkeproduzierenden Betrieben verursacht. Darüber hinaus droht nach der Auslieferung von Produkten, deren mikrobiologischer Befund noch nicht einwandfrei feststand ein beträchtlicher Imageverlust für das betreffende Unternehmen, wenn im Fall von Kontaminationen Rückholaktionen von verdorbenen Produktchargen nötig werden.

Im Folgenden werden die getränkeschädlichen Mikroorganismen und deren Nachweis, wie er im Stand der Technik erfolgt, im Detail beschrieben.

### Hefen und Schimmelpilze:

Zu denjenigen Mikroorganismen, die eine Hitzebehandlung überleben und anschließend Probleme in den Getränken verursachen können, zählen vor allem die Schimmelpilze *Byssochlamys fulva* und *B. nivea, Neosartorya fischeri* und *Talaromyces flavus* sowie einige Hefen. In carbonisierten Getränken sind die säuretoleranten, fermentativen Vertreter der Hefen (*Saccharomyces spp., Dekkera spp.* und *Zygosaccharomyces bailii*) vorherrschend. Neben der Beeinträchtigung der Produkte durch Geschmacksveränderungen und Trübung geht von diesen "gärfähigen Hefen" eine potenzielle Gefahr durch fallweise Explosion ("Bombagen") der Abfüllbehältnisse aus.

Der Nachweis von Hefen und Schimmelpilzen im AfG-Bereich erfolgt derzeit über die Kultivierung auf entsprechenden Nährmedien (z.B. SSL-Bouillon, OFS-Medium, Malzextrakt-Medium, Würze-Agar) und dauert zwischen 2 und 7 Tagen. Ein Nachweis auf Gattungs- oder gar Artebene ist sehr zeitaufwendig und wird in der Regel nicht durchgeführt.

WO 0/77259 und WO 01/53316 beschreiben Verfahren zum Nachweis von einzelnen *Zygosaccharomyces* Arten unter Verwendung von für diese Arten spezifischen Sonden bzw. Primern.

Milchsäurebakterien:

Die Vertreter der Milchsäurebakterien sind gram-positive, nicht sporenbildende, Katalase-negative Stäbchen oder Kokken, die sich durch einen sehr hohen Nährstoffanspruch (vor allem an Vitaminen, Aminosäuren, Purinen und Pyrimidinen) auszeichnen. Wie der Name schon andeutet, sind alle Milchsäurebakterien in der Lage, als Gärprodukt Milchsäure herzustellen.

Aufgrund ihres anaeroben Wachstums und der für anaerobe Mikroorganismen atypische hohe Toleranz und Unempfindlichkeit gegenüber Sauerstoff werden sie als aerotolerante Anaerobier bezeichnet.

Bis dato werden u.a. die Gattungen Lactobacillus, Lactococcus, Leuconostoc, Oenococcus, Carnobacterium, Bifidobacterium, Enterococcus, Pediococcus, Weissella und Streptococcus unter dem Begriff "Milchsäurebakterien" geführt.

Milchsäurebakterien haben in der Lebensmittelindustrie eine ambivalente Rolle. Einerseits ist ihr Vorhandensein in manchen Prozessen, wie z.B. der Herstellung von Sauerkraut, erwünscht und somit nicht wegzudenken. Andererseits kann ihr Vorkommen in Bier oder Fruchtsäften zu einem Verderb dieser Produkte führen. Das Wachstum dieser Bakterien äußert sich vornehmlich durch Trübung, Säuerung, Gas- und Schleimbildung.

In der AfG-Industrie sind hauptsächlich die Bakteriengattungen Leuconostoc, Lactococcus, Lactobacillus, Oenococcus, Weissella und Pediococcus als Kontaminanten von Bedeutung.
Milchsäurebakterien werden durch 5- bis 7-tägige Inkubation bei 25 °C auf MRS-Agar (pH 5,7) nachgewiesen.

Essigsäurebakterien:

Mit dem Trivialnamen "Essigsäurebakterien" werden Bakterien der Gattungen Acetobacter, Gluconobacter, Gluconoacetobacter und Acidomonas bezeichnet. Bakterien dieser Gattungen sind gram-negative, obligat aerobe, Oxidase-negative Stäbchen, deren optimale Vermehrungstemperatur um 30 °C liegt. Essigsäurebakterien sind in der Lage, sich auch bei pH-Werten um 2,2 bis 3,0 zu vermehren und können daher in Getränken mit diesem pH-Wert Produktschäden hervorrufen.

Phylogenetisch werden Bakterien dieser Gattung als Mitglieder der Alphaproteobakterien eingestuft.

Die Produktschädigungen gehen zumeist mit Trübungen und Geschmacksveränderungen durch die Bildung von Essigsäure und Gluconsäure einher.

Für den Nachweis von Essigsäurebakterien haben sich vor allem ACM-Agar (Inkubationszeit: 14 Tage) und DSM-Agar (Inkubationszeit: 3 bis 5 Tage) bewährt.

Bazillen:

Bazillen sind gram-positive aerobe, z.T. fakultativ anaerobe, zumeist Katalase-positive sporenbildende Stäbchen. In der AfG-Industrie wurde bis dato hauptsächlich *Bacillus coagulans* als Verderbniserreger identifiziert.

Der Nachweis erfolgt durch Ausstrich des Untersuchungsmaterials auf Dextrose-Caseinpepton-Agar oder Hefeextrakt-Pepton-Dextrose-Stärke-Agar und anschließender Inkubation bei 55 °C (Inkubationszeit: 3 Tage). Um eine Aktivierung bzw. eine Auskeimung der *B. coagulans*-Sporen zu erreichen, wird vor der eigentlichen Inkubation eine Erwärmung der Probe bei 80 °C für 10 min empfohlen.

Alicyclobazillen:

Alicyclobazillen sind gram-positive, aerobe, thermophile und Katalase-positive sporenbildende Stäbchen. Vertreter dieser Gattung bilden ω-alicyclische Fettsäuren als zelluläre Hauptfettsäuren.
In der AfG-Industrie wurde bis dato weltweit hauptsächlich *Alicyclobacillus acidoterrestris* als Verderbniserreger nachgewiesen. In seltenen Fällen wurden auch *A. acidocaldarius* und *A. acidiphilus* in verdorbenen Getränken identifiziert.

Der optimale Wachstumstemperaturbereich für *Alicyclobacillus spp.* liegt zwischen 26 und 55 °C. Der pH-Bereich, in dem sich Bakterien dieser Gattung vermehren können, liegt zwischen 2,2 und 5,8.

Das Wachstum von *A. acidoterrestris* führt in Fruchtsäften zu Verderb, der sich infolge der Bildung von Guajakol und Di-Bromphenol in Geruchs- und Geschmacksveränderungen äußert. Eine Kontamination mit diesem Organismus verläuft zumeist inapparent, was bedeutet, dass nur in seltenen Fällen eine Trübung in den infizierten Getränken auftritt. Alicyclobazillen können über mehrtägige Kultivierung bei 44 bis 46 °C auf Orangenserum-Agar, Kartoffel-Dextrose-Agar, K-Agar, YSG-Agar oder BAM-Agar nachgewiesen werden. Zudem ist zur sicheren Bestätigung des Befundes eine Reihe physiologischer Tests notwendig. Um eine Aktivierung bzw. eine Auskeimung der *Alicyclobacillus ssp.*-Sporen zu erreichen, wird vor der eigentlichen Inkubation eine Erwärmung der Probe bei 80 °C für 10 min empfohlen.

Die bisher in der Routineanalytik eingesetzten Nachweisverfahren für getränkeschädliche Mikroorganismen sind sehr langwierig und teilweise zu ungenau und verhindern somit schnelle und wirkungsvolle Gegenmaßnahmen zum Erhalt des kontaminierten Produktes. Die Ungenauigkeit resultiert beim Nachweis aus einer fehlenden Differenzierung bis auf Gattungs- und/oder Artebene.

Als logische Konsequenz aus den Schwierigkeiten, welche bei traditionellen Kultivierungsverfahren beim Nachweis von getränkeschädlichen Mikroorganismen auftreten, bieten sich daher Nachweisverfahren auf Nukleinsäurebasis zur schnellen, sicheren und spezifischen Identifizierung von Verderbniserregern in alkoholfreien Gertränken an.

Bei der PCR, der Polymerase-Kettenreaktion, wird mit spezifischen Primern ein charakteristisches Stück des jeweiligen Mikroorganismengenoms amplifiziert. Findet der Primer seine Zielstelle, so kommt es zu einer millionenfachen Vermehrung eines Stücks der Erbsubstanz. Bei der anschließenden Analyse, z.B. mittels eines DNA-Fragmente auftrennenden Agarose-Gels, kann eine qualitative Bewertung stattfinden. Im einfachsten Fall führt dies zu der Aussage, dass die Zielstellen für die verwendeten Primer in der untersuchten Probe vorhanden waren. Weitere Aussagen sind nicht möglich; diese Zielstellen können sowohl von einem lebenden Bakterium, als auch von einem toten Bakterium oder von nackter DNA stammen. Da die PCR-Reaktion auch bei Anwesenheit eines toten Bakteriums oder nackter DNA positiv ausfällt, kommt es hier häufig zu falsch positiven Ergebnissen. Eine Weiterführung dieser Technik stellt die quantitative PCR dar, bei der versucht wird, eine Korrelation zwischen der Menge an vorhandenen Mikroorganismen und der Menge an amplifizierter DNA herzustellen. Vorteile der PCR liegen in ihrer hohen Spezifität, leichten Anwendbarkeit und im geringen Zeitaufwand. Wesentliche Nachteile sind ihre hohe Anfälligkeit für Kontaminationen und damit falsch positive Ergebnisse sowie die bereits erwähnte fehlende Möglichkeit, zwischen lebenden und toten Zellen bzw. nackter DNA zu unterscheiden.

Einen einzigartigen Ansatz, die Spezifität der molekularbiologischen Methoden wie der PCR mit der Möglichkeit der Mikroorganismenvisualisierung, wie sie die Antikörper-Methoden ermöglichen, zu verbinden, bietet die Methode der Fluoreszenz-In-Situ-Hybridisierung (FISH; Amann, R. I., W. Ludwig und K.-H. Schleifer, 1995. Phylogenetic identification and in situ detection of individual microbial cells without cultivation. Microbial. Rev. 59, S. 143-169). Hierbei können Mikroorganismenarten, -gattungen oder -gruppen hochspezifisch identifiziert und visualisiert werden.

Die FISH-Technik basiert auf der Tatsache, dass es in Mikroorganismenzellen bestimmte Moleküle gibt, die aufgrund ihrer lebenswichtigen Funktion im Laufe der Evolution nur wenig mutiert sind: Die 16S, 18S, 23S und 26S ribosomale Ribonukleinsäure (rRNA). Sie sind Bestandteile der Ribosomen, den Orten der Proteinbiosynthese, und können aufgrund ihrer ubiquitären Verbreitung, ihrer Größe, und ihrer strukturellen und funktionellen Konstanz als spezifische Marker dienen (Woese, C. R., 1987. Bacterial evolution. Microbiol. Rev. 51, S. 221-271). Ausgehend von einer vergleichenden Sequenzanalyse können phylogenetische Beziehungen allein aufgrund dieser Daten aufgestellt werden. Dazu müssen diese Sequenzdaten in ein Alignment gebracht werden. Im Alignment, welches sich auf Kenntnisse über die Sekundärstruktur und Tertiärstruktur dieser Makromoleküle stützt, werden die homologen Positionen der ribosomalen Nukleinsäuren in Einklang miteinander gebracht.

Ausgehend von diesen Daten können phylogenetische Berechnungen durchgeführt werden. Der Einsatz modernster Computertechnologie macht es möglich, auch großangelegte Berechnungen schnell und effektiv auszuführen, sowie große Datenbanken, welche die Alignment-Sequenzen der 16S, 18S, 23S und 26S rRNA beinhalten, anzulegen. Durch den schnellen Zugriff auf dieses Datenmaterial können neu erhaltene Sequenzen in kurzer Zeit phylogenetisch analysiert werden. Diese rRNA Datenbanken können dazu verwendet werden, art- und gattungsspezifische Gensonden zu konstruieren. Hierbei werden alle verfügbaren rRNA Sequenzen miteinander verglichen und für bestimmte Sequenzstellen Sonden entworfen, die spezifisch eine Mikroorganismenart, -gattung oder -gruppe erfassen.

Bei der FISH (Fluoreszenz-In-Situ-Hybridisierung)-Technik werden diese Gensonden, die zu einer bestimmten Region auf der ribosomalen Zielsequenz komplementär sind, in die Zelle eingeschleust. Die Gensonden sind i.d.R. kleine, 16 bis 20 Basen lange, einzelsträngige Desoxyribonukleinsäurestücke und richten sich gegen eine Zielregion, welche typisch für eine Mikroorganismenart oder eine Mikroorganismengruppe ist. Findet die fluoreszenzmarkierte Gensonde in einer Mikroorganismenzelle ihre Zielsequenz, so bindet sie daran und die Zellen können aufgrund ihrer Fluoreszenz mit Hilfe eines Fluoreszenzmikroskops detektiert werden.

Die FISH-Analyse wird grundsätzlich auf einem Objektträger durchgeführt, da die Mikroorganismen bei der Auswertung durch Bestrahlung mit einem hochenergetischen Licht visualisiert, also sichtbar gemacht werden. Hierin liegt allerdings einer der Nachteile der klassischen FISH-Analyse: da auf einem Objektträger naturgemäß nur relativ kleine Volumina analysiert werden können, ist die Sensitivität der Methode unbefriedigend und für eine verlässliche Analyse nicht ausreichend.

Mit der vorliegenden Erfindung werden daher die Vorteile der klassischen FISH-Analyse mit denen der Kultivierung verknüpft. Durch einen vergleichsweise kurzen Kultivierungsschritt wird sichergestellt, dass die nachzuweisenden Mikroorganismen in ausreichender Zahl vorliegen, bevor der Nachweis der Mikroorganismen mittels spezifischer FISH durchgeführt wird.

Die Durchführung der in der vorliegenden Anmeldung beschriebenen Verfahren zum spezifischen Nachweis von getränkeschädlichen Hefen der Gattungen Zygosaccharomyces, Hanseniaspora, Candida, Brettanomyces, Dekkera, Pichia, Saccharomyces und Saccharomycodes, insbesondere der Spezies *Zygosaccharomyces bailii, Z. mellis, Z rouxii, Z. bisporus, Z. fermentati, Z microellipsoides, Hanseniaspora uvarum, Candida intermedia, C. crusei (Issatchenkia orientalis), C. parapsilosis, Brettanomyces bruxellensis, B. naardenensis, Dekkera anomala, Pichia membranaefaciens, P. minuta, P. anomala, Saccharomyces exiguus, S. cerevisiae, Saccharomycodes ludwigii* oder zum spezifischen Nachweis von getränkeschädlichen Schimmelpilzen der Gattungen Mucor, Byssochlamys, Neosartorya, Aspergillus und Talaromyces, insbesondere der Spezies *Mucor racemosus, Byssochlamys nivea, Neosartorya fischeri, Aspergillus fumigatus und A. fischeri, Talaromyces flavus, T. bacillisporus* und *T. flavus* oder zum spezifischen Nachweis von getränkeschädlichen Bakterien der Gattungen Lactobacillus, Leuconostoc, Oenococcus, Weissella, Lactococcus, Acetobacter, Gluconobacter, Gluconoacetobacter, Bacillus und Alicyclobacillus, insbesondere der Spezies *Lactobacillus collinoides, Leuconostoc mesenteroides, L. pseudomesenteroides, Oenococcus oeni, Bacillus coagulans, Alicyclobacillus ssp., A. acidoterrestris, A. cycloheptanicus* und *A. herbarius* umfasst somit die folgenden Schritte:

- Kultivieren der in der untersuchten Probe enthaltenen getränkeschädlichen Mikroorganismen
- Fixieren der in der Probe enthaltenen getränkeschädlichen Mikroorganismen
- Inkubieren der fixierten Mikroorganismen mit mindestens einer Oligonukleotidsonde, ggf. zusammen mit einer Kompetitorsonde, um eine Hybridisierung herbeizuführen,
- Entfernen bzw. Abwaschen der nicht hybridisierten Oligonukleotidsonden und
- Detektieren der mit den Oligonukleotidsonden hybridisierten getränkeschädlichen Mikroorganismen.

Im Rahmen der vorliegenden Erfindung wird unter "Kultivieren" die Vermehrung der in der Probe enthaltenen Mikroorganismen in einem geeigneten Kultivierungsmedium verstanden.

Zum Nachweis von Hefen und Schimmelpilzen kann die Kultivierung z.B. in SSL-Bouillon für 24 h bei 25 °C erfolgen. Zum Nachweis von Milchsäurebakterien kann die Kultivierung z.B. in MRS-Bouillon für 48 h bei 30 °C erfolgen. Zum Nachweis von Essigsäurebakterien kann die Kultivierung z.B. auf DSM-Agar für 48 h bei 28 °C erfolgen. Zum Nachweis von Bazillen, vornehmlich *B. coagulans,* kann die Kultivierung z.B. auf Dextrose-Caseinpepton-Agar für 48 h bei 55 °C erfolgen.

Zum Nachweis von Alicyclobazillen kann die Kultivierung z.B. in BAM-Bouillon für 48 h bei 44 °C erfolgen.

Der Fachmann kann die geeigneten Kultivierungsverfahren für jeden zu untersuchenden Mikroorganismus bzw. jede Mikroorganismengruppe dem Stand der Technik entnehmen.

Im Rahmen der vorliegenden Erfindung wird unter "Fixieren" der Mikroorganismen eine Behandlung verstanden, mit der die Hülle der Mikroorganismen für Nukleinsäuresonden durchlässig gemacht wird. Zur Fixierung wird üblicherweise Ethanol verwendet. Kann die Zellwand trotz dieser Behandlung nicht von den Nukleinsäuresonden penetriert werden, so sind dem Fachmann ausreichend weitere Maßnahmen bekannt, die zu demselben Ergebnis führen. Dazu zählen beispielsweise der Einsatz von Methanol, Mischungen von Alkoholen, einer niederprozentigen Paraformaldehydlösung oder einer verdünnten Formaldehydlösung, enzymatische Behandlungen oder ähnliches. Es kann sich in einer besonders bevorzugten Ausführungsform des erfindungsgemäßen Verfahrens ein enzymatischer Schritt zum vollständigen Aufschluss der Mikroorganismen anschließen. Als Enzyme sind hier bspw. Lysozym, Proteinase K und Mutanolysin zu nennen. Dem Fachmann sind hier genügend geeignete Verfahren bekannt, und er wird auf einfache Weise feststellen können, welches Mittel für den Zellaufschluss eines bestimmten Mikroorganismus besonders geeignet ist.

Im Rahmen der vorliegenden Erfindung werden für die "Hybridisierung" die fixierten Mikroorganismen mit fluoreszenzmarkierten Oligonukleotidsonden inkubiert. Diese Oligonukleotidsonden können nach dem Fixieren die Zellhülle penetrieren und an die der Oligonukleotidsonde entsprechende Zielsequenz im Zellinneren binden. Die Bindung ist als Ausbildung von Wasserstoffbrücken zwischen komplementären Nukleinsäurestücken zu verstehen.

Die Oligonukleotidsonde kann dabei komplementär zu einer chromosomalen oder episomalen DNA sein, aber auch zu einer mRNA oder rRNA des nachzuweisenden Mikroorganismus. Von Vorteil ist es, eine Oligonukleotidsonde zu wählen, die zu einem Bereich komplementär ist, der in einer Kopienzahl von mehr als 1 im nachzuweisenden Mikroorganismus vorhanden ist. Die nachzuweisende Sequenz liegt bevorzugt 500 bis 100.000 mal pro Zelle vor, besonders bevorzugt 1.000 bis 50.000 mal. Aus diesem Grunde wird bevorzugt eine Sequenz aus der rRNA als Zielsequenz verwendet, da die Ribosomen in der Zelle als Orte der Proteinbiosynthese viele tausendmal in jeder aktiven Zelle vorliegen.

Bei der Nukleinsäuresonde im Sinne der Erfindung kann es sich um eine DNA- oder RNA-Sonde handeln, die in der Regel zwischen 12 und 100 Nukleotide umfassen wird, bevorzugt zwischen 15 und 50, besonders bevorzugt zwischen 17 und 25 Nukleotide. Die Auswahl der Nukleinsäuresonden geschieht unter dem Gesichtspunkt, ob eine komplementäre Sequenz in dem nachzuweisenden Mikroorganismus vorliegt. Durch diese Auswahl einer definierten Sequenz kann eine Mikroorganismenart, eine Mikroorganismengattung oder eine ganze Mikroorganismengruppe erfasst werden. Komplementarität sollte bei einer Sonde von 15 Nukleotiden über 100 % der Sequenz gegeben sein. Bei Oligonukleotiden mit mehr als 15 Nukleotiden sind je nach Länge ein bis mehrere Fehlpaarungsstellen erlaubt.

Zur Erhöhung der Spezifität von Nukleinsäuresonden können Kompetitorsonden eingesetzt werden. Unter dem Begriff "Kompetitorsonden" werden im Rahmen der vorliegenden Erfindung insbesondere Oligonukleotide verstanden, die eventuell auftretende ungewollte Bindungen der Nukleinsäuresonden abdecken und dabei eine höhere Sequenzähnlichkeit zu nicht nachzuweisenden Mikroorganismengattungen bzw. -spezies aufweisen als zu den nachzuweisenden Mikroorganismengattungen bzw. -spezies. Durch den Einsatz von Kompetitorsonden kann verhindert werden, dass die Nukleinsäuresonde an die Nukleinsäuresequenz der nicht nachzuweisenden Mikroorganismengattungen bzw. -spezies bindet und zu falschen Signalen führt. Die unmarkierte Kompetitorsonde wird immer zusammen mit der entsprechenden markierten Oligonukleotidsonde eingesetzt.

Die Kompetitorsonde sollte komplementär sein zu einer Nukleinsäuresequenz mit hoher Sequenzähnlichkeit zur Nukleinsäuresequenz der nachzuweisenden Mikroorganismengattungen bzw. -spezies. Besonders bevorzugt ist die Kompetitorsonde komplementär zur rRNA von nicht nachzuweisenden Mikroorganismengattungen bzw. -spezies.

Bei der Kompetitorsonde kann es sich im Sinne der Erfindung um eine DNA- oder RNA-Sequenz handeln, die in der Regel zwischen 12 und 100 Nukleotide umfassen wird, bevorzugt zwischen 15 und 50, besonders bevorzugt zwischen 17 und 25 Nukleotide. Durch die Auswahl einer definierten Sequenz kann die Hybridisierung der markierten Oligonukleotidsonde an die Nukleinsäuresequenz einer Bakterienart, einer Bakteriengattung oder einer ganzen Bakteriengruppe abgeblockt werden. Komplementarität zu der abzublockenden Nukleinsäuresequenz sollte bei einer Sonde von 15 Nukleotiden über 100 % der Sequenz gegeben sein. Bei Oligonukleotiden mit mehr als 15 Nukleotiden sind je nach Länge ein bis mehrere Fehlpaarungsstellen erlaubt.

Im Rahmen der erfindungsgemäßen Verfahren haben die erfindungsgemäßen Nukleinsäuresondenmoleküle die nachstehend angegebenen Längen und Sequenzen (alle Nukleinsäuresondenmoleküle sind in 5'-3'-Richtung notiert).

Die erfindungsgemäßen Nukleinsäuresondenmoleküle sind zum spezifischen Nachweis von getränkeschädlichen Hefen der Gattung Zygosaccharomyces geeignet und werden entsprechend in dem erfindungsgemässen Nachweisverfahren eingesetzt. Des weiteren beschrieben sind Nukleinsäuremoleküle zum spezifischen Nachweis der Gattungen Hanseniaspora, Candida, Brettanomyces, Dekkera, Pichia, Saccharomyces und Saccharomycodes, insbesondere der Spezies *Zygosaccharomyces bailii, Z. mellis, Z. rouxii, Z. bisporus, Z. fermentati, Z. microellipsoides, Hanseniaspora uvarum, Candida intermedia, C. crusei (Issatchenkia orientalis), C. parapsilosis, Brettanomyces bruxellensis, B. naardenensis, Dekkera anomala, Pichia membranaefaciens, P. minuta, P. anomala, Saccharomyces exiguus, S. cerevisiae, Saccharomycodes ludwigii* oder zum spezifischen Nachweis von getränkeschädlichen Schimmelpilzen der Gattungen Mucor, Byssochlamys, Neosartorya, Aspergillus und Talaromyces, insbesondere der Spezies *Mucor racemosus, Byssochlamys nivea, Neosartorya fischeri, Aspergillus fumigatus und A. fischeri, Talaromyces flavus, T. bacillisporus* und *T. flavus* oder zum spezifischen Nachweis von getränkeschädlichen Bakterien der Gattungen Lactobacillus, Leuconostoc, Oenococcus, Weissella, Lactococcus, Acetobacter, Gluconobacter, Gluconoacetobacter, Bacillus und Alicyclobacillus, insbesondere der Spezies *Lactobacillus collinoides, Leuconostoc mesenteroides, L. pseudomesenteroides, Oenococcus oeni, Bacillus coagulans, Alicyclobacillus ssp., A. acidoterrestris, A. cycloheptanicus* und *A. herbarius.*

Im Rahmen der vorliegenden Erfindung können Sonden, die unterschiedliche Arten von Mikroorganismen nachweisen, zusammen eingesetzt werden, um dadurch den gleichzeitigen Nachweis von unterschiedlichen Arten von Mikroorganismen zu ermöglichen. Dies führt ebenfalls zu einer Beschleunigung des Nachweisverfahrens.

a) Nukleinsäuresondenmoleküle, die spezifisch getränkeschädliche Hefen nachweisen:

SEQ ID No. 1: 5'- GTTTGACCAGATTCTCCGCTC

Die Sequenz SEQ ID No. 1 ist vor allem zum Nachweis von Mikroorganismen der Gattung Zygosaccharomyces geeignet.

SEQ ID No. 2: 5'- GTTTGACCAGATTTTCCGCTCT
SEQ ID No. 3: 5'- GTTTGACCAAATTTTCCGCTCT
SEQ ID No. 4: 5'- GTTTGTCCAAATTCTCCGCTCT

Die Nukleinsäuresondenmoleküle gemäß SEQ ID No. 2 bis SEQ ID No. 4 werden als unmarkierte Kompetitorsonden für den Nachweis von Mikroorganismen der Gattung *Zygosaccharomyces* gemeinsam mit der Oligonukleotidsonde gemäß SEQ ID No. 1 eingesetzt, um das Binden der markierten, für Mikroorganismen der Gattung *Zygosaccharomyces* spezifischen Oligonukleotidsonde an Nukleinsäuresequenzen, die nicht spezifisch für Mikroorganismen der Gattung *Zygosaccharomyces* sind, zu verhindern.

SEQ ID No. 5: 5'- CCCGGTCGAATTAAAACC
SEQ ID No. 6: 5'- GCCCGGTCGAATTAAAAC
SEQ ID No. 7: 5'- GGCCCGGTCGAATTAAAA
SEQ ID No. 8: 5'- AGGCCCGGTCGAATTAAA
SEQ ID No. 9: 5'- AAGGCCCGGTCGAATTAA
SEQ ID No. 10: 5'- ATATTCGAGCGAAACGCC
SEQ ID No. 11: 5'- AAAGATCCGGACCGGCCG
SEQ ID No. 12 5'- GGAAAGATCCGGACCGGC
SEQ ID No. 13 5'- GAAAGATCCGGACCGGCC
SEQ ID No. 14 5'- GATCCGGACCGGCCGACC
SEQ ID No. 15 5'- AGATCCGGACCGGCCGAC
SEQ ID No. 16 5'- AAGATCCGGACCGGCCGA
SEQ ID No. 17 5'- GAAAGGCCCGGTCGAATT
SEQ ID No. 18 5'- AAAGGCCCGGTGGAATTA
SEQ ID No. 19 5'- GGAAAGGCCCGGTCGAAT
SEQ ID No. 20 5'- AGGAAAGGCCCGGTCGAA
SEQ ID No. 21 5'- AAGGAAAGGCCCGGTCGA

Die Sequenzen SEQ ID No. 5 bis SEQ ID No. 21 sind vor allem zum Nachweis von *Zygosaccharomyces bailii* geeignet.

SEQ ID No. 22: 5'- ATAGCACTGGGATCCTCGCC

Die Sequenz SEQ ID No. 22 ist vor allem zum Nachweis von *Zygosaccharomyces fermentati* geeignet.

SEQ ID No. 23: 5'- CCAGCCCCAAAGTTACCTTC
SEQ ID No. 24: 5'- TCCTTGACGTAAAGTCGCAG

Die Sequenzen SEQ ID No. 23 und SEQ ID No. 24 sind vor allem zum Nachweis von *Zygosaccharomyces microellipsoides* geeignet.

SEQ ID No. 25: 5'- GGAAGAAAACCAGTACGC
SEQ ID No. 26: 5'- CCGGTCGGAAGAAAACCA
SEQ ID No. 27: 5'- GAAGAAAACCAGTACGCG
SEQ ID No. 28: 5'- CCCGGTCGGAAGAAAACC
SEQ ID No. 29: 5'- CGGTCGGAAGAAAACCAG
SEQ ID No. 30: 5'- GGTCGGAAGAAAACCAGT
SEQ ID No. 31: 5'- AAGAAAACCAGTACGCGG
SEQ ID No. 32: 5'- GTACGCGGAAAAATCCGG
SEQ ID No. 33: 5'- AGTACGCGGAAAAATCCG
SEQ ID No. 34: 5'- GCGGAAAAATCCGGACCG
SEQ ID No. 35: 5'- CGGAAGAAAACCAGTACG
SEQ ID No. 36: 5'- GCCCGGTCGGAAGAAAAC
SEQ ID No. 37: 5'- CGCGGAAAAATCCGGACC
SEQ ID No. 38: 5'- CAGTACGCGGAAAAATCC
SEQ ID No. 39: 5'- AGAAAACCAGTACGCGGA
SEQ ID No. 40: 5'- GGCCCGGTCGGAAGAAAA
SEQ ID No. 41: 5'- ATAAACACCACCCGATCC
SEQ ID No. 42: 5'- ACGCGGAAAAATCCGGAC

(fortgesetzt)

| | |
|---|---|
| SEQ ID No. 43: | 5'- GAGAGGCCCGGTCGGAAG |
| SEQ ID No. 44: | 5'- AGAGGCCCGGTCGGAAGA |
| SEQ ID No. 45: | 5'- GAGGCCCGGTCGGAAGAA |
| SEQ ID No. 46: | 5'- AGGCCCGGTCGGAAGAAA |
| SEQ ID No. 47: | 5'- CCGAGTGGGTCAGTAAAT |
| SEQ ID No. 48: | 5'- CCAGTACGCGGAAAAATC |
| SEQ ID No. 49: | 5'- TAAACACCACCCGATCCC |
| SEQ ID No. 50: | 5'- GGAGAGGCCCGGTCGGAA |
| SEQ ID No. 51: | 5'- GAAAACCAGTACGCGGAA |
| SEQ ID No. 52: | 5'- TACGCGGAAAAATCCGGA |
| SEQ ID No. 53: | 5'- GGCCACAGGGACCCAGGG |
| SEQ ID No. 54: | 5'- TCACCAAGGGCCACAGGG |
| SEQ ID No. 55: | 5'- GGGCCACAGGGACCCAGG |
| SEQ ID No. 56: | 5'- TTCACCAAGGGCCACAGG |
| SEQ ID No. 57: | 5'- ACAGGGACCCAGGGCTAG |
| SEQ ID No. 58: | 5'- AGGGCCACAGGGACCCAG |
| SEQ ID No. 59: | 5'- GTTCACCAAGGGCCACAG |
| SEQ ID No. 60: | 5'- GCCACAGGGACCCAGGGC |
| SEQ ID No. 61: | 5'- CAGGGACCCAGGGCTAGC |
| SEQ ID No. 62: | 5'- AGGGACCCAGGGCTAGCC |
| SEQ ID No. 63: | 5'- ACCAAGGGCCACAGGGAC |
| SEQ ID No. 64: | 5'- CCACAGGGACCCAGGGCT |
| SEQ ID No. 65: | 5'- CACAGGGACCCAGGGCTA |
| SEQ ID No. 66: | 5'- CACCAAGGGCCACAGGGA |
| SEQ ID No. 67: | 5'- GGGACCCAGGGCTAGCCA |
| SEQ ID No. 68: | 5'- AGGAGAGGCCCGGTCGGA |
| SEQ ID No. 69: | 5'- AAGGAGAGGCCCGGTCGG |
| SEQ ID No. 70: | 5'- GAAGGAGAGGCCCGGTCG |
| SEQ ID No. 71: | 5'- AGGGCTAGCCAGAAGGAG |
| SEQ ID No. 72: | 5'- GGGCTAGCCAGAAGGAGA |
| SEQ ID No. 73: | 5'- AGAAGGAGAGGCCCGGTC |
| SEQ ID No. 74: | 5'- CAAGGGCCACAGGGACCC |
| SEQ ID No. 75: | 5'- CCAAGGGCCACAGGGACC |

Die Sequenzen SEQ ID No. 25 bis SEQ ID No. 75 sind vor allem zum Nachweis von *Zygosaccharomyces mellis* geeignet.

| | |
|---|---|
| SEQ ID No. 76: | 5'- GTCGGAAAAACCAGTACG |
| SEQ ID No. 77: | 5'- GCCCGGTCGGAAAAACCA |
| SEQ ID No. 78: | 5'- CCGGTCGGAAAAACCAGT |
| SEQ ID No. 79: | 5'- CCCGGTCGGAAAAACCAG |
| SEQ ID No. 80: | 5'- TCGGAAAAACCAGTACGC |
| SEQ ID No. 81: | 5'- CGGAAAAACCAGTACGCG |
| SEQ ID No. 82: | 5'- GGAAAAACCAGTACGCGG |
| SEQ ID No. 83: | 5'- GTACGCGGAAAAATCCGG |
| SEQ ID No. 84: | 5'- AGTACGCGGAAAAATCCG |
| SEQ ID No. 85: | 5'- GCGGAAAAATCCGGACCG |
| SEQ ID No. 86: | 5'- GGTCGGAAAAACCAGTAC |
| SEQ ID No. 87: | 5'- ACTCCTAGTGGTGCCCTT |
| SEQ ID No. 88: | 5'- GCTCCACTCCTAGTGGTG |
| SEQ ID No. 89: | 5'- CACTCCTAGTGGTGCCCT |
| SEQ ID No. 90: | 5'- CTCCACTCCTAGTGGTGC |

(fortgesetzt)

SEQ ID No. 91: 5'- TCCACTCCTAGTGGTGCC
SEQ ID No. 92: 5'- CCACTCCTAGTGGTGCCC
SEQ ID No. 93: 5'- GGCTCCACTCCTAGTGGT
SEQ ID No. 94: 5'- AGGCTCCACTCCTAGTGG
SEQ ID No. 95: 5'- GGCCCGGTCGGAAAAACC
SEQ ID No. 96: 5'- GAAAAACCAGTACGCGGA
SEQ ID No. 97: 5'- CGCGGAAAAATCCGGACC
SEQ ID No. 98: 5'- CAGTACGCGGAAAAATCC
SEQ ID No. 99: 5'- CGGTCGGAAAAACCAGTA
SEQ ID No. 100: 5'- AAGGCCCGGTCGGAAAAA
SEQ ID No. 101: 5'- CAGGCTCCACTCCTAGTG
SEQ ID No. 102: 5'- CTCCTAGTGGTGCCCTTC
SEQ ID No. 103: 5'- TCCTAGTGGTGCCCTTCC
SEQ ID No. 104: 5'- GCAGGCTCCACTCCTAGT
SEQ ID No. 105: 5'- AGGCCCGGTCGGAAAAAC
SEQ ID No. 106: 5'- ACGCGGAAAAATCCGGAC
SEQ ID No. 107: 5'- CCAGTACGCGGAAAAATC
SEQ ID No. 108: 5'- CTAGTGGTGCCCTTCCGT
SEQ ID No. 109: 5'- GAAAGGCCCGGTCGGAAA
SEQ ID No. 110: 5'- AAAGGCCCGGTCGGAAAA
SEQ ID No. 111: 5'- TACGCGGAAAAATCCGGA
SEQ ID No. 112: 5'- GGAAAGGCCCGGTCGGAA
SEQ ID No. 113: 5'- ATCTCTTCCGAAAGGTCG
SEQ ID No. 114: 5'- CATCTCTTCCGAAAGGTC
SEQ ID No. 115: 5'- CTCTTCCGAAAGGTCGAG
SEQ ID No. 116: 5'- CTTCCGAAAGGTCGAGAT
SEQ ID No. 117: 5'- TCTCTTCCGAAAGGTCGA
SEQ ID No. 118: 5'- TCTTCCGAAAGGTCGAGA
SEQ ID No. 119: 5'- CCTAGTGGTGCCCTTCCG
SEQ ID No. 120: 5'- TAGTGGTGCCCTTCCGTC
SEQ ID No. 121: 5'- AGTGGTGCCCTTCCGTCA
SEQ ID No. 122: 5'- GCCAAGGTTAGACTCGTT
SEQ ID No. 123: 5'- GGCCAAGGTTAGACTCGT
SEQ ID No. 124: 5'- CCAAGGTTAGACTCGTTG
SEQ ID No. 125: 5'- CAAGGTTAGACTCGTTGG
SEQ ID No. 126: 5'- AAGGTTAGACTCGTTGGC

Die Sequenzen SEQ ID No. 76 bis SEQ ID No. 126 sind vor allem zum Nachweis von *Zygosaccharomyces rouxii* geeignet.

SEQ ID No. 127: 5'- CTCGCCTCACGGGGTTCTCA

Die Sequenz SEQ ID No. 127 ist vor allem zum gleichzeitigen Nachweis von *Zygosaccharomyces mellis* und *Zygosaccharomyces rouxii* geeignet.

SEQ ID No. 128: 5'- GGCCCGGTCGAAATTAAA
SEQ ID No. 129: 5'- AGGCCCGGTCGAAATTAA
SEQ ID No. 130: 5'- AAGGCCCGGTCGAAATTA
SEQ ID No. 131: 5'- AAAGGCCCGGTCGAAATT
SEQ ID No. 132: 5'- GAAAGGCCCGGTCGAAAT
SEQ ID No. 133: 5'- ATATTCGAGCGAAACGCC
SEQ ID No. 134: 5'- GGAAAGGCCCGGTCGAAA

(fortgesetzt)

SEQ ID No. 135: 5'- AAAGATCCGGACCGGCCG
SEQ ID No. 136: 5'- GGAAAGATCCGGACCGGC
SEQ ID No. 137: 5'- GAAAGATCCGGACCGGCC
SEQ ID No. 138: 5'- GATCCGGACCGGCCGACC
SEQ ID No. 139: 5'- AGATCCGGACCGGCCGAC
SEQ ID No. 140: 5'- AAGATCCGGACCGGCCGA
SEQ ID No. 141: 5'- AGGAAAGGCCCGGTCGAA
SEQ ID No. 142: 5'- AAGGAAAGGCCCGGTCGA

Die Sequenzen SEQ ID No. 128 bis SEQ ID No. 142 sind vor allem zum Nachweis von *Zygosaccharomyces bisporus* geeignet.

SEQ ID No. 143: 5'-CGAGCAAAACGCCTGCTTTG
SEQ ID No. 144: 5'-CGCTCTGAAAGAGAGTTGCC

Die Sequenzen SEQ ID No. 143 und SEQ ID No. 144 sind vor allem zum Nachweis von *Hanseniaspora uvarum* geeignet.

SEQ ID No. 145: 5'-AGTTGCCCCCTACACTAGAC
SEQ ID No. 146: 5'-GCTTCTCCGTCCCGCGCCG

Die Sequenzen SEQ ID No. 145 und SEQ ID No. 146 sind vor allem zum Nachweis von *Candida intermedia* geeignet.

SEQ ID No. 147: 5'- AGATTYTCCGCTCTGAGATGG

Das Nukleinsäuresondenmoleküle gemäß SEQ ID No. 147 wird als unmarkierte Kompetitorsonde für den Nachweis von *Candida intermedia* gemeinsam mit der Oligonukleotidsonde gemäß SEQ ID No. 146 eingesetzt, um das Binden der markierten, für *Candida intermedia* spezifischen Oligonukleotidsonde an Nukleinsäuresequenzen, die nicht spezifisch für *Candida intermedia* sind, zu verhindern.

SEQ ID No. 148: 5'- CCTGGTTCGCCAAAAAGGC

Die Sequenz SEQ ID No. 148 ist vor allem zum Nachweis von *Candida parapsilosis* geeignet.

SEQ ID No. 149: 5'-GATTCTCGGCCCCATGGG

Die Sequenz SEQ ID No. 149 ist vor allem zum Nachweis von *Candida crusei (Issatchenkia orientalis)* geeignet.

SEQ ID No. 150: 5'- ACCCTCTACGGCAGCCTGTT

Die Sequenz SEQ ID No. 150 ist vor allem zum gleichzeitigen Nachweis von *Dekkera anomala* und *Brettanomyces (Dekkera) bruxellensis* geeignet.

SEQ ID No. 151: 5'- GATCGGTCTCCAGCGATTCA

Die Sequenz SEQ ID No. 151 ist vor allem zum Nachweis von *Brettanomyces (Dekkera) bruxellensis* geeignet.

SEQ ID No. 152: 5'- ACCCTCCACGGCGGCCTGTT

Die Sequenz SEQ ID No. 152 ist vor allem zum Nachweis von *Brettanomyces (Dekkera) naardenensis* geeignet.

SEQ ID No. 153: 5'- GATTCTCCGCGCCATGGG

Die Sequenz SEQ ID No. 153 ist vor allem zum Nachweis von *Pichia membranaefaciens* geeignet.

SEQ ID No. 154: 5'- TCATCAGACGGGATTCTCAC

Die Sequenz SEQ ID No. 154 ist vor allem zum gleichzeitigen Nachweis von *Pichia minuta* und *Pichia anomala* geeignet.

SEQ ID No. 155: 5'- CTCATCGCACGGGATTCTCACC
SEQ ID No. 156: 5'- CTCGCCACACGGGATTCTCACC

Die Nukleinsäuresondenmoleküle gemäß SEQ ID No. 155 und SEQ ID No. 156 werden als unmarkierte Kompetitorsonden für den gemeinsamen Nachweis von *Pichia minuta* und *Pichia anomala* gemeinsam mit der Oligonukleotidsonde gemäß SEQ ID No. 154 eingesetzt, um das Binden der markierten, für *Pichia minuta* und *Pichia anomala* spezifischen Oligonukleotidsonde an Nukleinsäuresequenzen, die nicht spezifisch für *Pichia minuta* und *Pichia anomala* sind, zu verhindern.

SEQ ID No. 157: 5'-AGTTGCCCCCTCCTCTAAGC

Die Sequenz SEQ ID No. 157 ist vor allem zum Nachweis von *Saccharomyces exiguus* geeignet.

SEQ ID No. 158: 5'-CTGCCACAAGGACAAATGGT
SEQ ID No. 159: 5'-TGCCCCCTCTTCTAAGCAAAT

Die Sequenzen SEQ ID No. 158 und SEQ ID No. 159 sind vor allem zum Nachweis von *Saccharomycodes ludwigii* geeignet.

SEQ ID No. 160: 5'-CCCCAAAGTTGCCCTCTC

Die Sequenz SEQ ID No. 160 ist vor allem zum Nachweis von *Saccharomyces cerevisiae* geeignet.

SEQ ID No. 161: 5'-GCCGCCCCAAAGTCGCCCTCTAC
SEQ ID No. 162: 5'-GCCCCAGAGTCGCCTTCTAC

Die Nukleinsäuresondenmoleküle gemäß SEQ ID No. 161 und SEQ ID No. 162 werden als unmarkierte Kompetitorsonden für den Nachweis von *Saccharomyces cerevisiae* gemeinsam mit der Oligonukleotidsonde gemäß SEQ ID No. 160 eingesetzt, um das Binden der markierten, für *Saccharomyces cerevisiae* spezifischen Oligonukleotidsonde an Nukleinsäuresequenzen, die nicht spezifisch für *Saccharomyces cerevisiae* sind, zu verhindern.
b) Nukleinsäuresondenmoleküle, die spezifisch getränkeschädliche Schimmelpilze nachweisen:

SEQ ID No. 163: 5'-AAGACCAGGCCACCTCAT

Die Sequenz SEQ ID No. 163 ist vor allem zum Nachweis von *Mucor racemosus* geeignet.

SEQ ID No. 164: 5'- CATCATAGAACACCGTCC

Die Sequenz SEQ ID No. 164 ist vor allem zum Nachweis von *Byssochlamys nivea* geeignet.

SEQ ID No. 165: 5'- CCTTCCGAAGTCGAGGTTTT

Die Sequenz SEQ ID No. 165 ist vor allem zum spezifischen Nachweis von *Neosartorya fischeri* geeignet.

SEQ ID No. 166: 5'- GGGAGTGTTGCCAACTC

Die Sequenz SEQ ID No. 166 ist vor allem zum gleichzeitigen Nachweis von *Aspergillus fumigatus* und *A. fischeri* geeignet.

SEQ ID No. 167: 5'- AGCGGTCGTTCGCAACCCT

Die Sequenz SEQ ID No. 167 ist vor allem zum Nachweis von *Talaromyces flavus* geeignet.

SEQ ID No. 168: 5'- CCGAAGTCGGGGTTTTGCGG

Die Sequenz SEQ ID No. 168 ist vor allem zum gleichzeitigen Nachweis von *Talaromyces bacillisporus* und *T. flavus* geeignet.

c) Nukleinsäuresondenmoleküle, die spezifisch getränkeschädliche Milchsäurebakterien nachweisen:

SEQ ID No. 169: 5'- GATAGCCGAAACCACCTTTC
SEQ ID No. 170: 5'- GCCGAAACCACCTTTCAAAC
SEQ ID No. 171: 5'- GTGATAGCCGAAACCACCTT
SEQ ID No. 172: 5'- AGTGATAGCCGAAACCACCT
SEQ ID No. 173: 5'- TTTAACGGGATGCGTTCGAC
SEQ ID No. 174: 5'- AAGTGATAGCCGAAACCACC
SEQ ID No. 175: 5'- GGTTGAATACCGTCAACGTC
SEQ ID No. 176: 5'- GCACAGTATGTCAAGACCTG
SEQ ID No. 177: 5'- CATCCGATGTGCAAGCACTT
SEQ ID No. 178: 5'- TCATCCGATGTGCAAGCACT
SEQ ID No. 179: 5'- CCGATGTGCAAGCACTTCAT
SEQ ID No. 180: 5'- CCACTCATCCGATGTGCAAG
SEQ ID No. 181: 5'- GCCACAGTTCGCCACTCATC
SEQ ID No. 182: 5'- CCTCCGCGTTTGTCACCGGC
SEQ ID No. 183: 5'- ACCAGTTCGCCACAGTTCGC
SEQ ID No. 184: 5'- CACTCATCCGATGTGCAAGC
SEQ ID No. 185: 5'- CCAGTTCGCCACAGTTCGCC
SEQ ID No. 186: 5'- CTCATCCGATGTGCAAGCAC
SEQ ID No. 187: 5'- TCCGATGTGCAAGCACTTCA
SEQ ID No. 188: 5'- CGCCACTCATCCGATGTGCA
SEQ ID No. 189: 5'- CAGTTCGCCACAGTTCGCCA
SEQ ID No. 190: 5'- GCCACTCATCCGATGTGCAA
SEQ ID No. 191: 5'- CGCCACAGTTCGCCACTCAT
SEQ ID No. 192: 5'- ATCCGATGTGCAAGCACTTC
SEQ ID No. 193: 5'- GTTCGCCACAGTTCGCCACT
SEQ ID No. 194: 5'- TCCTCCGCGTTTGTCACCGG
SEQ ID No. 195: 5'- CGCCAGGGTTCATCCTGAGC
SEQ ID No. 196: 5'- AGTTCGCCACAGTTCGCCAC
SEQ ID No. 197: 5'- TCGCCACAGTTCGCCACTCA
SEQ ID No. 198: 5'- TTAACGGGATGCGTTCGACT
SEQ ID No. 199: 5'- TCGCCACTCATCCGATGTGC
SEQ ID No. 200: 5'- CCACAGTTCGCCACTCATCC
SEQ ID No. 201: 5'- GATTTAACGGGATGCGTTCG
SEQ ID No. 202: 5'- TAACGGGATGCGTTCGACTT
SEQ ID No. 203: 5'- AACGGGATGCGTTCGACTTG
SEQ ID No. 204: 5'- CGAAGGTTACCGAACCGACT
SEQ ID No. 205: 5'- CCGAAGGTTACCGAACCGAC
SEQ ID No. 206: 5'- CCCGAAGGTTACCGAACCGA
SEQ ID No. 207: 5'- TTCCTCCGCGTTTGTCACCG

(fortgesetzt)

| | |
|---|---|
| SEQ ID No. 208: | 5'- CCGCCAGGGTTCATCCTGAG |
| SEQ ID No. 209: | 5'- TCCTTCCAGAAGTGATAGCC |
| SEQ ID No. 210: | 5'- CACCAGTTCGCCACAGTTCG |
| SEQ ID No. 211: | 5'- ACGGGATGCGTTCGACTTGC |
| SEQ ID No. 212: | 5'- GTCCTTCCAGAAGTGATAGC |
| SEQ ID No. 213: | 5'- GCCAGGGTTCATCCTGAGCC |
| SEQ ID No. 214: | 5'- ACTCATCCGATGTGCAAGCA |
| SEQ ID No. 215: | 5'- ATCATTGCCTTGGTGAACCG |
| SEQ ID No. 216: | 5'- TCCGCGTTTGTCACCGGCAG |
| SEQ ID No. 217: | 5'- TGAACCGTTACTCCACCAAC |
| SEQ ID No. 218: | 5'- GAAGTGATAGCCGAAACCAC |
| SEQ ID No. 219: | 5'- CCGCGTTTGTCACCGGCAGT |
| SEQ ID No. 220: | 5'- TTCGCCACTCATCCGATGTG |
| SEQ ID No. 221: | 5'- CATTTAACGGGATGCGTTCG |
| SEQ ID No. 222: | 5'- CACAGTTCGCCACTCATCCG |
| SEQ ID No. 223: | 5'- TTCGCCACAGTTCGCCACTC |
| SEQ ID No. 224: | 5'- CTCCGCGTTTGTCACCGGCA |
| SEQ ID No. 225: | 5'- ACGCCGCCAGGGTTCATCCT |
| SEQ ID No. 226: | 5'- CCTTCCAGAAGTGATAGCCG |
| SEQ ID No. 227: | 5'- TCATTGCCTTGGTGAACCGT |
| SEQ ID No. 228: | 5'- CACAGTATGTCAAGACCTGG |
| SEQ ID No. 229: | 5'- TTGGTGAACCGTTACTCCAC |
| SEQ ID No. 230: | 5'- CTTGGTGAACCGTTACTCCA |
| SEQ ID No. 231: | 5'- GTGAACCGTTACTCCACCAA |
| SEQ ID No. 232: | 5'- GGCTCCCGAAGGTTACCGAA |
| SEQ ID No. 233: | 5'- GAAGGTTACCGAACCGACTT |
| SEQ ID No. 234: | 5'- TGGCTCCCGAAGGTTACCGA |
| SEQ ID No. 235: | 5'- TAATACGCCGCGGGTCCTTC |
| SEQ ID No. 236: | 5'- GAACCGTTACTCCACCAACT |
| SEQ ID No. 237: | 5'- TACGCCGCGGGTCCTTCCAG |
| SEQ ID No. 238: | 5'- TCACCAGTTCGCCACAGTTC |
| SEQ ID No. 239: | 5'- CCTTGGTGAACCGTTACTCC |
| SEQ ID No. 240: | 5'- CTCACCAGTTCGCCACAGTT |
| SEQ ID No. 241: | 5'- CGCCGCCAGGGTTCATCCTG |
| SEQ ID No. 242: | 5'- CCTTGGTGAACCATTACTCC |
| SEQ ID No. 243: | 5'- TGGTGAACCATTACTCCACC |
| SEQ ID No. 244: | 5'- GCCGCCAGGGTTCATCCTGA |
| SEQ ID No. 245: | 5'- GGTGAACCATTACTCCACCA |
| SEQ ID No. 246: | 5'- CCAGGGTTCATCCTGAGCCA |
| SEQ ID No. 247: | 5'- AATACGCCGCGGGTCCTTCC |
| SEQ ID No. 248: | 5'- CACGCCGCCAGGGTTCATCC |
| SEQ ID No. 249: | 5'- AGTTCGCCACTCATCCGATG |
| SEQ ID No. 250: | 5'- CGGGATGCGTTCGACTTGCA |
| SEQ ID No. 251: | 5'- CATTGCCTTGGTGAACCGTT |
| SEQ ID No. 252: | 5'- GCACGCCGCCAGGGTTCATC |
| SEQ ID No. 253: | 5'- CTTCCTCCGCGTTTGTCACC |
| SEQ ID No. 254: | 5'- TGGTGAACCGTTACTCCACC |
| SEQ ID No. 255: | 5'- CCTTCCTCCGCGTTTGTCAC |
| SEQ ID No. 256: | 5'- ACGCCGCGGGTCCTTCCAGA |
| SEQ ID No. 257: | 5'- GGTGAACCGTTACTCCACCA |
| SEQ ID No. 258: | 5'- GGGTCCTTCCAGAAGTGATA |

(fortgesetzt)

SEQ ID No. 259: 5'- CTTCCAGAAGTGATAGCCGA
SEQ ID No. 260: 5'- GCCTTGGTGAACCATTACTC
SEQ ID No. 261: 5'- ACAGTTCGCCACTCATCCGA
SEQ ID No. 262: 5'- ACCTTCCTCCGCGTTTGTCA
SEQ ID No. 263: 5'- CGAACCGACTTTGGGTGTTG
SEQ ID No. 264: 5'- GAACCGACTTTGGGTGTTGC
SEQ ID No. 265: 5'- AGGTTACCGAACCGACTTTG
SEQ ID No. 266: 5'- ACCGAACCGACTTTGGGTGT
SEQ ID No. 267: 5'- TTACCGAACCGACTTTGGGT
SEQ ID No. 268: 5'- TACCGAACCGACTTTGGGTG
SEQ ID No. 269: 5'- GTTACCGAACCGACTTTGGG

Die Sequenzen SEQ ID No. 169 bis SEQ ID No. 269 sind vor allem zum Nachweis von *Lactobacillus collinoides* geeignet.

SEQ ID No. 270: 5'- CCTTTCTGGTATGGTACCGTC
SEQ ID No. 271: 5'- TGCACCGCGGAYCCATCTCT

Die Sequenzen SEQ ID No. 270 und SEQ ID No. 271 sind vor allem zum Nachweis von Mikroorganismen der Gattung Leuconostoc geeignet.

SEQ ID No. 272: 5'- AGTTGCAGTCCAGTAAGCCG
SEQ ID No. 273: 5'- GTTGCAGTCCAGTAAGCCGC
SEQ ID No. 274: 5'- CAGTTGCAGTCCAGTAAGCC
SEQ ID No. 275: 5'- TGCAGTCCAGTAAGCCGCCT
SEQ ID No. 276: 5'- TCAGTTGCAGTCCAGTAAGC
SEQ ID No. 277: 5'- TTGCAGTCCAGTAAGCCGCC
SEQ ID No. 278: 5'- GCAGTCCAGTAAGCCGCCTT
SEQ ID No. 279: 5'- GTCAGTTGCAGTCCAGTAAG
SEQ ID No. 280: 5'- CTCTAGGTGACGCCGAAGCG
SEQ ID No. 281: 5'- ATCTCTAGGTGACGCCGAAG
SEQ ID No. 282: 5'- TCTAGGTGACGCCGAAGCGC
SEQ ID No. 283: 5'- TCTCTAGGTGACGCCGAAGC
SEQ ID No. 284: 5'- CCATCTCTAGGTGACGCCGA
SEQ ID No. 285: 5'- CATCTCTAGGTGACGCCGAA
SEQ ID No. 286: 5'- TAGGTGACGCCGAAGCGCCT
SEQ ID No. 287: 5'- CTAGGTGACGCCGAAGCGCC
SEQ ID No. 288: 5'- CTTAGACGGCTCCTTCCTAA
SEQ ID No. 289: 5'- CCTTAGACGGCTCCTTCCTA
SEQ ID No. 290: 5'- ACGTCAGTTGCAGTCCAGTA
SEQ ID No. 291: 5'- CGTCAGTTGCAGTCCAGTAA
SEQ ID No. 292: 5'- ACGCCGAAGCGCCTTTTAAC
SEQ ID No. 293: 5'- GACGCCGAAGCGCCTTTTAA
SEQ ID No. 294: 5'- GCCGAAGCGCCTTTTAACTT
SEQ ID No. 295: 5'- CGCCGAAGCGCCTTTTAACT
SEQ ID No. 296: 5'- GTGACGCCGAAGCGCCTTTT
SEQ ID No. 297: 5'- TGACGCCGAAGCGCCTTTTA
SEQ ID No. 298: 5'- AGACGGCTCCTTCCTAAAAG
SEQ ID No. 299: 5'- ACGGCTCCTTCCTAAAAGGT
SEQ ID No. 300: 5'- GACGGCTCCTTCCTAAAAGG

(fortgesetzt)

SEQ ID No. 301: 5'- CCTTCCTAAAAGGTTAGGCC

Die Sequenzen SEQ ID No. 272 bis SEQ ID No. 301 sind vor allem zum gleichzeitigen Nachweis von *Leuconostoc mesenteroides* und *L. pseudomesenteroides* geeignet.

SEQ ID No. 302: 5'- GGTGACGCCAAAGCGCCTTT
SEQ ID No. 303: 5'- AGGTGACGCCAAAGCGCCTT
SEQ ID No. 304: 5'- TAGGTGACGCCAAAGCGCCT
SEQ ID No. 305: 5'- CTCTAGGTGACGCCAAAGCG
SEQ ID No. 306: 5'- TCTAGGTGACGCCAAAGCGC
SEQ ID No. 307: 5'- CTAGGTGACGCCAAAGCGCC
SEQ ID No. 308: 5'- ACGCCAAAGCGCCTTTTAAC
SEQ ID No. 309: 5'- CGCCAAAGCGCCTTTTAACT
SEQ ID No. 310: 5'- TGACGCCAAAGCGCCTTTTA
SEQ ID No. 311: 5'- TCTCTAGGTGACGCCAAAGC
SEQ ID No. 312: 5'- GTGACGCCAAAGCGCCTTTT
SEQ ID No. 313: 5'- GACGCCAAAGCGCCTTTTAA
SEQ ID No. 314: 5'- ATCTCTAGGTGACGCCAAAG
SEQ ID No. 315: 5'- CATCTCTAGGTGACGCCAAA
SEQ ID No. 316: 5'- TCCATCTCTAGGTGACGCCA
SEQ ID No. 317: 5'- CCATCTCTAGGTGACGCCAA
SEQ ID No. 318: 5'- CTGCCTTAGACGGCTCCCCC
SEQ ID No. 319: 5'- CCTGCCTTAGACGGCTCCCC
SEQ ID No. 320: 5'- GTGTCATGCGACACTGAGTT
SEQ ID No. 321: 5'- TGTGTCATGCGACACTGAGT
SEQ ID No. 322: 5'- CTTTGTGTCATGCGACACTG
SEQ ID No. 323: 5'- TTGTGTCATGCGACACTGAG
SEQ ID No. 324: 5'- TGCCTTAGACGGCTCCCCCT
SEQ ID No. 325: 5'- AGACGGCTCCCCCTAAAAGG
SEQ ID No.326: 5'- TAGACGGCTCCCCCTAAAAG
SEQ ID No. 327: 5'- GCCTTAGACGGCTCCCCCTA
SEQ ID No. 328: 5'- GCTCCCCCTAAAAGGTTAGG
SEQ ID No. 329: 5'- GGCTCCCCCTAAAAGGTTAG
SEQ ID No. 330: 5'- CTCCCCCTAAAAGGTTAGGC
SEQ ID No. 331: 5'- TCCCCCTAAAAGGTTAGGCC
SEQ ID No. 332: 5'- CCCTAAAAGGTTAGGCCACC
SEQ ID No. 333: 5'- CCCCTAAAAGGTTAGGCCAC
SEQ ID No. 334: 5'- CGGCTCCCCCTAAAAGGTTA
SEQ ID No. 335: 5'- CCCCCTAAAAGGTTAGGCCA
SEQ ID No. 336: 5'- CTTAGACGGCTCCCCCTAAA
SEQ ID No. 337: 5'- TTAGACGGCTCCCCCTAAAA
SEQ ID No. 338: 5'- GGGTTCGCAACTCGTTGTAT
SEQ ID No. 339: 5'- CCTTAGACGGCTCCCCCTAA
SEQ ID No. 340: 5'- ACGGCTCCCCCTAAAAGGTT
SEQ ID No. 341: 5'- GACGGCTCCCCCTAAAAGGT

Die Sequenzen SEQ ID No. 302 bis SEQ ID No. 341 sind vor allem zum Nachweis von *Leuconostoc pseudomesenteroides* geeignet.

SEQ ID No. 342: 5'- ACGCCGCAAGACCATCCTCT
SEQ ID No. 343: 5'- CTAATACGCCGCAAGACCAT

(fortgesetzt)

| | |
|---|---|
| SEQ ID No. 344: | 5'- TACGCCGCAAGACCATCCTC |
| SEQ ID No. 345: | 5'- GTTACGATCTAGCAAGCCGC |
| SEQ ID No. 346: | 5'- AATACGCCGCAAGACCATCC |
| SEQ ID No. 347: | 5'- CGCCGCAAGACCATCCTCTA |
| SEQ ID No. 348: | 5'- GCTAATACGCCGCAAGACCA |
| SEQ ID No. 349: | 5'- ACCATCCTCTAGCGATCCAA |
| SEQ ID No. 350: | 5'- TAATACGCCGCAAGACCATC |
| SEQ ID No. 351: | 5'- AGCCATCCCTTTCTGGTAAG |
| SEQ ID No. 352: | 5'- ATACGCCGCAAGACCATCCT |
| SEQ ID No. 353: | 5'- AGTTACGATCTAGCAAGCCG |
| SEQ ID No. 354: | 5'- AGCTAATACGCCGCAAGACC |
| SEQ ID No. 355: | 5'- GCCGCAAGACCATCCTCTAG |
| SEQ ID No. 356: | 5'- TTACGATCTAGCAAGCCGCT |
| SEQ ID No. 357: | 5'- GACCATCCTCTAGCGATCCA |
| SEQ ID No. 358: | 5'- TTGCTACGTCACTAGGAGGC |
| SEQ ID No. 359: | 5'- ACGTCACTAGGAGGCGGAAA |
| SEQ ID No. 360: | 5'- TTTGCTACGTCACTAGGAGG |
| SEQ ID No. 361: | 5'- GCCATCCCTTTCTGGTAAGG |
| SEQ ID No. 362: | 5'- TACGTCACTAGGAGGCGGAA |
| SEQ ID No. 363: | 5'- CGTCACTAGGAGGCGGAAAC |
| SEQ ID No. 364: | 5'- AAGACCATCCTCTAGCGATC |
| SEQ ID No. 365: | 5'- GCACGTATTTAGCCATCCCT |
| SEQ ID No. 366: | 5'- CTCTAGCGATCCAAAAGGAC |
| SEQ ID No. 367: | 5'- CCTCTAGCGATCCAAAAGGA |
| SEQ ID No. 368: | 5'- CCATCCTCTAGCGATCCAAA |
| SEQ ID No. 369: | 5'- GGCACGTATTTAGCCATCCC |
| SEQ ID No. 370: | 5'- TACGATCTAGCAAGCCGCTT |
| SEQ ID No. 371: | 5'- CAGTTACGATCTAGCAAGCC |
| SEQ ID No. 372: | 5'- CCGCAAGACCATCCTCTAGC |
| SEQ ID No. 373: | 5'- CCATCCCTTTCTGGTAAGGT |
| SEQ ID No. 374: | 5'- AGACCATCCTCTAGCGATCC |
| SEQ ID No. 375: | 5'- CAAGACCATCCTCTAGCGAT |
| SEQ ID No. 376: | 5'- GCTACGTCACTAGGAGGCGG |
| SEQ ID No. 377: | 5'- TGCTACGTCACTAGGAGGCG |
| SEQ ID No. 378: | 5'- CTACGTCACTAGGAGGCGGA |
| SEQ ID No. 379: | 5'- CCTCAACGTCAGTTACGATC |
| SEQ ID No. 380: | 5'- GTCACTAGGAGGCGGAAACC |
| SEQ ID No. 381: | 5'- TCCTCTAGCGATCCAAAAGG |
| SEQ ID No. 382: | 5'- TGGCACGTATTTAGCCATCC |
| SEQ ID No. 383: | 5'- ACGATCTAGCAAGCCGCTTT |
| SEQ ID No. 384: | 5'- GCCAGTCTCTCAACTCGGCT |
| SEQ ID No. 385: | 5'- AAGCTAATACGCCGCAAGAC |
| SEQ ID No. 386: | 5'- GTTTGCTACGTCACTAGGAG |
| SEQ ID No. 387: | 5'- CGCCACTCTAGTCATTGCCT |
| SEQ ID No. 388: | 5'- GGCCAGCCAGTCTCTCAACT |
| SEQ ID No. 389: | 5'- CAGCCAGTCTCTCAACTCGG |
| SEQ ID No. 390: | 5'- CCCGAAGATCAATTCAGCGG |
| SEQ ID No. 391: | 5'- CCGGCCAGTCTCTCAACTCG |
| SEQ ID No. 392: | 5'- CCAGCCAGTCTCTCAACTCG |
| SEQ ID No. 393: | 5'- TCATTGCCTCACTTCACCCG |
| SEQ ID No. 394: | 5'- GCCAGCCAGTCTCTCAACTC |

(fortgesetzt)

| | |
|---|---|
| SEQ ID No. 395: | 5'- CACCCGAAGATCAATTCAGC |
| SEQ ID No. 396: | 5'- GTCATTGCCTCACTTCACCC |
| SEQ ID No. 397: | 5'- CATTGCCTCACTTCACCCGA |
| SEQ ID No. 398: | 5'- ATTGCCTCACTTCACCCGAA |
| SEQ ID No. 399: | 5'- CGAAGATCAATTCAGCGGCT |
| SEQ ID No. 400: | 5'- AGTCATTGCCTCACTTCACC |
| SEQ ID No. 401: | 5'- TCGCCACTCTAGTCATTGCC |
| SEQ ID No. 402: | 5'- TTGCCTCACTTCACCCGAAG |
| SEQ ID No. 403: | 5'- CGGCCAGTCTCTCAACTCGG |
| SEQ ID No. 404: | 5'- CTGGCACGTATTTAGCCATC |
| SEQ ID No. 405: | 5'- ACCCGAAGATCAATTCAGCG |
| SEQ ID No. 406: | 5'- TCTAGCGATCCAAAAGGACC |
| SEQ ID No. 407: | 5'- CTAGCGATCCAAAAGGACCT |
| SEQ ID No. 408: | 5'- GCACCCATCGTTTACGGTAT |
| SEQ ID No. 409: | 5'- CACCCATCGTTTACGGTATG |
| SEQ ID No. 410: | 5'- GCCACTCTAGTCATTGCCTC |
| SEQ ID No. 411: | 5'- CGTTTGCTACGTCACTAGGA |
| SEQ ID No. 412: | 5'- GCCTCAACGTCAGTTACGAT |
| SEQ ID No. 413: | 5'- GCCGGCCAGTCTCTCAACTC |
| SEQ ID No. 414: | 5'- TCACTAGGAGGCGGAAACCT |
| SEQ ID No. 415: | 5'- AGCCTCAACGTCAGTTACGA |
| SEQ ID No. 416: | 5'- AGCCAGTCTCTCAACTCGGC |
| SEQ ID No. 417: | 5'- GGCCAGTCTCTCAACTCGGC |
| SEQ ID No. 418: | 5'- CAAGCTAATACGCCGCAAGA |
| SEQ ID No. 419: | 5'- TTCGCCACTCTAGTCATTGC |
| SEQ ID No. 420: | 5'- CCGAAGATCAATTCAGCGGC |
| SEQ ID No. 421: | 5'- CGCAAGACCATCCTCTAGCG |
| SEQ ID No. 422: | 5'- GCAAGACCATCCTCTAGCGA |
| SEQ ID No. 423: | 5'- GCGTTTGCTACGTCACTAGG |
| SEQ ID No. 424: | 5'- CCACTCTAGTCATTGCCTCA |
| SEQ ID No. 425: | 5'- CACTCTAGTCATTGCCTCAC |
| SEQ ID No. 426: | 5'- CCAGTCTCTCAACTCGGCTA |
| SEQ ID No. 427: | 5'- TTACCTTAGGCACCGGCCTC |
| SEQ ID No. 428: | 5'- ACAAGCTAATACGCCGCAAG |
| SEQ ID No. 429: | 5'- TTTACCTTAGGCACCGGCCT |
| SEQ ID No. 430: | 5'- TTTTACCTTAGGCACCGGCC |
| SEQ ID No. 431: | 5'- ATTTTACCTTAGGCACCGGC |
| SEQ ID No. 432: | 5'- GATTTTACCTTAGGCACCGG |
| SEQ ID No. 433: | 5'- CTCACTTCACCCGAAGATCA |
| SEQ ID No. 434: | 5'- ACGCCACCAGCGTTCATCCT |
| SEQ ID No. 435: | 5'- GCCAAGCGACTTTGGGTACT |
| SEQ ID No. 436: | 5'- CGGAAAATTCCCTACTGCAG |
| SEQ ID No. 437: | 5'- CGATCTAGCAAGCCGCTTTC |
| SEQ ID No. 438: | 5'- GGTACCGTCAAGCTGAAAAC |
| SEQ ID No. 439: | 5'- TGCCTCACTTCACCCGAAGA |
| SEQ ID No. 440: | 5'- GGCCGGCCAGTCTCTCAACT |
| SEQ ID No. 441: | 5'- GGTAAGGTACCGTCAAGCTG |
| SEQ ID No. 442: | 5'- GTAAGGTACCGTCAAGCTGA |
| SEQ ID No. 443: | 5'- CCGCAAGACCATCCTCTAGG |
| SEQ ID No. 444: | 5'- ATTTAGCCATCCCTTTCTGG |

Die Sequenzen SEQ ID No. 342 bis SEQ ID No. 444 sind vor allem zum Nachweis von *Oenococcus oeni* geeignet.

SEQ ID No. 445: 5’- AACCCTTCATCACACACG
SEQ ID No. 446: 5’- CGAAACCCTTCATCACAC
SEQ ID No. 447: 5’- ACCCTTCATCACACACGC
SEQ ID No. 448: 5’-TACCGTCACACACTGAAC
SEQ ID No. 449: 5’- AGATACCGTCACACACTG
SEQ ID No. 450: 5’- CACTCAAGGGCGGAAACC
SEQ ID No. 451: 5’- ACCGTCACACACTGAACA
SEQ ID No. 452: 5’- CGTCACACACTGAACAGT
SEQ ID No. 453: 5’- CCGAAACCCTTCATCACA
SEQ ID No. 454: 5’- CCGTCACACACTGAACAG
SEQ ID No. 455: 5’- GATACCGTCACACACTGA
SEQ ID No. 456: 5’- GGTAAGATACCGTCACAC
SEQ ID No. 457: 5’- CCCTTCATCACACACGCG
SEQ ID No. 458: 5’- ACAGTGTTTTACGAGCCG
SEQ ID No. 459: 5’- CAGTGTTTTACGAGCCGA
SEQ ID No. 460: 5’- ACAAAGCGTTCGACTTGC
SEQ ID No. 461: 5’- CGGATAACGCTTGGAACA
SEQ ID No. 462: 5’- AGGGCGGAAACCCTCGAA
SEQ ID No. 463: 5’- GGGCGGAAACCCTCGAAC
SEQ ID No. 464: 5’- GGCGGAAACCCTCGAACA
SEQ ID No. 465: 5’- TGAGGGCTTTCACTTCAG
SEQ ID No. 466: 5’- AGGGCTTTCACTTCAGAC
SEQ ID No. 467: 5’- GAGGGCTTTCACTTCAGA
SEQ ID No. 468: 5’- ACTGCACTCAAGTCATCC
SEQ ID No. 469: 5’- CCGGATAACGCTTGGAAC
SEQ ID No. 470: 5’- TCCGGATAACGCTTGGAA
SEQ ID No. 471: 5’- TATCCCCTGCTAAGAGGT
SEQ ID No. 472: 5’- CCTGCTAAGAGGTAGGTT
SEQ ID No. 473: 5’- CCCTGCTAAGAGGTAGGT
SEQ ID No. 474: 5’- CCCCTGCTAAGAGGTAGG
SEQ ID No. 475: 5’- TCCCCTGCTAAGAGGTAG
SEQ ID No. 476: 5’- ATCCCCTGCTAAGAGGTA
SEQ ID No. 477: 5’- CCGTTCCTTTCTGGTAAG
SEQ ID No. 478: 5’- GCCGTTCCTTTCTGGTAA
SEQ ID No. 479: 5’- AGCCGTTCCTTTCTGGTA
SEQ ID No. 480: 5’- GCACGTATTTAGCCGTTC
SEQ ID No. 481: 5’- CACGTATTTAGCCGTTCC
SEQ ID No. 482: 5’- GGCACGTATTTAGCCGTT
SEQ ID No. 483: 5’- CACTTTCCTCTACTGCAC
SEQ ID No. 484: 5’- CCACTTTCCTCTACTGCA
SEQ ID No. 485: 5’- TCCACTTTCCTCTACTGC
SEQ ID No. 486: 5’- CTTTCCTCTACTGCACTC
SEQ ID No. 487: 5’- TAGCCGTTCCTTTCTGGT
SEQ ID No. 488: 5’- TTAGCCGTTCCTTTCTGG
SEQ ID No. 489: 5’- TTATCCCCTGCTAAGAGG
SEQ ID No. 490: 5’- GTTATCCCCTGCTAAGAG
SEQ ID No. 491: 5’- CCCGTTCGCCACTCTTTG
SEQ ID No. 492: 5’- AGCTGAGGGCTTTCACTT
SEQ ID No. 493: 5’- GAGCTGAGGGCTTTCACT
SEQ ID No. 494: 5’- GCTGAGGGCTTTCACTTC

(fortgesetzt)

SEQ ID No. 495: 5'- CTGAGGGCTTTCACTTCA

Die Sequenzen SEQ ID No. 445 bis SEQ ID No. 495 sind vor allem zum Nachweis von Bakterien der Gattung Weissella geeignet.

SEQ ID No. 496: 5' CCCGTGTCCCGAAGGAAC
SEQ ID No. 497: 5' GCACGAGTATGTCAAGAC
SEQ ID No. 498: 5' GTATCCCGTGTCCCGAAG
SEQ ID No. 499: 5' TCCCGTGTCCCGAAGGAA
SEQ ID No. 500: 5' ATCCCGTGTCCCGAAGGA
SEQ ID No. 501: 5' TATCCCGTGTCCCGAAGG
SEQ ID No. 502: 5' CTTACCTTAGGAAGCGCC
SEQ ID No. 503: 5' TTACCTTAGGAAGCGCCC
SEQ ID No. 504: 5' CCTGTATCCCGTGTCCCG
SEQ ID No. 505: 5' CCACCTGTATCCCGTGTC
SEQ ID No. 506: 5' CACCTGTATCCCGTGTCC
SEQ ID No. 507: 5' ACCTGTATCCCGTGTCCC
SEQ ID No. 508: 5' CTGTATCCCGTGTCCCGA
SEQ ID No. 509: 5' TGTATCCCGTGTCCCGAA
SEQ ID No. 510: 5' CACGAGTATGTCAAGACC
SEQ ID No. 511: 5' CGGTCTTACCTTAGGAAG
SEQ ID No. 512: 5' TAGGAAGCGCCCTCCTTG
SEQ ID No. 513: 5' AGGAAGCGCCCTCCTTGC
SEQ ID No. 514: 5' TTAGGAAGCGCCCTCCTT
SEQ ID No. 515: 5' CTTAGGAAGCGCCCTCCT
SEQ ID No. 516: 5' CCTTAGGAAGCGCCCTCC
SEQ ID No. 517: 5' ACCTTAGGAAGCGCCCTC
SEQ ID No. 518: 5' TGCACACAATGGTTGAGC
SEQ ID No. 519: 5' TACCTTAGGAAGCGCCCT
SEQ ID No. 520: 5' ACCACCTGTATCCCGTGT
SEQ ID No. 521: 5' GCACCACCTGTATCCCGT
SEQ ID No. 522: 5' CACCACCTGTATCCCGTG
SEQ ID No. 523: 5' GCGGTTAGGCAACCTACT
SEQ ID No. 524: 5' TGCGGTTAGGCAACCTAC
SEQ ID No. 525: 5' TTGCGGTTAGGCAACCTA
SEQ ID No. 526: 5' GGTCTTACCTTAGGAAGC
SEQ ID No. 527: 5' GCTAATACAACGCGGGAT
SEQ ID No. 528: 5' CTAATACAACGCGGGATC
SEQ ID No. 529: 5' ATACAACGCGGGATCATC
SEQ ID No. 530: 5' CGGTTAGGCAACCTACTT
SEQ ID No. 531: 5' TGCACCACCTGTATCCCG
SEQ ID No. 532: 5' GAAGCGCCCTCCTTGCGG
SEQ ID No. 533: 5' GGAAGCGCCCTCCTTGCG
SEQ ID No. 534: 5' CGTCCCTTTCTGGTTAGA
SEQ ID No. 535: 5' AGCTAATACAACGCGGGA
SEQ ID No. 536: 5' TAGCTAATACAACGCGGG
SEQ ID No. 537: 5' CTAGCTAATACAACGCGG
SEQ ID No. 538: 5' GGCTATGTATCATCGCCT
SEQ ID No. 539: 5' GAGCCACTGCCTTTTACA
SEQ ID No. 540: 5' GTCGGCTATGTATCATCG
SEQ ID No. 541: 5' GGTCGGCTATGTATCATC

(fortgesetzt)

SEQ ID No. 542: 5’ CAGGTCGGCTATGTATCA
SEQ ID No. 543: 5’ CGGCTATGTATCATCGCC
SEQ ID No. 544: 5’ TCGGCTATGTATCATCGC
SEQ ID No. 545: 5’ GTCTTACCTTAGGAAGCG
SEQ ID No. 546: 5’ TCTTACCTTAGGAAGCGC

Die Sequenzen SEQ ID No. 496 bis SEQ ID No. 546 sind vor allem zum Nachweis von Bakterien der Gattung Lactococcus geeignet.

d) Nukleinsäuresondenmoleküle, die spezifisch getränkeschädliche Essigsäurebakterien nachweisen:

SEQ ID No. 547: 5’- GTACAAACCGCCTACACGCC
SEQ ID No. 548: 5’- TGTACAAACCGCCTACACGC
SEQ ID No. 549: 5’- GATCAGCACGATGTCGCCAT
SEQ ID No. 550: 5’- CTGTACAAACCGCCTACACG
SEQ ID No. 551: 5’- GAGATCAGCACGATGTCGCC
SEQ ID No. 552: 5’- AGATCAGCACGATGTCGCCA
SEQ ID No. 553: 5’- ATCAGCACGATGTCGCCATC
SEQ ID No. 554: 5’- TCAGCACGATGTCGCCATCT
SEQ ID No. 555: 5’- ACTGTACAAACCGCCTACAC
SEQ ID No. 556: 5’- CCGCCACTAAGGCCGAAACC
SEQ ID No. 557: 5’- CAGCACGATGTCGCCATCTA
SEQ ID No. 558: 5’- TACAAACCGCCTACACGCCC
SEQ ID No. 559: 5’- AGCACGATGTCGCCATCTAG
SEQ ID No. 560: 5’- CGGCTTTTAGAGATCAGCAC
SEQ ID No. 561: 5’- TCCGCCACTAAGGCCGAAAC
SEQ ID No. 562: 5’- GACTGTACAAACCGCCTACA
SEQ ID No. 563: 5’- GTCCGCCACTAAGGCCGAAA
SEQ ID No. 564: 5’- GGGGATTTCACATCTGACTG
SEQ ID No. 565: 5’- CATACAAGCCCTGGTAAGGT
SEQ ID No. 566: 5’- ACAAGCCCTGGTAAGGTTCT
SEQ ID No. 567: 5’- ACAAACCGCCTACACGCCCT
SEQ ID No. 568: 5’- CTGACTGTACAAACCGCCTA
SEQ ID No. 569: 5’- TGACTGTACAAACCGCCTAC
SEQ ID No. 570: 5’- ACGATGTCGCCATCTAGCTT
SEQ ID No. 571: 5’- CACGATGTCGCCATCTAGCT
SEQ ID No. 572: 5’- CGATGTCGCCATCTAGCTTC
SEQ ID No. 573: 5’- GCACGATGTCGCCATCTAGC
SEQ ID No. 574: 5’- GATGTCGCCATCTAGCTTCC
SEQ ID No. 575: 5’- ATGTCGCCATCTAGCTTCCC
SEQ ID No. 576: 5’- TGTCGCCATCTAGCTTCCCA
SEQ ID No. 577: 5’- GCCATCTAGCTTCCCACTGT
SEQ ID No. 578: 5’- TCGCCATCTAGCTTCCCACT
SEQ ID No. 579: 5’- CGCCATCTAGCTTCCCACTG
SEQ ID No. 580: 5’- GTCGCCATCTAGCTTCCCAC
SEQ ID No. 581: 5’- TACAAGCCCTGGTAAGGTTC
SEQ ID No. 582: 5’- GCCACTAAGGCCGAAACCTT
SEQ ID No. 583: 5’- ACTAAGGCCGAAACCTTCGT
SEQ ID No. 584: 5’- CTAAGGCCGAAACCTTCGTG
SEQ ID No. 585: 5’- CACTAAGGCCGAAACCTTCG
SEQ ID No. 586: 5’- AAGGCCGAAACCTTCGTGCG
SEQ ID No. 587: 5’- CCACTAAGGCCGAAACCTTC

(fortgesetzt)

SEQ ID No. 588: 5'- TAAGGCCGAAACCTTCGTGC
SEQ ID No. 589: 5'- AGGCCGAAACCTTCGTGCGA
SEQ ID No. 590: 5'- TCTGACTGTACAAACCGCCT
SEQ ID No. 591: 5'- CATCTGACTGTACAAACCGC
SEQ ID No. 592: 5'- ATCTGACTGTACAAACCGCC
SEQ ID No. 593: 5'- CTTCGTGCGACTTGCATGTG
SEQ ID No. 594: 5'- CCTTCGTGCGACTTGCATGT
SEQ ID No. 595: 5'- CTCTCTAGAGTGCCCACCCA
SEQ ID No. 596: 5'- TCTCTAGAGTGCCCACCCAA
SEQ ID No. 597: 5'- ACGTATCAAATGCAGCTCCC
SEQ ID No. 598: 5'- CGTATCAAATGCAGCTCCCA
SEQ ID No. 599: 5'- CGCCACTAAGGCCGAAACCT
SEQ ID No. 600: 5'- CCGAAACCTTCGTGCGACTT
SEQ ID No. 601: 5'- GCCGAAACCTTCGTGCGACT
SEQ ID No. 602: 5'- AACCTTCGTGCGACTTGCAT
SEQ ID No. 603: 5'- CGAAACCTTCGTGCGACTTG
SEQ ID No. 604: 5'- ACCTTCGTGCGACTTGCATG
SEQ ID No. 605: 5'- GAAACCTTCGTGCGACTTGC
SEQ ID No. 606: 5'- GGCCGAAACCTTCGTGCGAC
SEQ ID No. 607: 5'- AAACCTTCGTGCGACTTGCA
SEQ ID No. 608: 5'- CACGTATCAAATGCAGCTCC

Die Sequenzen SEQ ID No. 547 bis SEQ ID No. 608 sind vor allem zum gleichzeitigen Nachweis von Bakterien der Gattungen Acetobacter und Gluconobacter geeignet.

SEQ ID No. 609: 5'- GCTCACCGGCTTAAGGTCAA
SEQ ID No. 610: 5'- CGCTCACCGGCTTAAGGTCA
SEQ ID No. 611: 5'- TCGCTCACCGGCTTAAGGTC
SEQ ID No. 612: 5'- CTCACCGGCTTAAGGTCAAA
SEQ ID No. 613: 5'- CCCGACCGTGGTCGGCTGCG
SEQ ID No. 614: 5'- GCTCACCGGCTTAAGGTCAA
SEQ ID No. 615: 5'- CGCTCACCGGCTTAAGGTCA
SEQ ID No. 616: 5'- TCGCTCACCGGCTTAAGGTC
SEQ ID No. 617: 5'- CTCACCGGCTTAAGGTCAAA
SEQ ID No. 618: 5'- CCCGACCGTGGTCGGCTGCG
SEQ ID No. 619: 5'- TCACCGGCTTAAGGTCAAAC
SEQ ID No. 620: 5'- CAACCCTCTCTCACACTCTA
SEQ ID No. 621: 5'- ACAACCCTCTCTCACACTCT
SEQ ID No. 622: 5'- CCACAACCCTCTCTCACACT
SEQ ID No. 623: 5'- AACCCTCTCTCACACTCTAG
SEQ ID No. 624: 5'- CACAACCCTCTCTCACACTC
SEQ ID No. 625: 5'- TCCACAACCCTCTCTCACAC
SEQ ID No. 626: 5'- TTCCACAACCCTCTCTCACA
SEQ ID No. 627: 5'- ACCCTCTCTCACACTCTAGT
SEQ ID No. 628: 5'- GAGCCAGGTTGCCGCCTTCG
SEQ ID No. 629: 5'- AGGTCAAACCAACTCCCATG
SEQ ID No. 630: 5'- ATGAGCCAGGTTGCCGCCTT
SEQ ID No. 631: 5'- TGAGCCAGGTTGCCGCCTTC
SEQ ID No. 632: 5'- AGGCTCCTCCACAGGCGACT
SEQ ID No. 633: 5'- CAGGCTCCTCCACAGGCGAC
SEQ ID No. 634: 5'- GCAGGCTCCTCCACAGGCGA

(fortgesetzt)

SEQ ID No. 635: 5'- TTCGCTCACCGGCTTAAGGT
SEQ ID No. 636: 5'- GTTCGCTCACCGGCTTAAGG
SEQ ID No. 637: 5'- GGTTCGCTCACCGGCTTAAG
SEQ ID No. 638: 5'- ATTCCACAACCCTCTCTCAC
SEQ ID No. 639: 5'- TGACCCGACCGTGGTCGGCT
SEQ ID No. 640: 5'- CCCTCTCTCACACTCTAGTC
SEQ ID No. 641: 5'- GAATTCCACAACCCTCTCTC
SEQ ID No. 642: 5'- AGCCAGGTTGCCGCCTTCGC
SEQ ID No. 643: 5'- GCCAGGTTGCCGCCTTCGCC
SEQ ID No. 644: 5'- GGAATTCCACAACCCTCTCT
SEQ ID No. 645: 5'- GGGAATTCCACAACCCTCTC
SEQ ID No. 646: 5'- AACGCAGGCTCCTCCACAGG
SEQ ID No. 647: 5'- CGGCTTAAGGTCAAACCAAC
SEQ ID No. 648: 5'- CCGGCTTAAGGTCAAACCAA
SEQ ID No. 649: 5'- CACCGGCTTAAGGTCAAACC
SEQ ID No. 650: 5'- ACCGGCTTAAGGTCAAACCA
SEQ ID No. 651: 5'- ACCCAACATCCAGCACACAT
SEQ ID No. 652: 5'- TCGCTGACCCGACCGTGGTC
SEQ ID No. 653: 5'- CGCTGACCCGACCGTGGTCG
SEQ ID No. 654: 5'- GACCCGACCGTGGTCGGCTG
SEQ ID No. 655: 5'- GCTGACCCGACCGTGGTCGG
SEQ ID No. 656: 5'- CTGACCCGACCGTGGTCGGC
SEQ ID No. 657: 5'- CAGGCGACTTGCGCCTTTGA
SEQ ID No. 658: 5'- TCATGCGGTATTAGCTCCAG
SEQ ID No. 659: 5'- ACTAGCTAATCGAACGCAGG
SEQ ID No. 660: 5'- CATGCGGTATTAGCTCCAGT
SEQ ID No. 661: 5'- CGCAGGCTCCTCCACAGGCG
SEQ ID No. 662: 5'- ACGCAGGCTCCTCCACAGGC
SEQ ID No. 663: 5'- CTCAGGTGTCATGCGGTATT
SEQ ID No. 664: 5'- CGCCTTTGACCCTCAGGTGT
SEQ ID No. 665: 5'- ACCCTCAGGTGTCATGCGGT
SEQ ID No. 666: 5'- CCTCAGGTGTCATGCGGTAT
SEQ ID No. 667: 5'- TTTGACCCTCAGGTGTCATG
SEQ ID No. 668: 5'- GACCCTCAGGTGTCATGCGG
SEQ ID No. 669: 5'- TGACCCTCAGGTGTCATGCG
SEQ ID No. 670: 5'- GCCTTTGACCCTCAGGTGTC
SEQ ID No. 671: 5'- TTGACCCTCAGGTGTCATGC
SEQ ID No. 672: 5'- CCCTCAGGTGTCATGCGGTA
SEQ ID No. 673: 5'- CCTTTGACCCTCAGGTGTCA
SEQ ID No. 674: 5'- CTTTGACCCTCAGGTGTCAT
SEQ ID No. 675: 5'- AGTTATCCCCCACCCATGGA
SEQ ID No. 676: 5'- CCAGCTATCGATCATCGCCT
SEQ ID No. 677: 5'- ACCAGCTATCGATCATCGCC
SEQ ID No. 678: 5'- CAGCTATCGATCATCGCCTT
SEQ ID No. 679: 5'- AGCTATCGATCATCGCCTTG
SEQ ID No. 680: 5'- GCTATCGATCATCGCCTTGG
SEQ ID No. 681: 5'- CTATCGATCATCGCCTTGGT
SEQ ID No. 682: 5'- TTCGTGCGACTTGCATGTGT
SEQ ID No. 683: 5'- TCGATCATCGCCTTGGTAGG
SEQ ID No. 684: 5'- ATCGATCATCGCCTTGGTAG
SEQ ID No. 685: 5'- CACAGGCGACTTGCGCCTTT

(fortgesetzt)

| | |
|---|---|
| SEQ ID No. 686: | 5'- CCACAGGCGACTTGCGCCTT |
| SEQ ID No. 687: | 5'- TCCACAGGCGACTTGCGCCT |
| SEQ ID No. 688: | 5'- TCCTCCACAGGCGACTTGCG |
| SEQ ID No. 689: | 5'- CCTCCACAGGCGACTTGCGC |
| SEQ ID No. 690: | 5'- CTCCACAGGCGACTTGCGCC |
| SEQ ID No. 691: | 5'- ACAGGCGACTTGCGCCTTTG |
| SEQ ID No. 692: | 5'- GCTCACCGGCTTAAGGTCAA |
| SEQ ID No. 693: | 5'- CGCTCACCGGCTTAAGGTCA |
| SEQ ID No. 694: | 5'- TCGCTCACCGGCTTAAGGTC |
| SEQ ID No. 695: | 5'- CTCACCGGCTTAAGGTCAAA |
| SEQ ID No. 696: | 5'- CCCGACCGTGGTCGGCTGCG |
| SEQ ID No. 697: | 5'- TCACCGGCTTAAGGTCAAAC |
| SEQ ID No. 698: | 5'- CAACCCTCTCTCACACTCTA |
| SEQ ID No. 699: | 5'- ACAACCCTCTCTCACACTCT |
| SEQ ID No. 700: | 5'- CCACAACCCTCTCTCACACT |
| SEQ ID No. 701: | 5'- AACCCTCTCTCACACTCTAG |
| SEQ ID No. 702: | 5'- CACAACCCTCTCTCACACTC |
| SEQ ID No. 703: | 5'- TCCACAACCCTCTCTCACAC |
| SEQ ID No. 704: | 5'- TTCCACAACCCTCTCTCACA |
| SEQ ID No. 705: | 5'- ACCCTCTCTCACACTCTAGT |
| SEQ ID No. 706: | 5'- GAGCCAGGTTGCCGCCTTCG |
| SEQ ID No. 707: | 5'- AGGTCAAACCAACTCCCATG |
| SEQ ID No. 708: | 5'- ATGAGCCAGGTTGCCGCCTT |
| SEQ ID No. 709: | 5'- TGAGCCAGGTTGCCGCCTTC |
| SEQ ID No. 710: | 5'- AGGCTCCTCCACAGGCGACT |
| SEQ ID No. 711: | 5'- CAGGCTCCTCCACAGGCGAC |
| SEQ ID No. 712: | 5'- GCAGGCTCCTCCACAGGCGA |
| SEQ ID No. 713: | 5'- TTCGCTCACCGGCTTAAGGT |
| SEQ ID No. 714: | 5'- GTTCGCTCACCGGCTTAAGG |
| SEQ ID No. 715: | 5'- GGTTCGCTCACCGGCTTAAG |
| SEQ ID No. 716: | 5'- ATTCCACAACCCTCTCTCAC |
| SEQ ID No. 717: | 5'- TGACCCGACCGTGGTCGGCT |
| SEQ ID No. 718: | 5'- CCCTCTCTCACACTCTAGTC |
| SEQ ID No. 719: | 5'- GAATTCCACAACCCTCTCTC |
| SEQ ID No. 720: | 5'- AGCCAGGTTGCCGCCTTCGC |
| SEQ ID No. 721: | 5'- GCCAGGTTGCCGCCTTCGCC |
| SEQ ID No. 722: | 5'- GGAATTCCACAACCCTCTCT |
| SEQ ID No. 723: | 5'- GGGAATTCCACAACCCTCTC |
| SEQ ID No. 724: | 5'- AACGCAGGCTCCTCCACAGG |
| SEQ ID No. 725: | 5'- CGGCTTAAGGTCAAACCAAC |
| SEQ ID No. 726: | 5'- CCGGCTTAAGGTCAAACCAA |
| SEQ ID No. 727: | 5'- CACCGGCTTAAGGTCAAACC |
| SEQ ID No. 728: | 5'- ACCGGCTTAAGGTCAAACCA |
| SEQ ID No. 729: | 5'- ACCCAACATCCAGCACACAT |
| SEQ ID No. 730: | 5'- TCGCTGACCCGACCGTGGTC |
| SEQ ID No. 731: | 5'- CGCTGACCCGACCGTGGTCG |
| SEQ ID No. 732: | 5'- GACCCGACCGTGGTCGGCTG |
| SEQ ID No. 733: | 5'- GCTGACCCGACCGTGGTCGG |
| SEQ ID No. 734: | 5'- CTGACCCGACCGTGGTCGGC |
| SEQ ID No. 735: | 5'- CAGGCGACTTGCGCCTTTGA |
| SEQ ID No. 736: | 5'- TCATGCGGTATTAGCTCCAG |

(fortgesetzt)

SEQ ID No. 737: 5'- ACTAGCTAATCGAACGCAGG
SEQ ID No. 738: 5'- CATGCGGTATTAGCTCCAGT
SEQ ID No. 739: 5'- CGCAGGCTCCTCCACAGGCG
SEQ ID No. 740: 5'- ACGCAGGCTCCTCCACAGGC
SEQ ID No. 741: 5'- CTCAGGTGTCATGCGGTATT
SEQ ID No. 742: 5'- CGCCTTTGACCCTCAGGTGT
SEQ ID No. 743: 5'- ACCCTCAGGTGTCATGCGGT
SEQ ID No. 744: 5'- CCTCAGGTGTCATGCGGTAT
SEQ ID No. 745: 5'- TTTGACCCTCAGGTGTCATG
SEQ ID No. 746: 5'- GACCCTCAGGTGTCATGCGG
SEQ ID No. 747: 5'- TGACCCTCAGGTGTCATGCG
SEQ ID No. 748: 5'- GCCTTTGACCCTCAGGTGTC
SEQ ID No. 749: 5'- TTGACCCTCAGGTGTCATGC
SEQ ID No. 750: 5'- CCCTCAGGTGTCATGCGGTA
SEQ ID No. 751: 5'- CCTTTGACCCTCAGGTGTCA
SEQ ID No. 752: 5'- CTTTGACCCTCAGGTGTCAT
SEQ ID No. 753: 5'- AGTTATCCCCCACCCATGGA
SEQ ID No. 754: 5'- CCAGCTATCGATCATCGCCT
SEQ ID No. 755: 5'- ACCAGCTATCGATCATCGCC
SEQ ID No. 756: 5'- CAGCTATCGATCATCGCCTT
SEQ ID No. 757: 5'- AGCTATCGATCATCGCCTTG
SEQ ID No. 758: 5'- GCTATCGATCATCGCCTTGG
SEQ ID No. 759: 5'- CTATCGATCATCGCCTTGGT
SEQ ID No. 760: 5'- TTCGTGCGACTTGCATGTGT
SEQ ID No. 761: 5'- TCGATCATCGCCTTGGTAGG
SEQ ID No. 762: 5'- ATCGATCATCGCCTTGGTAG
SEQ ID No. 763: 5'- CACAGGCGACTTGCGCCTTT
SEQ ID No. 764: 5'- CCACAGGCGACTTGCGCCTT
SEQ ID No. 765: 5'- TCCACAGGCGACTTGCGCCT
SEQ ID No. 766: 5'- TCCTCCACAGGCGACTTGCG
SEQ ID No. 767: 5'- CCTCCACAGGCGACTTGCGC
SEQ ID No. 768: 5'- CTCCACAGGCGACTTGCGCC
SEQ ID No. 769: 5'- ACAGGCGACTTGCGCCTTTG
SEQ ID No. 770: 5'- TCACCGGCTTAAGGTCAAAC
SEQ ID No. 771: 5'- CAACCCTCTCTCACACTCTA
SEQ ID No. 772: 5'- ACAACCCTCTCTCACACTCT
SEQ ID No. 773: 5'- CCACAACCCTCTCTCACACT
SEQ ID No. 774: 5'- AACCCTCTCTCACACTCTAG
SEQ ID No. 775: 5'- CACAACCCTCTCTCACACTC
SEQ ID No. 776: 5'- TCCACAACCCTCTCTCACAC
SEQ ID No. 777: 5'- TTCCACAACCCTCTCTCACA
SEQ ID No. 778: 5'- ACCCTCTCTCACACTCTAGT
SEQ ID No. 779: 5'- GAGCCAGGTTGCCGCCTTCG
SEQ ID No. 780: 5'- AGGTCAAACCAACTCCCATG
SEQ ID No. 781: 5'- ATGAGCCAGGTTGCCGCCTT
SEQ ID No. 782: 5'- TGAGCCAGGTTGCCGCCTTC
SEQ ID No. 783: 5'- AGGCTCCTCCACAGGCGACT
SEQ ID No. 784: 5'- CAGGCTCCTCCACAGGCGAC
SEQ ID No. 785: 5'- GCAGGCTCCTCCACAGGCGA
SEQ ID No. 786: 5'- TTCGCTCACCGGCTTAAGGT
SEQ ID No. 787: 5'- GTTCGCTCACCGGCTTAAGG

(fortgesetzt)

| | |
|---|---|
| SEQ ID No. 788: | 5'- GGTTCGCTCACCGGCTTAAG |
| SEQ ID No. 789: | 5'- ATTCCACAACCCTCTCTCAC |
| SEQ ID No. 790: | 5'- TGACCCGACCGTGGTCGGCT |
| SEQ ID No. 791: | 5'- CCCTCTCTCACACTCTAGTC |
| SEQ ID No. 792: | 5'- GAATTCCACAACCCTCTCTC |
| SEQ ID No. 793: | 5'- AGCCAGGTTGCCGCCTTCGC |
| SEQ ID No. 794: | 5'- GCCAGGTTGCCGCCTTCGCC |
| SEQ ID No. 795: | 5'- GGAATTCCACAACCCTCTCT |
| SEQ ID No. 796: | 5'- GGGAATTCCACAACCCTCTC |
| SEQ ID No. 797: | 5'- AACGCAGGCTCCTCCACAGG |
| SEQ ID No. 798: | 5'- CGGCTTAAGGTCAAACCAAC |
| SEQ ID No. 799: | 5'- CCGGCTTAAGGTCAAACCAA |
| SEQ ID No. 800: | 5'- CACCGGCTTAAGGTCAAACC |
| SEQ ID No. 801: | 5'- ACCGGCTTAAGGTCAAACCA |
| SEQ ID No. 802: | 5'- ACCCAACATCCAGCACACAT |
| SEQ ID No. 803: | 5'- TCGCTGACCCGACCGTGGTC |
| SEQ ID No. 804: | 5'- CGCTGACCCGACCGTGGTCG |
| SEQ ID No. 805: | 5'- GACCCGACCGTGGTCGGCTG |
| SEQ ID No. 806: | 5'- GCTGACCCGACCGTGGTCGG |
| SEQ ID No. 807: | 5'- CTGACCCGACCGTGGTCGGC |
| SEQ ID No. 808: | 5'- CAGGCGACTTGCGCCTTTGA |
| SEQ ID No. 809: | 5'- TCATGCGGTATTAGCTCCAG |
| SEQ ID No.810: | 5'- ACTAGCTAATCGAACGCAGG |
| SEQ ID No. 811: | 5'- CATGCGGTATTAGCTCCAGT |
| SEQ ID No. 812: | 5'- CGCAGGCTCCTCCACAGGCG |
| SEQ ID No. 813: | 5'- ACGCAGGCTCCTCCACAGGC |
| SEQ ID No. 814: | 5'- CTCAGGTGTCATGCGGTATT |
| SEQ ID No. 815: | 5'- CGCCTTTGACCCTCAGGTGT |
| SEQ ID No. 816: | 5'- ACCCTCAGGTGTCATGCGGT |
| SEQ ID No. 817: | 5'- CCTCAGGTGTCATGCGGTAT |
| SEQ ID No. 818: | 5'- TTTGACCCTCAGGTGTCATG |
| SEQ ID No. 819: | 5'- GACCCTCAGGTGTCATGCGG |
| SEQ ID No. 820: | 5'- TGACCCTCAGGTGTCATGCG |
| SEQ ID No. 821: | 5'- GCCTTTGACCCTCAGGTGTC |
| SEQ ID No. 822: | 5'- TTGACCCTCAGGTGTCATGC |
| SEQ ID No. 823: | 5'- CCCTCAGGTGTCATGCGGTA |
| SEQ ID No. 824: | 5'- CCTTTGACCCTCAGGTGTCA |
| SEQ ID No. 825: | 5'- CTTTGACCCTCAGGTGTCAT |
| SEQ ID No. 826: | 5'- AGTTATCCCCCACCCATGGA |
| SEQ ID No. 827: | 5'- CCAGCTATCGATCATCGCCT |
| SEQ ID No. 828: | 5'- ACCAGCTATCGATCATCGCC |
| SEQ ID No. 829: | 5'- CAGCTATCGATCATCGCCTT |
| SEQ ID No. 830: | 5'- AGCTATCGATCATCGCCTTG |
| SEQ ID No. 831: | 5'- GCTATCGATCATCGCCTTGG |
| SEQ ID No. 832: | 5'- CTATCGATCATCGCCTTGGT |
| SEQ ID No. 833: | 5'- TTCGTGCGACTTGCATGTGT |
| SEQ ID No. 834: | 5'- TCGATCATCGCCTTGGTAGG |
| SEQ ID No. 835: | 5'- ATCGATCATCGCCTTGGTAG |
| SEQ ID No. 836: | 5'- CACAGGCGACTTGCGCCTTT |
| SEQ ID No. 837: | 5'- CCACAGGCGACTTGCGCCTT |
| SEQ ID No. 838: | 5'- TCCACAGGCGACTTGCGCCT |

(fortgesetzt)

| | |
|---|---|
| SEQ ID No. 839: | 5'- TCCTCCACAGGCGACTTGCG |
| SEQ ID No. 840: | 5'- CCTCCACAGGCGACTTGCGC |
| SEQ ID No. 841: | 5'- CTCCACAGGCGACTTGCGCC |
| SEQ ID No. 842: | 5'- ACAGGCGACTTGCGCCTTTG |

Die Sequenzen SEQ ID No. 609 bis SEQ ID No. 842 sind vor allem zum gleichzeitigen Nachweis von Bakterien der Gattungen Acetobacter, Gluconobacter und Gluconoacetobacter geeignet.

e) Nukleinsäuresondenmoleküle, die spezifisch getränkeschädliche Bazillen nachweisen:

| | |
|---|---|
| SEQ ID No. 843: | 5'- AGCCCCGGTTTCCCGGCGTT |
| SEQ ID No. 844: | 5'- CGCCTTTCCTTTTTCCTCCA |
| SEQ ID No. 845: | 5'- GCCCCGGTTTCCCGGCGTTA |
| SEQ ID No. 846: | 5'- GCCGCCTTTCCTTTTTCCTC |
| SEQ ID No. 847: | 5'- TAGCCCCGGTTTCCCGGCGT |
| SEQ ID No. 848: | 5'- CCGGGTACCGTCAAGGCGCC |
| SEQ ID No. 849: | 5'- AAGCCGCCTTTCCTTTTTCC |
| SEQ ID No. 850: | 5'- CCCCGGTTTCCCGGCGTTAT |
| SEQ ID No. 851: | 5'- CCGGCGTTATCCCAGTCTTA |
| SEQ ID No. 852: | 5'- AGCCGCCTTTCCTTTTTCCT |
| SEQ ID No. 853: | 5'- CCGCCTTTCCTTTTTCCTCC |
| SEQ ID No. 854: | 5'- TTAGCCCCGGTTTCCCGGCG |
| SEQ ID No. 855: | 5'- CCCGGCGTTATCCCAGTCTT |
| SEQ ID No. 856: | 5'- GCCGGGTACCGTCAAGGCGC |
| SEQ ID No. 857: | 5'- GGCCGGGTACCGTCAAGGCG |
| SEQ ID No. 858: | 5'- TCCCGGCGTTATCCCAGTCT |
| SEQ ID No. 859: | 5'- TGGCCGGGTACCGTCAAGGC |
| SEQ ID No. 860: | 5'- GAAGCCGCCTTTCCTTTTTC |
| SEQ ID No. 861: | 5'- CCCGGTTTCCCGGCGTTATC |
| SEQ ID No. 862: | 5'- CGGCGTTATCCCAGTCTTAC |
| SEQ ID No. 863: | 5'- GGCGTTATCCCAGTCTTACA |
| SEQ ID No. 864: | 5'- GCGTTATCCCAGTCTTACAG |
| SEQ ID No. 865: | 5'- CGGGTACCGTCAAGGCGCCG |
| SEQ ID No. 866: | 5'- ATTAGCCCCGGTTTCCCGGC |
| SEQ ID No. 867: | 5'- AAGGGGAAGGCCCTGTCTCC |
| SEQ ID No. 868: | 5'- GGCCCTGTCTCCAGGGAGGT |
| SEQ ID No. 869: | 5'- AGGCCCTGTCTCCAGGGAGG |
| SEQ ID No. 870: | 5'- AAGGCCCTGTCTCCAGGGAG |
| SEQ ID No. 871: | 5'- GCCCTGTCTCCAGGGAGGTC |
| SEQ ID No. 872: | 5'- CGTTATCCCAGTCTTACAGG |
| SEQ ID No. 873: | 5'- GGGTACCGTCAAGGCGCCGC |
| SEQ ID No. 874: | 5'- CGGCAACAGAGTTTTACGAC |
| SEQ ID No. 875: | 5'- GGGGAAGGCCCTGTCTCCAG |
| SEQ ID No. 876: | 5'- AGGGGAAGGCCCTGTCTCCA |
| SEQ ID No. 877: | 5'- GCAGCCGAAGCCGCCTTTCC |
| SEQ ID No. 878: | 5'- TTCTTCCCCGGCAACAGAGT |
| SEQ ID No. 879: | 5'- CGGCACTTGTTCTTCCCCGG |
| SEQ ID No. 880: | 5'- GTTCTTCCCCGGCAACAGAG |
| SEQ ID No. 881: | 5'- GGCACTTGTTCTTCCCCGGC |
| SEQ ID No. 882: | 5'- GCACTTGTTCTTCCCCGGCA |
| SEQ ID No. 883: | 5'- CACTTGTTCTTCCCCGGCAA |
| SEQ ID No. 884: | 5'- TCTTCCCCGGCAACAGAGTT |

(fortgesetzt)

| | |
|---|---|
| SEQ ID No. 885: | 5'- TTGTTCTTCCCCGGCAACAG |
| SEQ ID No. 886: | 5'- ACTTGTTCTTCCCCGGCAAC |
| SEQ ID No. 887: | 5'- TGTTCTTCCCCGGCAACAGA |
| SEQ ID No. 888: | 5'- CTTGTTCTTCCCCGGCAACA |
| SEQ ID No. 889: | 5'- ACGGCACTTGTTCTTCCCCG |
| SEQ ID No. 890: | 5'- GTCCGCCGCTAACCTTTTAA |
| SEQ ID No. 891: | 5'- CTGGCCGGGTACCGTCAAGG |
| SEQ ID No. 892: | 5'- TCTGGCCGGGTACCGTCAAG |
| SEQ ID No. 893: | 5'- TTCTGGCCGGGTACCGTCAA |
| SEQ ID No. 894: | 5'- CAATGCTGGCAACTAAGGTC |
| SEQ ID No. 895: | 5'- CGTCCGCCGCTAACCTTTTA |
| SEQ ID No. 896: | 5'- CGAAGCCGCCTTTCCTTTTT |
| SEQ ID No. 897: | 5'- CCGAAGCCGCCTTTCCTTTT |
| SEQ ID No. 898: | 5'- GCCGAAGCCGCCTTTCCTTT |
| SEQ ID No. 899: | 5'- AGCCGAAGCCGCCTTTCCTT |
| SEQ ID No. 900: | 5'- ACCGTCAAGGCGCCGCCCTG |
| SEQ ID No. 901: | 5'- CCGTGGCTTTCTGGCCGGGT |
| SEQ ID No. 902: | 5'- GCTTTCTGGCCGGGTACCGT |
| SEQ ID No. 903: | 5'- GCCGTGGCTTTCTGGCCGGG |
| SEQ ID No. 904: | 5'- GGCTTTCTGGCCGGGTACCG |
| SEQ ID No. 905: | 5'- CTTTCTGGCCGGGTACCGTC |
| SEQ ID No. 906: | 5'- TGGCTTTCTGGCCGGGTACC |
| SEQ ID No. 907: | 5'- GTGGCTTTCTGGCCGGGTAC |
| SEQ ID No. 908: | 5'- CGTGGCTTTCTGGCCGGGTA |
| SEQ ID No. 909: | 5'- TTTCTGGCCGGGTACCGTCA |
| SEQ ID No. 910: | 5'- GGGAAGGCCCTGTCTCCAGG |
| SEQ ID No. 911: | 5'- CGAAGGGGAAGGCCCTGTCT |
| SEQ ID No. 912: | 5'- CCGAAGGGGAAGGCCCTGTC |
| SEQ ID No. 913: | 5'- GAAGGGGAAGGCCCTGTCTC |
| SEQ ID No. 914: | 5'- GGCGCCGCCCTGTTCGAACG |
| SEQ ID No. 915: | 5'- AGGCGCCGCCCTGTTCGAAC |
| SEQ ID No. 916: | 5'- AAGGCGCCGCCCTGTTCGAA |
| SEQ ID No. 917: | 5'- CCCGGCAACAGAGTTTTACG |
| SEQ ID No. 918: | 5'- CCCCGGCAACAGAGTTTTAC |
| SEQ ID No. 919: | 5'- CCATCTGTAAGTGGCAGCCG |
| SEQ ID No. 920: | 5'- TCTGTAAGTGGCAGCCGAAG |
| SEQ ID No. 921: | 5'- CTGTAAGTGGCAGCCGAAGC |
| SEQ ID No. 922: | 5'- CCCATCTGTAAGTGGCAGCC |
| SEQ ID No. 923: | 5'- TGTAAGTGGCAGCCGAAGCC |
| SEQ ID No. 924: | 5'- CATCTGTAAGTGGCAGCCGA |
| SEQ ID No. 925: | 5'- ATCTGTAAGTGGCAGCCGAA |
| SEQ ID No. 926: | 5'- CAGCCGAAGCCGCCTTTCCT |
| SEQ ID No. 927: | 5'- GGCAACAGAGTTTTACGACC |
| SEQ ID No. 928: | 5'- CCGGCAACAGAGTTTTACGA |
| SEQ ID No. 929: | 5'- TTCCCCGGCAACAGAGTTTT |
| SEQ ID No. 930: | 5'- CTTCCCCGGCAACAGAGTTT |
| SEQ ID No. 931: | 5'- TCCCCGGCAACAGAGTTTTA |
| SEQ ID No. 932: | 5'- CCGTCCGCCGCTAACCTTTT |

Die Sequenzen SEQ ID No. 843 bis SEQ ID No. 932 sind vor allem zum Nachweis von *Bacillus coagulans* geeignet.

f) Nukleinsäuresondenmoleküle, die spezifisch getränkeschädliche Alicyclobazillen nachweisen:

SEQ ID No. 933: 5'- CTTCCTCCGACTTACGCCGG
SEQ ID No. 934: 5'- CCTCCGACTTACGCCGGCAG
SEQ ID No. 935: 5'- TTCCTCCGACTTACGCCGGC
SEQ ID No. 936: 5'- TCCTCCGACTTACGCCGGCA
SEQ ID No. 937: 5'- TCCGACTTACGCCGGCAGTC
SEQ ID No. 938: 5'- CCGACTTACGCCGGCAGTCA
SEQ ID No. 939: 5'- GCCTTCCTCCGACTTACGCC
SEQ ID No. 940: 5'- CCTTCCTCCGACTTACGCCG
SEQ ID No. 941: 5'- GCTCTCCCCGAGCAACAGAG
SEQ ID No. 942: 5'- CTCTCCCCGAGCAACAGAGC
SEQ ID No. 943: 5'- CGCTCTCCCCGAGCAACAGA
SEQ ID No. 944: 5'- CTCCGACTTACGCCGGCAGT
SEQ ID No. 945: 5'- TCTCCCCGAGCAACAGAGCT
SEQ ID No. 946: 5'- CGACTTACGCCGGCAGTCAC
SEQ ID No. 947: 5'- TCGGCACTGGGGTGTGTCCC
SEQ ID No. 948: 5'- GGCACTGGGGTGTGTCCCCC
SEQ ID No. 949: 5'- CTGGGGTGTGTCCCCCCAAC
SEQ ID No. 950: 5'- CACTGGGGTGTGTCCCCCCA
SEQ ID No. 951: 5'- ACTGGGGTGTGTCCCCCCAA
SEQ ID No. 952: 5'- GCACTGGGGTGTGTCCCCCC
SEQ ID No. 953: 5'- TGGGGTGTGTCCCCCCAACA
SEQ ID No. 954: 5'- CACTCCAGACTTGCTCGACC
SEQ ID No. 955: 5'- TCACTCCAGACTTGCTCGAC
SEQ ID No. 956: 5'- CGGCACTGGGGTGTGTCCCC
SEQ ID No. 957: 5'- CGCCTTCCTCCGACTTACGC
SEQ ID No. 958: 5'- CTCCCCGAGCAACAGAGCTT
SEQ ID No. 959: 5'- ACTCCAGACTTGCTCGACCG
SEQ ID No. 960: 5'- CCCATGCCGCTCTCCCCGAG
SEQ ID No. 961: 5'- CCATGCCGCTCTCCCCGAGC
SEQ ID No. 962: 5'- CCCCATGCCGCTCTCCCCGA
SEQ ID No. 963: 5'- TCACTCGGTACCGTCTCGCA
SEQ ID No. 964: 5'- CATGCCGCTCTCCCCGAGCA
SEQ ID No. 965: 5'- ATGCCGCTCTCCCCGAGCAA
SEQ ID No. 966: 5'- TTCGGCACTGGGGTGTGTCC
SEQ ID No. 967: 5'- TGCCGCTCTCCCCGAGCAAC
SEQ ID No. 968: 5'- TTCACTCCAGACTTGCTCGA
SEQ ID No. 969: 5'- CCCGCAAGAAGATGCCTCCT
SEQ ID No. 970: 5'- AGAAGATGCCTCCTCGCGGG
SEQ ID No. 971: 5'- AAGAAGATGCCTCCTCGCGG
SEQ ID No. 972: 5'- CGCAAGAAGATGCCTCCTCG
SEQ ID No. 973: 5'- AAGATGCCTCCTCGCGGGCG
SEQ ID No. 974: 5'- CCGCAAGAAGATGCCTCCTC
SEQ ID No. 975: 5'- GAAGATGCCTCCTCGCGGGC
SEQ ID No. 976: 5'- CCCCGCAAGAAGATGCCTCC
SEQ ID No. 977: 5'- CAAGAAGATGCCTCCTCGCG
SEQ ID No. 978: 5'- TCCTTCGGCACTGGGGTGTG
SEQ ID No. 979: 5'- CCGCTCTCCCCGAGCAACAG
SEQ ID No. 980: 5'- TGCCTCCTCGCGGGCGTATC
SEQ ID No. 981: 5'- GACTTACGCCGGCAGTCACC
SEQ ID No. 982: 5'- GGCTCCTCTCTCAGCGGCCC
SEQ ID No. 983: 5'- CCTTCGGCACTGGGGTGTGT

(fortgesetzt)

| | |
|---|---|
| SEQ ID No. 984: | 5'- GGGGTGTGTCCCCCCAACAC |
| SEQ ID No. 985: | 5'- GCCGCTCTCCCCGAGCAACA |
| SEQ ID No. 986: | 5'- AGATGCCTCCTCGCGGGCGT |
| SEQ ID No. 987: | 5'- CACTCGGTACCGTCTCGCAT |
| SEQ ID No. 988: | 5'- CTCACTCGGTACCGTCTCGC |
| SEQ ID No. 989: | 5'- GCAAGAAGATGCCTCCTCGC |
| SEQ ID No. 990: | 5'- CTCCAGACTTGCTCGACCGC |
| SEQ ID No. 991: | 5'- TTACGCCGGCAGTCACCTGT |
| SEQ ID No. 992: | 5'- CTTCGGCACTGGGGTGTGTC |
| SEQ ID No. 993: | 5'- CTCGCGGGCGTATCCGGCAT |
| SEQ ID No. 994: | 5'- GCCTCCTCGCGGGCGTATCC |
| SEQ ID No. 995: | 5'- ACTCGGTACCGTCTCGCATG |
| SEQ ID No. 996: | 5'- GATGCCTCCTCGCGGGCGTA |
| SEQ ID No. 997: | 5'- GGGTGTGTCCCCCCAACACC |
| SEQ ID No. 998: | 5'- ACTTACGCCGGCAGTCACCT |
| SEQ ID No. 999: | 5'- CTTACGCCGGCAGTCACCTG |
| SEQ ID No. 1000: | 5'- ATGCCTCCTCGCGGGCGTAT |
| SEQ ID No. 1001: | 5'- GCGCCGCGGGCTCCTCTCTC |
| SEQ ID No. 1002: | 5'- GGTGTGTCCCCCCAACACCT |
| SEQ ID No. 1003: | 5'- GTGTGTCCCCCCAACACCTA |
| SEQ ID No. 1004: | 5'- CCTCGCGGGCGTATCCGGCA |
| SEQ ID No. 1005: | 5'- CCTCACTCGGTACCGTCTCG |
| SEQ ID No. 1006: | 5'- TCCTCACTCGGTACCGTCTC |
| SEQ ID No. 1007: | 5'- TCGCGGGCGTATCCGGCATT |
| SEQ ID No. 1008: | 5'- TTTCACTCCAGACTTGCTCG |
| SEQ ID No. 1009: | 5'- TACGCCGGCAGTCACCTGTG |
| SEQ ID No. 1010: | 5'- TCCAGACTTGCTCGACCGCC |
| SEQ ID No. 1011: | 5'- CTCGGTACCGTCTCGCATGG |
| SEQ ID No. 1012: | 5'- CGCGGGCGTATCCGGCATTA |
| SEQ ID No. 1013: | 5'- GCGTATCCGGCATTAGCGCC |
| SEQ ID No. 1014: | 5'- GGGCTCCTCTCTCAGCGGCC |
| SEQ ID No. 1015: | 5'- TCCCCGAGCAACAGAGCTTT |
| SEQ ID No. 1016: | 5'- CCCCGAGCAACAGAGCTTTA |
| SEQ ID No. 1017: | 5'- CCGAGCAACAGAGCTTTACA |
| SEQ ID No. 1018: | 5'- CCATCCCATGGTTGAGCCAT |
| SEQ ID No. 1019: | 5'- GTGTCCCCCCAACACCTAGC |
| SEQ ID No. 1020: | 5'- GCGGGCGTATCCGGCATTAG |
| SEQ ID No. 1021: | 5'- CGAGCGGCTTTTTGGGTTTC |
| SEQ ID No. 1022: | 5'- CTTTCACTCCAGACTTGCTC |
| SEQ ID No. 1023: | 5'- TTCCTTCGGCACTGGGGTGT |
| SEQ ID No. 1024: | 5'- CCGCCTTCCTCCGACTTACG |
| SEQ ID No. 1025: | 5'- CCCGCCTTCCTCCGACTTAC |
| SEQ ID No. 1026: | 5'- CCTCCTCGCGGGCGTATCCG |
| SEQ ID No. 1027: | 5'- TCCTCGCGGGCGTATCCGGC |
| SEQ ID No. 1028: | 5'- CATTAGCGCCCGTTTCCGGG |
| SEQ ID No. 1029: | 5'- GCATTAGCGCCCGTTTCCGG |
| SEQ ID No. 1030: | 5'- GGCATTAGCGCCCGTTTCCG |
| SEQ ID No. 1031: | 5'- GTCTCGCATGGGGCTTTCCA |
| SEQ ID No. 1032: | 5'- GCCATGGACTTTCACTCCAG |
| SEQ ID No. 1033: | 5'- CATGGACTTTCACTCCAGAC |

Die Sequenzen SEQ ID No. 933 bis SEQ ID No. 1033 sind vor allem zum Nachweis von Bakterien der Gattung Alicyclobacillus geeignet.

SEQ ID No. 1034: 5'- CCTTCCTCCGGCTTACGCCGGC
SEQ ID No. 1035: 5'- CCTTCCTCCGACTTGCGCCGGC
SEQ ID No. 1036: 5'- CCTTCCTCCGACTTTCACCGGC

Die Nukleinsäuresondenmoleküle gemäß SEQ ID No. 1034 bis SEQ ID No. 1036 werden als unmarkierte Kompetitorsonden für den Nachweis von Bakterien der Gattung Alicyclobacillus gemeinsam mit der Oligonukleotidsonde gemäß SEQ ID No. 933 eingesetzt, um das Binden der markierten, für Bakterien der Gattung Alicyclobacillus spezifischen Oligonukleotidsonde an Nukleinsäuresequenzen, die nicht spezifisch für Bakterien der Gattung Alicyclobacillus sind, zu verhindern.

SEQ ID No. 1037: 5'- ACCGTCTCACAAGGAGCTTT
SEQ ID No. 1038: 5'- TACCGTCTCACAAGGAGCTT
SEQ ID No. 1039: 5'- GTACCGTCTCACAAGGAGCT
SEQ ID No. 1040: 5'- GCCTACCCGTGTATTATCCG
SEQ ID No. 1041: 5'- CCGTCTCACAAGGAGCTTTC
SEQ ID No. 1042: 5'- CTACCCGTGTATTATCCGGC
SEQ ID No. 1043: 5'- GGTACCGTCTCACAAGGAGC
SEQ ID No. 1044: 5'- CGTCTCACAAGGAGCTTTCC
SEQ ID No. 1045: 5'- TCTCACAAGGAGCTTTCCAC
SEQ ID No. 1046: 5'- TACCCGTGTATTATCCGGCA
SEQ ID No. 1047: 5'- GTCTCACAAGGAGCTTTCCA
SEQ ID No. 1048: 5'- ACCCGTGTATTATCCGGCAT
SEQ ID No. 1049: 5'- CTCGGTACCGTCTCACAAGG
SEQ ID No. 1050: 5'- CGGTACCGTCTCACAAGGAG
SEQ ID No. 1051: 5'- ACTCGGTACCGTCTCACAAG
SEQ ID No. 1052: 5'- CGGCTGGCTCCATAACGGTT
SEQ ID No. 1053: 5'- ACAAGTAGATGCCTACCCGT
SEQ ID No. 1054: 5'- TGGCTCCATAACGGTTACCT
SEQ ID No. 1055: 5'- CAAGTAGATGCCTACCCGTG
SEQ ID No. 1056: 5'- CACAAGTAGATGCCTACCCG
SEQ ID No. 1057: 5'- GGCTCCATAACGGTTACCTC
SEQ ID No. 1058: 5'- ACACAAGTAGATGCCTACCC
SEQ ID No. 1059: 5'- CTGGCTCCATAACGGTTACC
SEQ ID No. 1060: 5'- GCTGGCTCCATAACGGTTAC
SEQ ID No. 1061: 5'- GGCTGGCTCCATAACGGTTA
SEQ ID No. 1062: 5'- GCTCCATAACGGTTACCTCA
SEQ ID No. 1063: 5'- AAGTAGATGCCTACCCGTGT
SEQ ID No. 1064: 5'- CTCCATAACGGTTACCTCAC
SEQ ID No. 1065: 5'- TGCCTACCCGTGTATTATCC
SEQ ID No. 1066: 5'- TCGGTACCGTCTCACAAGGA
SEQ ID No. 1067: 5'- CTCACAAGGAGCTTTCCACT
SEQ ID No. 1068: 5'- GTAGATGCCTACCCGTGTAT
SEQ ID No. 1069: 5'- CCTACCCGTGTATTATCCGG
SEQ ID No. 1070: 5'- CACTCGGTACCGTCTCACAA
SEQ ID No. 1071: 5'- CTCAGCGATGCAGTTGCATC
SEQ ID No. 1072: 5'- AGTAGATGCCTACCCGTGTA
SEQ ID No. 1073: 5'- GCGGCTGGCTCCATAACGGT
SEQ ID No. 1074: 5'-CCAAAGCAATCCCAAGGTTG
SEQ ID No. 1075: 5'- TCCATAACGGTTACCTCACC
SEQ ID No. 1076: 5'- CCCGTGTATTATCCGGCATT

(fortgesetzt)

| | |
|---|---|
| SEQ ID No. 1077: | 5'- TCTCAGCGATGCAGTTGCAT |
| SEQ ID No. 1078: | 5'- CCATAACGGTTACCTCACCG |
| SEQ ID No. 1079: | 5'- TCAGCGATGCAGTTGCATCT |
| SEQ ID No. 1080: | 5'- GGCGGCTGGCTCCATAACGG |
| SEQ ID No. 1081: | 5'- AAGCAATCCCAAGGTTGAGC |
| SEQ ID No. 1082: | 5'- TCACTCGGTACCGTCTGACA |
| SEQ ID No. 1083: | 5'- CCGAGTGTTATTCCAGTCTG |
| SEQ ID No. 1084: | 5'- CACAAGGAGCTTTCCACTCT |
| SEQ ID No. 1085: | 5'- ACAAGGAGCTTTCCACTCTC |
| SEQ ID No. 1086: | 5'- TCACAAGGAGCTTTCCACTC |
| SEQ ID No. 1087: | 5'- CAGCGATGCAGTTGCATCTT |
| SEQ ID No. 1088: | 5'- CAAGGAGCTTTCCACTCTCC |
| SEQ ID No. 1089: | 5'- CCAGTCTGAAAGGCAGATTG |
| SEQ ID No. 1090: | 5'- CAGTCTGAAAGGCAGATTGC |
| SEQ ID No. 1091: | 5'- CGGCGGCTGGCTCCATAACG |
| SEQ ID No. 1092: | 5'- CCTCTCTCAGCGATGCAGTT |
| SEQ ID No. 1093: | 5'- CTCTCTCAGCGATGCAGTTG |
| SEQ ID No. 1094: | 5'- TCTCTCAGCGATGCAGTTGC |
| SEQ ID No. 1095: | 5'- CTCTCAGCGATGCAGTTGCA |
| SEQ ID No. 1096: | 5'- CAATCCCAAGGTTGAGCCTT |
| SEQ ID No. 1097: | 5'- AATCCCAAGGTTGAGCCTTG |
| SEQ ID No. 1098: | 5'- AGCAATCCCAAGGTTGAGCC |
| SEQ ID No. 1099: | 5'- CTCACTCGGTACCGTCTCAC |
| SEQ ID No. 1100: | 5'- GCAATCCCAAGGTTGAGCCT |
| SEQ ID No. 1101: | 5'- GCCTTGGACTTTCACTTCAG |
| SEQ ID No. 1102: | 5'- CATAACGGTTACCTCACCGA |
| SEQ ID No. 1103: | 5'- CTCCTCTCTCAGCGATGCAG |
| SEQ ID No. 1104: | 5'- TCGGCGGCTGGCTCCATAAC |
| SEQ ID No. 1105: | 5'- AGTCTGAAAGGCAGATTGCC |
| SEQ ID No. 1106: | 5'- TCCTCTCTCAGCGATGCAGT |
| SEQ ID No. 1107: | 5'- CCCAAGGTTGAGCCTTGGAC |
| SEQ ID No. 1108: | 5'- ATAACGGTTACCTCACCGAC |
| SEQ ID No. 1109: | 5'- TCCCAAGGTTGAGCCTTGGA |
| SEQ ID No. 1110: | 5'- ATTATCCGGCATTAGCACCC |
| SEQ ID No. 1111: | 5'- CTACGTGCTGGTAACACAGA |
| SEQ ID No. 1112: | 5'- GCCGCTAGCCCCGAAGGGCT |
| SEQ ID No. 1113: | 5'- CTAGCCCCGAAGGGCTCGCT |
| SEQ ID No. 1114: | 5'- CGCTAGCCCCGAAGGGCTCG |
| SEQ ID No. 1115: | 5'- AGCCCCGAAGGGCTCGCTCG |
| SEQ ID No. 1116: | 5'- CCGCTAGCCCCGAAGGGCTC |
| SEQ ID No. 1117: | 5'- TAGCCCCGAAGGGCTCGCTC |
| SEQ ID No. 1118: | 5'- GCTAGCCCCGAAGGGCTCGC |
| SEQ ID No. 1119: | 5'- GCCCCGAAGGGCTCGCTCGA |
| SEQ ID No. 1120: | 5'- ATCCCAAGGTTGAGCCTTGG |
| SEQ ID No. 1121: | 5'- GAGCCTTGGACTTTCACTTC |
| SEQ ID No. 1122: | 5'- CAAGGTTGAGCCTTGGACTT |
| SEQ ID No. 1123: | 5'- GAGCTTTCCACTCTCCTTGT |
| SEQ ID No. 1124: | 5'- CCAAGGTTGAGCCTTGGACT |
| SEQ ID No. 1125: | 5'- CGGGCTCCTCTCTCAGCGAT |
| SEQ ID No. 1126: | 5'- GGAGCTTTCCACTCTCCTTG |
| SEQ ID No. 1127: | 5'- GGGCTCCTCTCTCAGCGATG |

(fortgesetzt)

SEQ ID No. 1128: 5'- TCTCCTTGTCGCTCTCCCCG
SEQ ID No. 1129: 5'- TCCTTGTCGCTCTCCCCGAG
SEQ ID No. 1130: 5'- AGCTTTCCACTCTCCTTGTC
SEQ ID No. 1131: 5'- CCACTCTCCTTGTCGCTCTC
SEQ ID No. 1132: 5'- GGCTCCTCTCTCAGCGATGC
SEQ ID No. 1133: 5'- CCTTGTCGCTCTCCCCGAGC
SEQ ID No. 1134: 5'- CACTCTCCTTGTCGCTCTCC
SEQ ID No. 1135: 5'- ACTCTCCTTGTCGCTCTCCC
SEQ ID No. 1136: 5'- CTCTCCTTGTCGCTCTCCCC
SEQ ID No. 1137: 5'- GCGGGCTCCTCTCTCAGCGA
SEQ ID No. 1138: 5'- GGCTCCATCATGGTTACCTC

Die Sequenzen SEQ ID No. 1037 bis SEQ ID No. 1138 sind vor allem zum Nachweis von *Alicyclobacillus acidoterrestris* geeignet.

SEQ ID No. 1139: 5'- CCGTCTCCTAAGGAGCTTTCCA

Das Nukleinsäuresondenmolekül gemäß SEQ ID No. 1139 wird als unmarkierte Kompetitorsonde für den Nachweis von *Alicyclobacillus acidoterrestris* gemeinsam mit der Oligonukleotidsonde gemäß SEQ ID No. 1044 eingesetzt, um das Binden der markierten, für *Alicyclobacillus acidoterrestris* spezifischen Oligonukleotidsonde an Nukleinsäuresequenzen, die nicht spezifisch für *Alicyclobacillus acidoterrestris* sind, zu verhindern.

SEQ ID No. 1140: 5'- TCCCTCCTTAACGGTTACCTCA
SEQ ID No. 1141: 5'- TGGCTCCATAA(A/T)GGTTACCTCA

Die Nukleinsäuresondenmoleküle gemäß SEQ ID No. 1140 bis SEQ ID No. 1141 werden als unmarkierte Kompetitorsonden für den Nachweis von *Alicyclobacillus acidoterrestris* gemeinsam mit der Oligonukleotidsonde gemäß SEQ ID No. 1057 eingesetzt, um das Binden der markierten, für *Alicyclobacillus acidoterrestris* spezifischen Oligonukleotidsonde an Nukleinsäuresequenzen, die nicht spezifisch für *Alicyclobacillus acidoterrestris* sind, zu verhindern.

SEQ ID No. 1142: 5'- CTTCCTCCGGCTTGCGCCGG
SEQ ID No. 1143: 5'- CGCTCTTCCCGA(G/T)TGACTGA
SEQ ID No. 1144: 5'- CCTCGGGCTCCTCCATC(A/T)GC

Die Sequenzen SEQ ID No. 1142 bis SEQ ID No. 1144 sind vor allem zum gleichzeitigen Nachweis von *Alicyclobacillus cycloheptanicus* und *A. herbarius* geeignet.

Ebenfalls beschrieben sind auch Abwandlungen der obigen Oligonukleotidsequenzen, die trotz der Abweichungen in der Sequenz und/oder Länge eine spezifische Hybridisierung mit Ziel-Nukleinsäuresequenzen des jeweiligen Mikroorganismus zeigen und sich dadurch für den Einsatz des Verfahrens eignen und einen spezifischen Nachweis des jeweiligen Mikroorganismus gewährleisten. Hierunter fallen insbesondere

a) Nukleinsäuremoleküle, die (i) mit einer der obigen Oligonukleotidsequenzen (SEQ ID No. 1, 5 bis 146, 148 bis 154, 157 bis 160, 163 bis 1033, 1037 bis 1138, 1142 bis 1144) in mindestens 80 %, bevorzugt in mindestens 90 % und besonders bevorzugt in mindestens 92 %, 94 %, 96 % der Basen übereinstimmen, oder die (ii) sich von obigen Oligonukleotidsequenzen durch eine oder mehrere Deletionen und/oder Additionen unterscheiden und eine spezifische Hybridisierung mit Nukleinsäuresequenzen von getränkeschädlichen Hefen der Gattungen Zygosaccharomyces, Hanseniaspora, Candida, Brettanomyces, Dekkera, Pichia, Saccharomyces und Saccharomycodes, insbesondere der Spezies *Zygosaccharomyces bailii, Z. mellis, Z. rouxii*, *Z. bisporus, Z. fermentati*, *Z. microellipsoides, Hanseniaspora uvarum, Candida intermedia, C. crusei (Issatchenkia orientalis), C. parapsilosis, Brettanomyces bruxellensis, B. naardenensis, Dekkera anomala, Pichia membranaefaciens, P. minuta, P. anomala, Saccharomyces exiguus, S. cerevisiae, Saccharomycodes ludwigii* oder von getränkeschädlichen Schimmelpilzen der Gattungen Mucor, Byssochlamys, Neosartorya, Aspergillus und Talaromyces, insbesondere der Spezies *Mucor racemosus, Byssochlamys nivea, Neosartorya fischeri, Aspergillus fumigatus und A. fischeri, Talaromyces flavus, T.*

*bacillisporus* und *T. flavus* oder von getränkeschädlichen Bakterien der Gattungen Lactobacillus, Leuconostoc, Oenococcus, Weissella, Lactococcus, Acetobacter, Gluconobacter, Gluconoacetobacter, Bacillus und Alicyclobacillus, insbesondere der Spezies *Lactobacillus collinoides, Leuconostoc mesenteroides, L. pseudomesenteroides, Oenococcus oeni, Bacillus coagulans, Alicyclobacillus ssp., A. acidoterrestris, A. cycloheptanicus* und *A. herbarius* ermöglichen. Dabei bedeutet "spezifische Hybridisierung", dass unter den hier beschriebenen oder dem Durchschnittsfachmann im Zusammenhang mit in situ-Hybridisierungstechniken bekannten stringenten Hybridisierungsbedingungen nur die ribosomale RNA der Ziel-Organismen, nicht aber die rRNA von Nicht-Ziel-Organismen an das Oligonukleotid bindet.

b) Nukleinsäuremoleküle, die mit einer zu den unter a) genannten Nukleinsäuremolekülen oder einer zu den Sonden SEQ ID No. 1, 5 bis 146, 148 bis 154, 157 bis 160, 163 bis 1033, 1037 bis 1138, 1142 bis 1144 komplementären Sequenz unter stringenten Bedingungen (s.u.) hybridisieren.

c) Nukleinsäuremoleküle, die eine Oligonukleotidsequenz von SEQ ID No. 1, 5 bis 146, 148 bis 154, 157 bis 160, 163 bis 1033, 1037 bis 1138, 1142 bis 1144 oder die Sequenz eines Nukleinsäuremoleküls nach a) oder b) umfassen und zusätzlich zu den genannten Sequenzen bzw. deren Abwandlungen nach a) oder b) mindestens ein weiteres Nukleotid aufweisen und eine spezifische Hybridisierung mit Nukleinsäuresequenzen von Ziel-Organismen ermöglichen.

Ebenso beschrieben sind Abwandlungen der obigen Kompetitorsondensequenzen, die trotz der Abweichungen in der Sequenz und/oder Länge eine spezifische Hybridisierung mit Nukleinsäuresequenzen von nicht nachzuweisenden Mikroorganismengattungen bzw. -spezies gewährleisten und dadurch das Binden der Oligonukleotidsonde an die Nukleinsäuresequenzen der nicht nachzuweisenden Mikroorganismengattungen bzw. -spezies verhindern. Sie eignen sich für den Einsatz des Verfahrens und gewährleisten einen spezifischen Nachweis des jeweiligen Mikroorganismus. Hierunter fallen insbesondere

a) Nukleinsäuremoleküle, die (i) mit einer der obigen Oligonukleotidsequenzen (SEQ ID No. 2 bis 4, 147, 155 bis 156, 161 bis 162, 1034 bis 1036, 1139 bis 1141) in mindestens 80 %, bevorzugt in mindestens 90 % und besonders bevorzugt in mindestens 92 %, 94 %, 96 % der Basen übereinstimmen, oder die (ii) sich von obigen Oligonukleotidsequenzen durch eine oder mehrere Deletionen und/oder Additionen unterscheiden und das Binden einer spezifischen Oligonukleotidsonde an die Nukleinsäuresequenz eines nicht nachzuweisenden Mikroorganismus verhindern.

b) Nukleinsäuremoleküle, die mit einer zu den unter a) genannten Nukleinsäuremolekülen oder einer zu den Sonden SEQ ID No. 2 bis 4, 147, 155 bis 156, 161 bis 162, 1034 bis 1036, 1139 bis 1141 komplementären Sequenz unter stringenten Bedingungen (s.u.) hybridisieren.

c) Nukleinsäuremoleküle, die eine Oligonukleotidsequenz von SEQ ID No. 2 bis 4, 147, 155 bis 156,161 bis 162,1034 bis 1036, 1139 bis 1141 oder die Sequenz eines Nukleinsäuremoleküls nach a) oder b) umfassen und zusätzlich zu den genannten Sequenzen bzw. deren Abwandlungen nach a) oder b) mindestens ein weiteres Nukleotid aufweisen und das Binden einer spezifischen Oligonukleotidsonde an die Nukleinsäuresequenz eines nicht nachzuweisenden Mikroorganismus verhindern.

Der Grad der Sequenzidentität eines Nukleinsäuresondenmoleküls mit den Oligonukleotidsonden mit der SEQ ID No. 1 bis SEQ ID No. 1144 kann mit üblichen Algorithmen bestimmt werden. Geeignet ist hierzu beispielsweise das Programm zur Bestimmung der Sequenzidentität, das unter http://www.ncbi.nlm.nih.gov/BLAST (auf dieser Seite z.B. der Link "Standard nucleotide-nucleotide BLAST [blastn]") zugänglich ist.

"Hybridisieren" kann im Rahmen dieser Erfindung gleichbedeutend sein mit "komplementär". Beschrieben sind auch solche Oligonukleotide umfasst, die mit dem (theoretischen) Gegenstrang eines beschriebenen Oligonukleotids, einschließlich der beschriebenen Abwandlungen der SEQ ID No. 1 bis SEQ ID No. 1144, hybridisieren.

Der Begriff "stringente Bedingungen" steht allgemein für Bedingungen, unter denen eine Nukleinsäuresequenz präferenziell an ihre Zielsequenz hybridisieren wird, und zu einem deutlich geringeren Ausmaß oder gar nicht an andere Sequenzen. Stringente Bedingungen sind z.T. Sequenz-abhängig und werden unter verschiedenen Umständen unterschiedlich sein. Längere Sequenzen hybridisieren spezifisch bei höheren Temperaturen. Im Allgemeinen werden stringente Bedingungen so ausgewählt, dass die Temperatur etwa 5°C unter dem thermischen Schmelzpunkt ($T_m$) für die spezifische Sequenz bei einer definierten Ionenstärke und einem definierten pH liegt. Die $T_m$ ist die Temperatur (unter definierter Ionenstärke, pH und Nukleinsäurekonzentration), bei der 50 % der zu der Zielsequenz komplementären Moleküle zu der Zielsequenz im Gleichgewichtszustand hybridisieren.

Die Nukleinsäuresondenmoleküle können im Rahmen des Nachweisverfahrens mit verschiedenen Hybridisierungslösungen eingesetzt werden. Verschiedene organische Lösungsmittel können hierbei in Konzentrationen von 0 % bis 80 % eingesetzt werden. Durch das Einhalten von stringenten Hybridisierungsbedingungen wird gewährleistet, dass das Nukleinsäuresondenmolekül auch tatsächlich mit der Zielsequenz hybridisiert. Moderate Bedingungen im Sinne der Erfindung sind z.B. 0 % Formamid in einem Hybridisierungspuffer wie er nachfolgend beschrieben ist. Stringente Bedingungen im Sinne der Erfindung sind beispielsweise 20 % bis 80 % Formamid im Hybridisierungspuffer.

Im Rahmen des erfindungsgemäßen Verfahrens zum spezifischen Nachweis von Hefen der Gattung Zygosaccharomyces, enhält eine typische Hybridisierungslösung 0 % bis 80 % Formamid, bevorzugt 20 % bis 60 % Formamid, besonders bevorzugt 40 % Formamid. Sie hat außerdem eine Salzkonzentration von 0,1 Mol/l bis 1,5 Mol/l, bevorzugt von 0,7 Mol/l bis 1,0 Mol/l, besonders bevorzugt von 0,9 Mol/l, wobei es sich bei dem Salz vorzugsweise um Natriumchlorid handelt. Weiter umfasst die Hybridisierungslösung üblicherweise ein Detergens, wie z.B. Natriumdodecylsulfat (SDS), in einer Konzentration von 0,001 % bis 0,2 %, vorzugsweise in einer Konzentration von 0,005 % bis 0,05 %, besonders bevorzugt in einer Konzentration von 0,01 %. Zum Puffern der Hybridisierungslösung können verschiedene Verbindungen wie Tris-HCl, Natrium-Citrat, PIPES oder HEPES verwendet werden, die üblicherweise in Konzentrationen von 0,01 Mol/l bis 0,1 Mol/l eingesetzt werden, bevorzugt von 0,01 Mol/l bis 0,05 Mol/l, in einem pH-Wert-Bereich von 6,0 bis 9,0, bevorzugt 7,0 bis 8,0. Die besonders bevorzugte erfindungsgemäße Ausführung der Hybridisierungslösung beinhaltet 0,02 Mol/l Tris-HCl, pH 8,0.

Im Rahmen der ebenfalls beschriebenen Verfahren zum spezifischen Nachweis von Schimmelpilzen der Gattungen Mucor, Byssochlamys, Neosartorya, Aspergillus und Talaromyces, insbesondere der Spezies *Mucor racemosus, Byssochlamys nivea, Neosartorya fischeri, Aspergillus fumigatus und A. fischeri, Talaromyces flavus, T. bacillisporus* und *T. flavus* enthält eine typische Hybridisierungslösung 0 % bis 80 % Formamid, bevorzugt 10 % bis 60 % Formamid, besonders bevorzugt 20 % Formamid. Sie hat außerdem eine Salzkonzentration von 0,1 Mol/l bis 1,5 Mol/l, bevorzugt von 0,7 Mol/l bis 1,0 Mol/l, besonders bevorzugt von 0,9 Mol/l, wobei es sich bei dem Salz vorzugsweise um Natriumchlorid handelt. Weiter umfasst die Hybridisierungslösung üblicherweise ein Detergens, wie z.B. Natriumdodecylsulfat (SDS), in einer Konzentration von 0,001 % bis 0,2 %, vorzugsweise in einer Konzentration von 0,005 % bis 0,05 %, besonders bevorzugt in einer Konzentration von 0,01 %. Zum Puffern der Hybridisierungslösung können verschiedene Verbindungen wie Tris-HCl, Natrium-Citrat, PIPES oder HEPES verwendet werden, die üblicherweise in Konzentrationen von 0,01 Mol/l bis 0,1 Mol/l eingesetzt werden, bevorzugt von 0,01 Mol/l bis 0,05 Mol/l, in einem pH-Wert-Bereich von 6,0 bis 9,0, bevorzugt 7,0 bis 8,0. Die besonders bevorzugte Ausführung der Hybridisierungslösung beinhaltet 0,02 Mol/l Tris-HCl, pH 8,0.

Im Rahmen des beschriebenen Verfahrens zum spezifischen Nachweis von Bakterien der Gattungen Lactobacillus, Leuconostoc, Oenococcus, Weissella, Lactococcus, Acetobacter, Gluconobacter, Gluconoacetobacter, Bacillus und Alicyclobacillus, insbesondere der Spezies *Lactobacillus collinoides, Leuconostoc mesenteroides, L. pseudomesenteroides, Oenococcus oeni, Bacillus coagulans, Alicyclobacillus ssp., A. acidoterrestris, A. cycloheptanicus* und *A. herbarius* enthält eine typische Hybridisierungslösung 0 % bis 80 % Formamid, bevorzugt 10 % bis 60 % Formamid, besonders bevorzugt 20 % Formamid. Sie hat außerdem eine Salzkonzentration von 0,1 Mol/l bis 1,5 Mol/l, bevorzugt von 0,7 Mol/l bis 1,0 Mol/l, besonders bevorzugt von 0,9 Mol/l, wobei es sich bei dem Salz vorzugsweise um Natriumchlorid handelt. Weiter umfasst die Hybridisierungslösung üblicherweise ein Detergens, wie z.B. Natriumdodecylsulfat (SDS), in einer Konzentration von 0,001 % bis 0,2 %, vorzugsweise in einer Konzentration von 0,005 % bis 0,05 %, besonders bevorzugt in einer Konzentration von 0,01 %. Zum Puffern der Hybridisierungslösung können verschiedene Verbindungen wie Tris-HCl, Natrium-Citrat, PIPES oder HEPES verwendet werden, die üblicherweise in Konzentrationen von 0,01 Mol/l bis 0,1 Mol/l eingesetzt werden, bevorzugt von 0,01 Mol/l bis 0,05 Mol/1, in einem pH-Wert-Bereich von 6,0 bis 9,0, bevorzugt 7,0 bis 8,0. Die besonders bevorzugte Ausführung der Hybridisierungslösung beinhaltet 0,02 Mol/l Tris-HCl, pH 8,0.

Es versteht sich, dass der Fachmann die angegebenen Konzentrationen der Bestandteile des Hybridisierungspuffers derart auswählen kann, dass die gewünschte Stringenz der Hybridisierungsreaktion erzielt wird. Besonders bevorzugte Ausführungsformen geben stringente bis besonders stringente Hybridisierungsbedingungen wieder. Unter Einsatz dieser stringenten Bedingungen kann der Fachmann feststellen, ob ein bestimmtes Nukleinsäuremolekül einen spezifischen Nachweis von Nukleinsäuresequenzen von Ziel-Organismen ermöglicht und somit im Rahmen der Erfindung zuverlässig eingesetzt werden kann.

Die Konzentration der Nukleinsäuresonde im Hybridisierungspuffer ist abhängig von der Art ihrer Markierung und der Anzahl der Zielstrukturen. Um eine schnelle und effiziente Hybridisierung zu ermöglichen, sollte die Anzahl der Nukleinsäuresondenmoleküle die Anzahl der Zielstrukturen um mehrere Größenordnungen überschreiten. Allerdings ist bei der Fluoreszenz in situ-Hybridisierung (FISH) darauf zu achten, dass eine zu hohe Menge an fluoreszenzmarkierten Nukleinsäuresondenmolekülen zu erhöhter Hintergrundfluoreszenz führt. Die Konzentration der Nukleinsäuresondenmoleküle sollte deshalb in einem Bereich zwischen 0,5 bis 500 ng/μl liegen. Die im Rahmen der erfindungsgemäßen Verfahren bevorzugte Konzentration beträgt 1 bis 10 ng jedes verwendeten Nukleinsäuresondenmoleküls pro μl Hybridisierungslösung. Das verwendete Volumen der Hybridisierungslösung sollte zwischen 8 μl und 100 ml liegen, bei einer besonders bevorzugten Ausführungsform der erfindungsgemäßen Verfahren beträgt es 30 μl.

Die Konzentration der Kompetitorsonde im Hybridisierungspuffer ist abhängig von der Anzahl der Zielstrukturen. Um eine schnelle und effiziente Hybridisierung zu ermöglichen, sollte die Anzahl der Kompetitorsondenmoleküle die Anzahl der Zielstrukturen um mehrere Größenordnungen überschreiten. Die Konzentration der Kompetitorsondenmoleküle sollte deshalb in einem Bereich zwischen 0,5 bis 500 ng/μl liegen. Die im Rahmen der erfindungsgemäßen Verfahren bevorzugte Konzentration beträgt 1 bis 10 ng jedes verwendeten Kompetitorsondenmoleküls pro μl Hybridisierungslösung. Das verwendete Volumen der Hybridisierungslösung sollte zwischen 8 μl und 100 ml liegen, bei einer besonders bevorzugten Ausführungsform der erfindungsgemäßen Verfahren beträgt es 30 μl.

Die Dauer der Hybridisierung beträgt üblicherweise zwischen 10 Minuten und 12 Stunden; bevorzugt erfolgt die Hybridisierung für etwa 1,5 Stunden. Die Hybridisierungstemperatur beträgt bevorzugt zwischen 44 °C und 48 °C, besonders bevorzugt 46 °C, wobei der Parameter der Hybridisierungstemperatur, wie auch die Konzentration an Salzen und Detergenzien in der Hybridisierungslösung in Abhängigkeit von den Nukleinsäuresonden, insbesondere deren Längen und dem Grad der Komplementarität zur Zielsequenz in der nachzuweisenden Zelle optimiert werden kann. Der Fachmann ist mit einschlägigen Berechnungen hierzu vertraut.

Nach erfolgter Hybridisierung sollten die nicht hybridisierten und überschüssigen Nukleinsäuresondenmoleküle entfernt bzw. abgewaschen werden, was üblicherweise mittels einer herkömmlichen Waschlösung erfolgt. Diese Waschlösung kann, falls gewünscht, 0,001 % bis 0,1 % eines Detergens wie SDS, bevorzugt 0,005 % bis 0,05 %, besonders bevorzugt 0,01 %, sowie Tris-HCl in einer Konzentration von 0,001 Mol/l bis 0,1 Mol/1, bevorzugt 0,01 Mol/l bis 0,05 Mol/l, besonders bevorzugt 0,02 Mol/l enthalten, wobei der pH-Wert von Tris-HCl im Bereich von 6,0 bis 9,0, vorzugsweise bei 7,0 bis 8,0, besonders bevorzugt bei 8,0 liegt. Ein Detergens kann enthalten sein, ist aber nicht zwingend erforderlich. Weiter enthält die Waschlösung üblicherweise NaCl, wobei die Konzentration je nach benötigter Stringenz von 0,003 Mol/l bis 0,9 Mol/l, bevorzugt von 0,01 Mol/l bis 0,9 Mol/l, beträgt. Des weiteren kann die Waschlösung EDTA enthalten, wobei die Konzentration vorzugsweise 0,005 Mol/l beträgt. Ferner kann die Waschlösung auch dem Fachmann geläufige Konservierungsmittel in geeigneten Mengen enthalten.

Allgemein kommen bei dem Waschschritt Pufferlösungen zum Einsatz, die prinzipiell sehr ähnlich aussehen können wie die Hybridisierungspuffer (gepufferte Natriumchloridlösung), nur dass der Waschschritt in der Regel in einem Puffer mit niedrigerer Salzkonzentration bzw. bei höherer Temperatur durchgeführt wird. Zur theoretischen Abschätzung der Hybridisierungsbedingungen kann folgende Formel verwendet werden:

$$Td = 81{,}5 + 16{,}6\ lg[Na+] + 0{,}4\ x\ (\%\ GC) - 820/n - 0{,}5\ x\ (\%\ FA)$$

| | |
|---|---|
| Td | = Dissoziationstemperatur in °C |
| [Na+] | = Molarität der Natriumionen |
| % GC | = Anteil der Guanin- und Cytosinnukleotide an der Anzahl der Basen |
| n | = Länge des Hybrids |
| %FA = | Formamidgehalt |

Mit Hilfe dieser Formel kann z.B. der Formamidanteil (der wegen der Toxizität des Formamids möglichst gering sein sollte) des Waschpuffers durch einen entsprechend niedrigeren Natriumchloridgehalt ersetzt werden. Allerdings ist dem Fachmann aus der umfangreichen Literatur zu in situ-Hybridisierungsmethoden bekannt, dass und auf welche Weise die genannten Bestandteile variiert werden können. Bezüglich der Stringenz der Hybridisierungsbedingungen gilt das oben im Zusammenhang mit dem Hybridisierungspuffer Gesagte.

Das "Abwaschen" der nicht gebundenen Nukleinsäuresondenmoleküle erfolgt üblicherweise bei einer Temperatur im Bereich von 44 °C bis 52 °C, bevorzugt von 44 °C bis 50°C und besonders bevorzugt bei 46 °C für eine Dauer von 10 bis 40 Minuten, vorzugsweise für 15 Minuten.

Die spezifisch hybridisierten Nukleinsäuresondenmoleküle können anschließend in den jeweiligen Zellen detektiert werden. Voraussetzung hierfür ist, dass das Nukleinsäuresondenmolekül nachweisbar ist, z.B. dadurch dass das Nukleinsäuresondenmolekül durch kovalente Bindung mit einem Marker verknüpft ist. Als detektierbare Marker werden z.B. fluoreszierende Gruppen wie z.B. CY2 (erhältlich von Amersham Life Sciences, Inc., Arlington Heights, USA), CY3 (ebenfalls erhältlich von Amersham Life Sciences), CY5 (ebenfalls zu beziehen von Amersham Life Sciences), FITC (Molecular Probes Inc., Eugene, USA), FLUOS (erhältlich von Roche Diagnostics GmbH, Mannheim, Deutschland), TRITC (erhältlich von Molecular Probes Inc. Eugene, USA), 6-FAM oder FLUOS-PRIME verwendet, die dem Fachmann alle wohlbekannt sind. Auch chemische Marker, radioaktive Marker oder enzymatische Marker wie Meerrettich-Peroxidase, saure Phosphatase, alkalische Phosphatase und Peroxidase können verwendet werden. Für jedes dieser Enzyme ist eine Reihe von Chromogenen bekannt, die anstelle des natürlichen Substrates umgesetzt werden können und entweder zu farbigen oder zu fluoreszierenden Produkten umgesetzt werden können. Beispiele für solche Chromogene sind in der nachfolgenden Tabelle angegeben:

**Tabelle**

| Enzyme | Chromogen |
|---|---|
| 1. Alkalische Phosphatase und saure Phosphatase | 4-Methylumbelliferylphosphat (*), Bis(4-Methyiumbelliferylphosphat), (*) 3-O-Methylfluoreszein, Flavon-3-Diphosphattriammoniumsalz (*), p-Nitrophenylphosphatdinatriumsalz |
| 2. Peroxidase | Tyraminhydrochlorid (*), 3-(p-Hydroxyphenyl)-Propionsäure (*), p-Hydroxy-phenethylalkohol(*), 2,2'-Azino-di-3-ethylbenzthiazolinsulfonsäure (ABTS), ortho-Phenylendiamindihydrochlorid, o-Dianisidin, 5-Aminosalicylsäure, p-Ucresol (*), 3,3'-dimethyloxybenzidin, 3-Methyl-2-benzothiazolinhydrazon, Tetramethylbenzidin |
| 3. Meerrettichperoxidase | $H_2O_2$ + Diammoniumbenzidin $H_2O_2$ + Tetramethylbenzidin |
| 4. β-D-Galaktosidase | o-Nitrophenyl-β-D-galaktopyranosid, 4-Methylumbelliferyl-β-D-galaktosid |
| 5. Glukoseoxidase | ABTS, Glukose und Thiazolylblau |
| *Fluoreszenz | |

Schließlich ist es möglich, die Nukleinsäuresondenmoleküle so zu gestalten, dass an ihrem 5'- oder 3'-Ende eine weitere zur Hybridisierung geeignete Nukleinsäuresequenz vorhanden ist. Diese Nukleinsäuresequenz umfasst wiederum ca. 15 bis 100, bevorzugt 15 bis 50 Nukleotide. Dieser zweite Nukleinsäurebereich kann wiederum von einem Nukleinsäuresondenmolekül erkannt werden, welches durch eines der oben erwähnten Mittel nachweisbar ist.

Eine weitere Möglichkeit besteht in der Kopplung der nachweisbaren Nukleinsäuresondenmoleküle mit einem Hapten, das anschließend mit einem das Hapten erkennenden Antikörper in Kontakt gebracht werden kann. Als Beispiel für solch ein Hapten kann Digoxigenin angeführt werden. Dem Fachmann sind über die angegebenen Beispiele hinaus noch weitere wohlbekannt.

Die abschließende Auswertung ist in Abhängigkeit von der Art der Markierung der verwendeten Sonde mit einem Lichtmikroskop, Epifluoreszenzmikroskop, Chemoluminometer, Fluorometer u.a. möglich.

Ein wichtiger Vorteil der beschriebenen Verfahren zum spezifischen Nachweis von getränkeschädlichen Hefen der Gattungen Zygosaccharomyces, Hanseniaspora, Candida, Brettanomyces, Dekkera, Pichia, Saccharomyces und Saccharomycodes, insbesondere der Spezies *Zygosaccharomyces bailii, Z. mellis, Z. rouxii, Z. bisporus, Z. fermentati, Z. microellipsoides, Hanseniaspora uvarum, Candida intermedia, C. crusei (Issatchenkia orientalis), C. parapsilosis, Brettanomyces bruxellensis, B. naardenensis, Dekkera anomala, Pichia membranaefaciens, P. minuta, P. anomala, Saccharomyces exiguus, S. cerevisiae, Saccharomycodes ludwigii* oder zum spezifischen Nachweis von getränkeschädlichen Schimmelpilzen der Gattungen Mucor, Byssochlamys, Neosartorya, Aspergillus und Talaromyces, insbesondere der Spezies *Mucor racemosus, Byssochlamys nivea, Neosartorya fischeri, Aspergillus fumigatus und A. fischeri, Talaromyces flavus, T. bacillisporus* und *T. flavus* oder zum spezifischen Nachweis von getränkeschädlichen Bakterien der Gattungen Lactobacillus, Leuconostoc, Oenococcus, Weissella, Lactococcus, Acetobacter, Gluconobacter, Gluconoacetobacter, Bacillus und Alicyclobacillus, insbesondere der Spezies *Lactobacillus collinoides, Leuconostoc mesenteroides, L. pseudomesenteroides, Oenococcus oeni, Bacillus coagulans, Alicyclobacillus ssp., A. acidoterrestris, A. cycloheptanicus* und *A. herbarius* gegenüber den weiter oben beschriebenen Nachweismethoden ist die außergewöhnliche Schnelligkeit. Im Vergleich zu herkömmlichen Kultivierungsverfahren, die bis zu zehn Tage benötigen, liegt das Ergebnis bei Anwendung des erfindungsgemäßen und der weiteren beschriebenen Verfahren innerhalb von 24 bis 48 Stunden vor.

Ein weiterer Vorteil liegt in der Befähigung, eine genaue Unterscheidung der nachzuweisenden, getränkerelevanten Mikroorganismen vorzunehmen. Mit bislang geläufigen Verfahren wurde beim Nachweis keine Differenzierung der Mikroorganismen bis auf Gattungs- und/oder Artebene vorgenommen, da die Differenzierung entweder gar nicht möglich oder zu zeitaufwendig war.

Ein weiterer Vorteil liegt in der Spezifität dieser Verfahren. Durch die verwendeten Nukleinsäuresondenmoleküle können hochspezifisch getränkeschädliche Hefen der Gattungen Zygosaccharomyces, Hanseniaspora, Candida, Brettanomyces, Dekkera, Pichia, Saccharomyces und Saccharomycodes, insbesondere der Spezies *Zygosaccharomyces bailii, Z. mellis, Z. rouxii, Z. bisporus, Z. fermentati, Z. microellipsoides, Hanseniaspora uvarum, Candida intermedia, C. crusei (Issatchenkia orientalis), C. parapsilosis, Brettanomyces bruxellensis, B. naardensis, Dekkera anomala, Pichia membranaefaciens, P. minuta, P. anomala, Saccharomyces exiguus, S. cerevisiae, Saccharomycodes ludwigii* oder getränkeschädliche Schimmelpilzen der Gattungen Mucor, Byssochlamys, Neosartorya, Aspergillus und Talaromyces, insbesondere der Spezies *Mucor racemosus, Byssochlamys nivea, Neosartorya fischeri, Aspergillus fumigatus*

*und A. fischeri, Talaromyces flavus, T. bacillisporus* und *T. flavus* oder getränkeschädliche Bakterien der Gattungen Lactobacillus, Leuconostoc, Oenococcus, Weissella, Lactococcus, Acetobacter, Gluconobacter, Gluconoacetobacter, Bacillus und Alicyclobacillus, insbesondere der Spezies *Lactobacillus collinoides, Leuconostoc mesenteroides, L. pseudomesenteroides, Oenococcus oeni, Bacillus coagulans, Alicyclobacillus ssp., A. acidoterrestris, A. cycloheptanicus* und *A. herbarius* nachgewiesen werden. Durch die Visualisierung der Mikroorganismen kann eine gleichzeitige visuelle Kontrolle stattfinden. Falsch positive Ergebnisse, wie sie häufig bei der Polymerase-Ketten-Reaktion auftreten, sind somit ausgeschlossen.

Ein weiterer Vorteil der erfindungsgemäßen Verfahren liegt in der leichten Handhabbarkeit. So können durch die Verfahren leicht große Mengen an Proben auf das Vorhandensein von Mikroorganismen der Gattung *Zygosaccharomyces* getestet werden.

Schließlich stellt die ebenfalls beschriebene Möglichkeit des gleichzeitigen Nachweises mehrerer der genannten Keime durch den Einsatz von entsprechenden Mischungen von Sonden einen wesentlichen Vorteil gegenüber dem Stand der Technik dar. Dadurch können alle in der Praxis relevanten getränkeschädlichen Mikroorganismen in wenigen Versuchsansätzen nachgewiesen werden.

Verschiedene Sonden können dabei mit unterschiedlichen Markierungen versehen sein, so dass die verschiedenen, nachgewiesenen Mikroorganismen auf einfache und zuverlässige Weise diskriminiert werden können. Z. B. kann ein erstes Oligonukleotid spezifisch mit einem grünen Fluoreszenzfarbstoff markiert werden und zum Nachweis einer ersten Mikroorganismengattung oder -art dienen. Ein zweites Oligonukleotid wird ebenfalls spezifisch, etwa mit einem roten Fluoreszenzfarbstoff, markiert und dient dem Nachweis einer zweiten Mikroorganismengattung oder -art. Die als Kompetitorsonden bezeichneten Oligonukleotide bleiben unmarkiert und verhindern das Binden des markierten ersten und/oder zweiten Oligonukleotids an Bakterien, die nicht zur nachzuweisenden Gattung oder Spezies gehören. Die verschiedenen Marker, z.B. ein grüner Fluoreszenzfarbstoff einerseits und ein roter Fluoreszenzfarbstoff andererseits, sind voneinander auf einfache Weise unterscheidbar, z.B. durch den Einsatz verschiedener Filter in der Fluoreszenzmikroskopie.

Die erfindungsgemäßen Verfahren können vielfältig angewendet werden.

So können beispielsweise alkoholfreie Getränke (z.B. Fruchtsäfte, Fruchtnektare, Fruchtkonzentrate, Fruchtpürees, Erfrischungsgetränke und Wässer) auf die Anwesenheit von Mikroorganismen der Gattung *Zygosaccharomyces* untersucht werden.

Auch können beispielsweise Umweltproben auf das Vorhandensein der nachzuweisenden Mikroorganismen der Gattung *Zygosaccharomyces* untersucht werden. Diese Proben können hierzu z.B. aus dem Boden entnommen oder auch Teile von Pflanzen sein.

Das erfindungsgemäße Verfahren kann weiter zur Untersuchung von Abwasserproben oder Silageproben eingesetzt werden.

Das erfindungsgemäße Verfahren kann weiter zur Untersuchung medizinischer Proben, z.B. von Stuhlproben, Blutkulturen, Sputum, Gewebeproben (auch Schnitte), Wundmaterial, Urin, Proben aus dem Respirationstrakt, Implantate und Katheteroberflächen eingesetzt werden.

Ein weiteres Anwendungsgebiet für das erfindungsgemäße Verfahren ist die Kontrolle von Lebensmitteln. In bevorzugten Ausführungsformen werden die Lebensmittelproben aus Milch oder Milchprodukten (Joghurt, Käse, Quark, Butter, Buttermilch), Trinkwasser, alkoholischen Getränken (z.B. Bier, Wein, Spirituosen), Backwaren oder Fleischwaren entnommen.

Ein weiteres Anwendungsgebiet für das erfindungsgemäße Verfahren ist die Untersuchung pharmazeutischer und kosmetischer Produkte, z.B. Salben, Cremes, Tinkturen, Säfte, Lösungen, Tropfen etc.

Erfindungsgemäß werden weiterhin Kits zur Durchführung der entsprechenden Verfahren zur Verfügung gestellt. Die in diesen Kits enthaltene Hybridisierungsanordnung ist z.B. in der deutschen Patentanmeldung 100 61 655.0 beschrieben.

Außer der beschriebenen Hybridisierungsanordnung (als VIT-Reaktor bezeichnet) umfassen die Kits als wichtigsten Bestandteil die jeweilige Hybridisierungslösung mit den weiter oben beschriebenen für die nachzuweisenden Mikroorganismen spezifischen Nukleinsäuresondenmolekülen (VIT-Lösung). Weiterhin ist jeweils enthalten der entsprechende Hybridisierungspuffer (Solution C) und ein Konzentrat der entsprechenden Waschlösung (Solution D). Weiterhin sind enthalten gegebenenfalls Fixierungslösungen (Solution A und Solution B) sowie gegebenenfalls eine Einbettlösung (Finisher). Gegebenenfalls sind Lösungen zur parallelen Durchführung einer Positivkontrolle (Positive Control) sowie einer Negativkontrolle (Negative Control) enthalten.

Das folgende Beispiel soll die Erfindung erläutern, ohne sie einzuschränken:

Beispiel

Spezifischer Schnellnachweis getränkeschädlicher Mikroorganismen in einer Probe

Eine Probe wird in geeigneter Weise 20 bis 48 h kultiviert. Zum Nachweis von Hefen und Schimmelpilzen kann die Kultivierung z.B. in SSL-Bouillon für 24 h bei 25 °C erfolgen. Zum Nachweis von Milchsäurebakterien kann die Kultivierung z.B. in MRS-Bouillon für 48 h bei 30 °C erfolgen. Zum Nachweis von Essigsäurebakterien kann die Kultivierung z.B. auf DSM-Agar für 48 h bei 28 °C erfolgen. Zum Nachweis von Bazillen, vornehmlich *B. coagulans* kann die Kultivierung z.B. auf Dextrose-Caseinpepton Agar für 48 h bei 55°C erfolgen.
Zum Nachweis von Alicyclobazillen kann die Kultivierung z.B. in BAM-Bouillon für 48 h bei 44 °C erfolgen.

Zu einem Aliquot der Kultur wird dasselbe Volumen Fixierungslösung (Solution B, Ethanol absolut) zugegeben. Alternativ kann auch ein Aliquot der Kultur zentrifugiert werden (4 000 g, 5 min, Raumtemperatur) und - nach Verwerfen des Überstandes - das Pellet in 4 Tropfen Fixierungslösung (Solution B) aufgenommen werden.

Zur Durchführung der Hybridisierung wird ein geeignetes Aliquot der fixierten Zellen (bevorzugt 5 µl) auf einen Objektträger aufgebracht und getrocknet (46 °C, 30 min oder bis vollständig trocken). Alternativ können die Zellen auch auf andere Trägermaterialien (z. B. eine Mikrotiterplatte oder einen Filter) aufgebracht werden. Anschließend werden die getrockneten Zellen vollständig dehydratisiert durch erneuten Zusatz der Fixierungslösung (Solution B). Der Objektträger wird erneut getrocknet (Raumtemperatur, 3 min oder bis vollständig trocken).

Anschließend wird auf die fixierten, dehydratisierten Zellen die Hybridisierungslösung (VIT-Lösung, Hybridisierungspuffer mir markierten Sondenmolekülen) mit den weiter oben beschriebenen für die nachzuweisenden Mikroorganismen spezifischen Nukleinsäuresondenmolekülen aufgebracht. Das bevorzugte Volumen beträgt 40 µl. Der Objektträger wird anschließend in einer mit Hybridisierungspuffer (Solution C) befeuchteten Kammer, bevorzugt dem VIT-Reaktor (siehe DE 100 61 655.0), inkubiert (46 °C, 90 min).

Anschließend wird der Objektträger aus der Kammer entnommen, die Kammer mit Waschlösung befüllt (Solution D, 1:10 verdünnt in destilliertem Wasser) und der Objektträger in dieser inkubiert (46 °C, 15 min).

Anschließend wird die Kammer mit destilliertem Wasser befüllt, der Objektträger kurz eingetaucht und anschließend in seitlicher Stellung luftgetrocknet (46 °C, 30 min oder bis vollständig trocken).

Anschließend wird der Objektträger in einem geeigneten Medium (Finisher) eingebettet.

Abschließend wird die Probe mit Hilfe eines Fluoreszenzmikroskops analysiert.

**Patentansprüche**

1. Verfahren zum Nachweis von getränkeschädlichen Mikroorganismen in einer Probe, wobei der Nachweis mittels mindestens einer Oligonukleotidsonde mit der Sequenz (in 5'->3'-Richtung):

   SEQ ID No. 1: 5'-GTTTGACCAGATTCTCCGCTC

   erfolgt.

2. Verfahren nach Anspruch 1, wobei getränkeschädliche Mikroorganismen der Gattung Zygosaccharomyces mittels der Oligonukleotidsonde SEQ ID No.1 nachgewiesen werden.

3. Verfahren nach Anspruch 2, **dadurch gekennzeichnet, dass** die Oligonukleotidsonde zusammen mit einer oder mehreren Kompetitorsonden verwendet wird.

4. Verfahren nach Anspruch 3, **dadurch gekennzeichnet, dass** die Oligonukleotidsonde SEQ ID No. 1 zusammen mit einer oder mehrerer Kompetitorsonden, ausgewählt aus der Gruppe bestehend aus SEQ ID No. 2 bis SEQ ID No. 4, verwendet wird.

5. Verfahren nach Anspruch 1 bis 4, **dadurch gekennzeichnet, dass** es die folgenden Schritte umfasst:

   a) Kultivieren der in der Probe enthaltenen getränkeschädlichen Mikroorganismen,
   b) Fixieren der in der Probe enthaltenen getränkeschädlichen Mikroorganismen,
   c) Inkubieren der fixierten Mikroorganismen mit mindestens der Oligonukleotidsonde, ggf. zusammen mit einer Kompetitorsonde,
   d) Entfernen nicht hybridisierter Oligonukleotidsonden,
   e) Detektieren und Visualisieren sowie ggf. Quantifizieren der getränkeschädlichen Mikroorganismen mit den hybridisierten Oligonukleotidsonden.

6. Verfahren nach Anspruch 1 bis 5, **dadurch gekennzeichnet, dass** es sich bei der Probe um eine Probe aus alkoholfreien Getränken handelt.

7. Kit zur Durchführung eines Verfahrens nach einem der Ansprüche 1 bis 6, enthaltend mindestens das Oligonukleotid nach Anspruch 1.

**Claims**

1. A method for the detection of drink-spoiling microorganisms in a sample, whereby the detection is carried out using at least one oligonucleotide probe having the sequence (in 5' -> 3' direction):

SEQ ID No. 1: 5' - GTTTGACCAGATTCTCCGCTC

2. The method according to claim 1, whereby drink-spoiling microorganisms belonging to the genus *Zygosaccharomyces* are detected using the oligonucleotide probe SEQ ID No. 1.

3. The method according to claim 2, **characterised in that** the oligonucleotide probe is used in conjunction with one or more than one competitor probes.

4. The method according to claim 3, **characterised in that** the oligonucleotide probe SEQ ID No. 1 is used in conjunction with one or more than one competitor probes selected from the group consisting of SEQ ID No. 2 to SEQ ID No. 4.

5. The method according to any of claims 1 to 4, **characterised in that** it comprises the following steps:

a) Cultivating the drink-spoiling microorganisms contained in the sample,
b) Fixing the drink-spoiling microorganisms contained in the sample,
c) Incubating the fixed microorganisms with at least the oligonucleotide probe, optionally in conjunction with a competitor probe,
d) Removing non-hybridised oligonucleotide probes,
e) Detecting and visualising and optionally quantifying the drink-spoiling microorganisms using the hybridised oligonucleotide probes.

6. The method according to any of claims 1 to 5, **characterised in that** the sample is a sample made of non-alcoholic beverages.

7. A kit for performing a method according to any of claims 1 to 6, containing at least the oligonucleotide according to claim 1.

**Revendications**

1. Procédé de détection de micro-organismes nocifs pour les boissons dans un échantillon, la détection s'effectuant au moyen d'au moins une sonde d'oligonucléotides avec la séquence (dans le sens 5' > 3'):

SEQ ID N° : 1 :5' - GTTTGACCAGATTCTCCGCTC

2. Procédé selon la revendication 1, dans lequel les micro-organismes nocifs pour les boissons du genre *Zygosaccharomyces* sont détectés au moyen de la sonde d'oligonucléotides SEQ ID N° : 1.

3. Procédé selon la revendication 2, **caractérisé en ce que** la sonde d'oligonucléotide est utilisée conjointement avec une ou plusieurs sondes compétitrices.

4. Procédé selon la revendication 3, **caractérisé en ce que** la sonde d'oligonucléotides SEQ ID N° : 1 est utilisée conjointement avec une ou plusieurs sondes compétitrices, choisies dans le groupe comprenant SEQ ID N° 2 à SEQ ID N° : 4.

5. Procédé selon la revendication 1 à 4, **caractérisé en ce qu'**il comprend les étapes suivantes :

a) culture des micro-organismes nocifs pour les boissons contenus dans l'échantillon,
b) fixation des micro-organismes nocifs pour les boissons contenus dans l'échantillon,
c) incubation des microorganismes fixés avec au moins la sonde d'oligonucléotides éventuellement, conjointement avec une sonde compétitrice,
d) élimination des sondes d'oligonucléotides non hybridés,
e) détection et visualisation et éventuellement, quantification des microorganismes nocifs pour les boissons avec les sondes d'oligonucléotides hybridées.

6. Procédé selon les revendications 1 à 5, **caractérisé en ce que** la sonde est une sonde de boissons sans alcool.

7. Kit de réalisation d'un procédé selon l'une quelconque des revendications 1 à 6, contenant au moins l'oligonucléotide selon la revendication 1.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 077259 A
- WO 0153316 A
- DE 10061655
- DE 10344057


### In der Beschreibung aufgeführte Nicht-Patentliteratur


- **AMANN, R. I. ; W. LUDWIG ; K.-H. SCHLEIFER.** Phylogenetic identification and in situ detection of individual microbial cells without cultivation. *Microbial. Rev.,* 1995, vol. 59, 143-169
- **WOESE, C. R.** Bacterial evolution. *Microbiol. Rev.,* 1987, vol. 51, 221-271